# EUROPEAN PATENT SPECIFICATION

(11) **EP 0 584 290 B1**
(45) Date of publication and mention of the grant of the patent: **30.08.2000**
(21) Application number: 92923584.4
(22) Date of filing: 24.04.1992
(51) Int. Cl.: C07K 5/10, C07K 7/06, A61K 38/08

(54) **ENDOTHELIN ANTAGONISTS**
ENDOTHELINANTAGONISTEN
ANTAGONISTES D'ENDOTHELINE

(30) Priority: 16.05.1991 US 701274; 18.12.1991 US 809746
(43) Date of publication of application: 02.03.1994
(73) Proprietor: WARNER-LAMBERT COMPANY, Ann Arbor, Michigan 48105 (US)
(72) Inventor: CODY, Wayne, Livingston, Saline, MI 48176 (US); DePUE, Patricia, Canton, MI 48187 (US); DOHERTY, Annette, Marian, Ann Arbor, MI 48103 (US); TAYLOR, Michael, Douglas, Ann Arbor, MI 48103 (US)
(74) Representative: Mansmann, Ivo
(86) International application number: US9203408
(87) International publication number: WO9220706

(56) References cited:
- Journal of Cardiovascular Pharmacology, (Endothelin II, Proceedings of the Second International Conference on Endothelin, Tsukaba, 9-12 December 1990), vol. 17, supplement 7, 1991, (New York, US), A.M. DOHERTY et al.: "Structure-activity studies of the C-terminal region of the endothelins and the sarafotoxins", pages S59-S61, see page S60, table 1 and the summary
- G.R. PETTIT: "Synthetic Peptides", vol. 1, 1970, Van Nostrand Reinhold Co., New York, US, see page 149, peptides no. 6,16
- Biochemical and Biophysical Research Communications, vol. 163, no. 1, 30 August 1989, (Duluth, US), K. NAKAJIMA et al.: "Structure-activity relationship of endothelin: importance of charged groups", pages 424-429, see page 428, peptide no. 10

## Description

### BACKGROUND OF THE INVENTION

The present invention relates to novel antagonists of endothelin useful as pharmaceutical agents, to methods for their production, to pharmaceutical compositions which include these compounds and a pharmaceutically acceptable carrier.

More particularly, the novel compounds of the present invention are antagonists of endothelin useful in controlling hypertension, myocardial infarction, metabolic, endocrinological, and neurological disorders, congestive heart failure, endotoxic shock, subarachnoid hemorrhage, arrhythmias, asthma, acute renal failure, preeclampsia, and diabetes.

Endothelin-1 (ET-1), a potent vasoconstrictor, is a 21 amino acid bicyclic peptide that was first isolated from cultured porcine aortic endothelial cells. Endothelin-1, is one of a family of structurally similar bicyclic peptides which include; ET-2, ET-3, vasoactive intestinal contractor (VIC), and the sarafotoxins (SRTX's). The unique bicyclic structure and corresponding arrangement of the disulfide bridges of ET-1, which are the same for the endothelins, VIC, and the sarafotoxins, has led to significant speculation as to the importance of the resulting induced secondary structure to receptor binding and functional activity. ET-1 analogues with incorrect disulfide pairings exhibit at least 100-fold less vasoconstrictor activity. The flexible C-terminal hexapeptide of ET-1 has been shown to be important for binding to the ET receptor and functional activity in selected tissues. Additionally, the C-terminal amino acid (Trp-21) has a critical role in binding and vasoconstrictor activity, since ET[1-20] exhibits approximately 1000-fold less functional activity.

Rovero, P., et al, British Journal of Pharmacology 101, pages 232-236 (1990) disclosed various analogs of the C-terminal hexapeptide of ET-1, none of which were reported to be antagonists of ET-1.

Doherty, A. M., et al, Abstract, Second International Conference on Endothelin, Tsukuba, Japan, December 9, 1990, and the published manuscript (J. Cardiovasc. Pharm. 17 (Suppl. 7), 1991, pp. 559-561) disclosed various analogs of the C-terminal hexapeptide of ET-1, none of which exhibited any functional activity.

However, we have surprisingly and unexpectedly found that a series of C-terminal hexapeptide and related analogs of ET-1 are receptor antagonists of endothelin.

In the Report on a Conference, held on December 9-12, 1990 at the University of Tsukuba, Japan (D1), compound 13 of (D1) was made public by oral presentation. This compound has been excluded from the claims by disclaimer. In (D1) it is disclosed that none of the described compounds had functional activity as either an agonist or antagonist on ET-1 induced contractions in the rabbit isolated pulmonary at concentrations up to 30 micromolar.

### SUMMARY OF THE INVENTION

Accordingly, the present invention is a compound of Formula I

AA¹-AA²⁻AA³-AA⁴-AA⁵-AA⁶ I

in which
AA¹ is 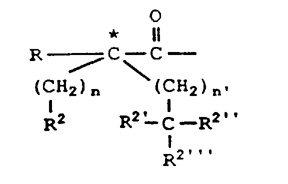 wherein
   R is
      hydrogen,
      C₁-C₁₂ alkyl,
      C₂-C₁₂ alkenyl,
      C₂-C₁₂ alkynyl,
      C₃-C₁₂ cycloalkyl,
      C₃-C₁₂ cycloalkyl C₁-C₁₂alkyl,
      an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from C₁-C₁₂ alkyl, C₁-C₁₂ alkoxy, C₁-C₁₂-thioalkoxy, hydroxy, thiol, nitro, halogen, amino, 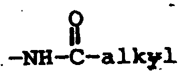 wherein alkyl is as defined above, 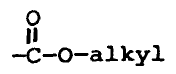 wherein alkyl is as defined above, 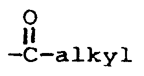 wherein alkyl is as defined above, or aryl,
         or a
      heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, an aromatic radical as defined above, alkoxy as defined above, thioalkoxy as defined above, hydroxy, thiol, nitro, halogen, formyl, amino, 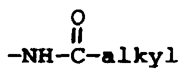 wherein alkyl is as defined above, 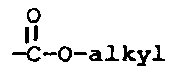 wherein alkyl is as defined above, 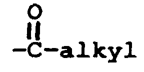 wherein alkyl is as defined above, or phenyl,
      fluorenylmethyl, 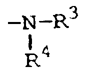 wherein R³ and R⁴ are each the same or different and each is
      hydrogen,
      C₁-C₁₂ alkyl,
      C₂-C₁₂ alkenyl,
      C₂-C₁₂ alkynyl,
      C₃-C₁₂ cycloalkyl,
      C₃-C₁₂ cycloalkyl C₁-C₁₂alkyl,
      an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 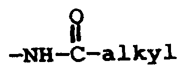 wherein alkyl is as defined above, 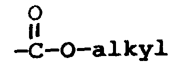 wherein alkyl is as defined above, 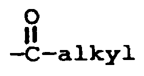 wherein alkyl is as defined above, or aryl,
         or a
      heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aromatic radical as defined above, alkoxy as defined above, thioalkoxy as defined above, hydroxy, thiol, nitro, halogen, formyl, amino, 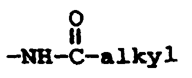 wherein alkyl is as defined above, 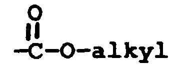 wherein alkyl is as defined above, 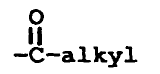 wherein alkyl is as defined above or phenyl, or
      fluoroenylmethyl, 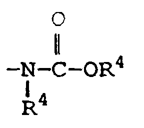 wherein R³ and R⁴ are each the same or different and each is as defined above, and R^{2'}, R^{2''}, and R^{2'''} are each the same or different and each is
      hydrogen,
      C₁-C₁₂ alkyl,
      an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 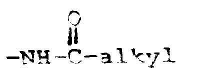 wherein alkyl is as defined above, 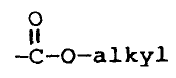 wherein alkyl is as defined above, 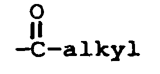 wherein alkyl is as defined above, or aryl,
         or a
      heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aromatic radical as defined above, alkoxy as defined above, thioalkoxy as defined above, hydroxy, thiol, nitro, halogen, formyl, amino, 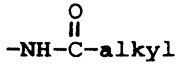 wherein alkyl is as defined above, 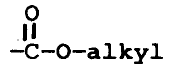 wherein alkyl is as defined above, 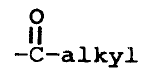 wherein alkyl is as defined above or phenyl, with the proviso that at least one of R², R^{2'}, and R^{2''} is an aromatic radical or heteroaryl radical as defined above, and R^{2'''} is hydrogen or methyl,
   R² is hydrogen or methyl,
   n is zero, and
   n' is zero or an integer of 1, 2, or 3,
AA² is 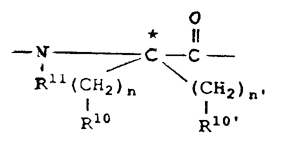 wherein
   R¹¹ is hydrogen or methyl,
   n is zero,
   R¹⁰ is hydrogen or methyl,
   n' is zero or an integer of 1, 2, 3,
   or 4, and
   R¹⁰ is C₁-C₁₂ alkyl,
   OH, 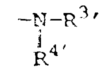 wherein R^{3'} and R^{4'} are each the same or different and each is
   hydrogen,
   C₁-C₁₂ alkyl, or
   an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 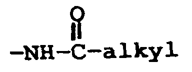 wherein alkyl is as defined above, 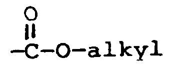 wherein alkyl is as defined above, 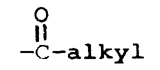 wherein alkyl is as defined above, or aryl, 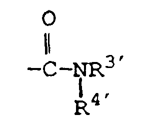 wherein R^{3'} and R^{4'} are as defined above, 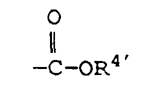 wherein R^{4'} is as defined above;
AA³ is 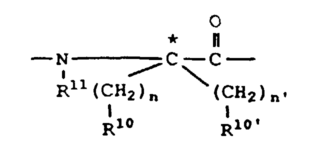 wherein
   R¹¹ is hydrogen or methyl,
   n is zero,
   R¹⁰ is hydrogen or methyl,
   n'' is zero or an integer of 1, 2, or 3, and
   R^{11'} is C₁-C₁₂ alkyl,
   an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 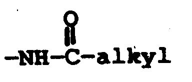 wherein alkyl is as defined above, 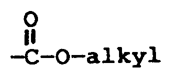 wherein alkyl 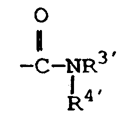 is as defined above, 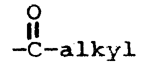 wherein alkyl is as defined above, or aryl, wherein R^{3'} and R^{4'} are 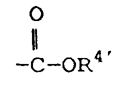 as defined above, wherein R^{4'} is as defined above,
AA⁴ and AA⁵ are each 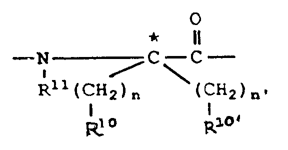 wherein
   R¹¹ is hydrogen or methyl,
   n is zero,
   R¹⁰ is hydrogen or methyl,
   n' is zero, and
   R^{10'} is C₁-C₁₂ alkyl,
      or C₃-C₁₂ cycloalkyl,
AA⁶ is 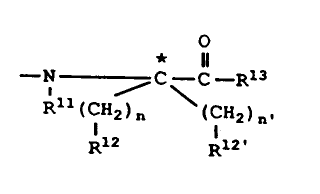 wherein
   R¹¹ is hydrogen or methyl,
   n is zero,
   R¹² is hydrogen, or methyl,
   n' is zero or an integer of 1, 2, of 3,
   R^{12'} is
   an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 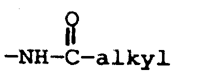 wherein alkyl is as defined above, 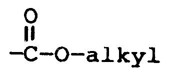 wherein alkyl is as defined above, 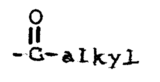 wherein alkyl is as defined above, or aryl,
      or a
   heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 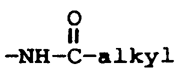 wherein alkyl is as defined above, 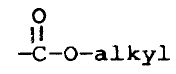 wherein alkyl is as defined above, 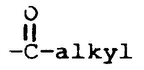 wherein alkyl is as defined above or phenyl,
   R¹³ is
      -(CH₂)ₙ-CO₂H wherein n is zero or an integer of 1, 2, 3, 4, 5, or 6,
      -(CH₂)ₙ-OH wherein n is zero or an integer of 1, 2, 3, 4, 5, or 6, or 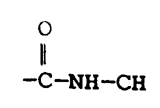 wherein R¹⁴ is hydrogen or
      -CH₂CO₂H,
   stereochemistry at 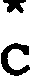 in AA¹ is D,
   stereochemistry at 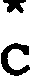 in AA², AA³, AA⁴, or AA⁵ is D or L and
   stereochemistry at 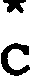 in AA⁶ is L,
   or a pharmaceutically acceptable salt thereof.

Elevated levels of endothelin have been postulated to be involved in a number of pathophysiological states including diseases associated with the cardiovascular system as well as various metabolic and endocrinological disorders. As antagonists of endothelin, the compounds of Formula I are useful in the treatment of hypertension, myocardial infarction, metabolic, endocrinological and neurological disorders, congestive heart failure, endotoxic shock, subarachnoid hemorrhage, arrhythmias, asthma, acute renal failure, preeclampsia, and diabetes.

A still further embodiment of the present invention is a pharmaceutical composition for administering an effective amount of a compound of Formula I in unit dosage form in the treatment methods mentioned above.

Finally, the present invention is directed to methods for production of a compound of Formula I.

### DETAILED DESCRIPTION OF THE INVENTION

In the compounds of Formula I, the term "C₁-C₁₂ alkyl" means a straight or branched hydrocarbon radical having from 1 to 12 carbon atoms and includes, for example, methyl, ethyl, n-propyl, isopropyl, n-butyl, sec-butyl, isobutyl, tert-butyl, n-pentyl, n-hexyl, n-heptyl, n-octyl, n-nonyl, n-decyl, undecyl, or dodecyl.

The term "C₂-C₁₂ alkenyl" means a straight or branched unsaturated hydrocarbon radical having from 2 to 12 carbon atoms and includes, for example, ethenyl, 2-propenyl, 1-butenyl, 2-butenyl, 1-pentenyl, 2-pentenyl, 3-methyl-3-butenyl, 1-hexenyl, 2-hexenyl, 3-hexenyl, 3-heptenyl, 1-octenyl, 1-nonenyl, 1-decenyl, 1-undecenyl or 1-dodecenyl.

The term "C₂-C₁₂ alkynyl" means a straight or branched triple bonded unsaturated hydrocarbon radical having from 2 to 12 carbon atoms and includes, for example, ethynyl, 2-propynyl, 1-butynyl, 2-butynyl, 3-butynyl, 1-pentynyl, 3-pentynyl, 1-hexynyl, 2-hexynyl, 3-hexynyl, 3-heptynyl, 1-octynyl, 2-octynyl, 1-nonynyl, 2-nonynyl, 3-nonynyl, 4-nonynyl, 1-decynyl, 2-decynyl, 2-undecynyl, 3-undecynyl, or 3-dodecynyl.

The term "C₃-C₁₂ cycloalkyl" means a saturated hydrocarbon ring which contains from 3 to 12 carbon atoms, for example, cyclopropyl, cyclobutyl, cyclopentyl, cyclohexyl or adamantyl.

The term "cycloalkylalkyl" means a saturated hydrocarbon ring attached to an alkyl group wherein alkyl is as defined above. The saturated hydrocarbon ring contains from 3 to 12 carbon atoms. Examples of such are cyclopropylmethyl, cyclopentylmethyl, cyclohexylmethyl, adamantylmethyl and the like.

The terms "alkoxy" and "thioalkoxy" are O-alkyl or S-alkyl as defined above for alkyl.

The term aromatic radical means a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 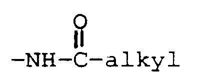 wherein alkyl is as defined above, 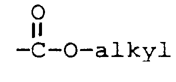 wherein alkyl is as defined above, 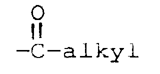 wherein alkyl is as defined above, or aryl.

The term heteroaromatic radical means a 2-or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 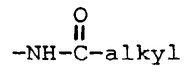 wherein alkyl is as defined above, 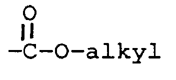 wherein alkyl is as defined above, 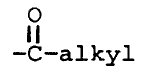 wherein alkyl is as defined above or phenyl.

The term "heterocycloalkyl" means 2- or 3-tetrahydrothieno, 2- or 3-tetrahydrofurano, 2- or 3-pyrrolidino, 2-, 4-, or 5-thiazolidino, 2-, 4-, or 5-oxazolidino, 2-, 3-, or 4-piperidino, N-morpholinyl or N-thiamorpholinyl.

"Halogen" is fluorine, chlorine, bromine or iodine.

The following table provides a list of abbreviations and definitions thereof used in the present invention.

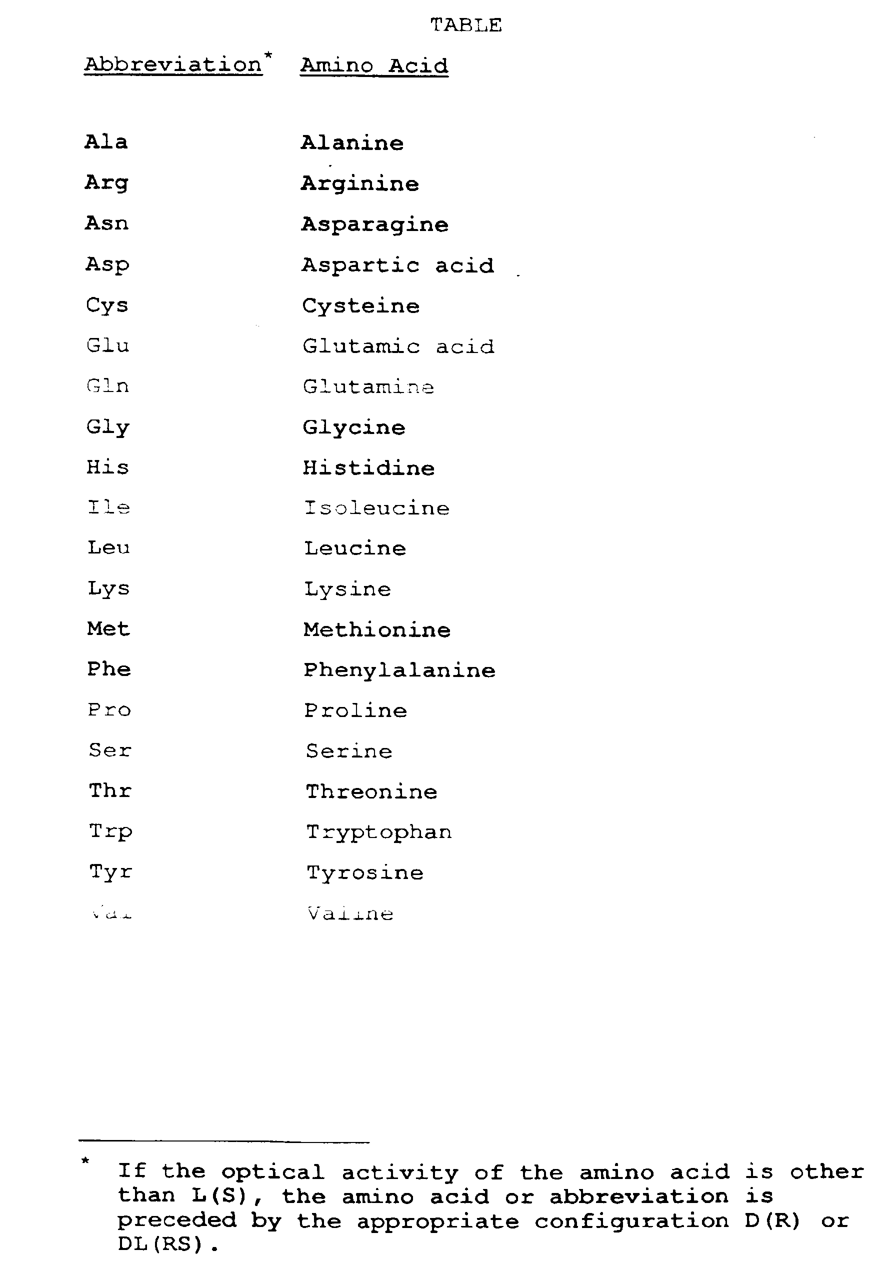

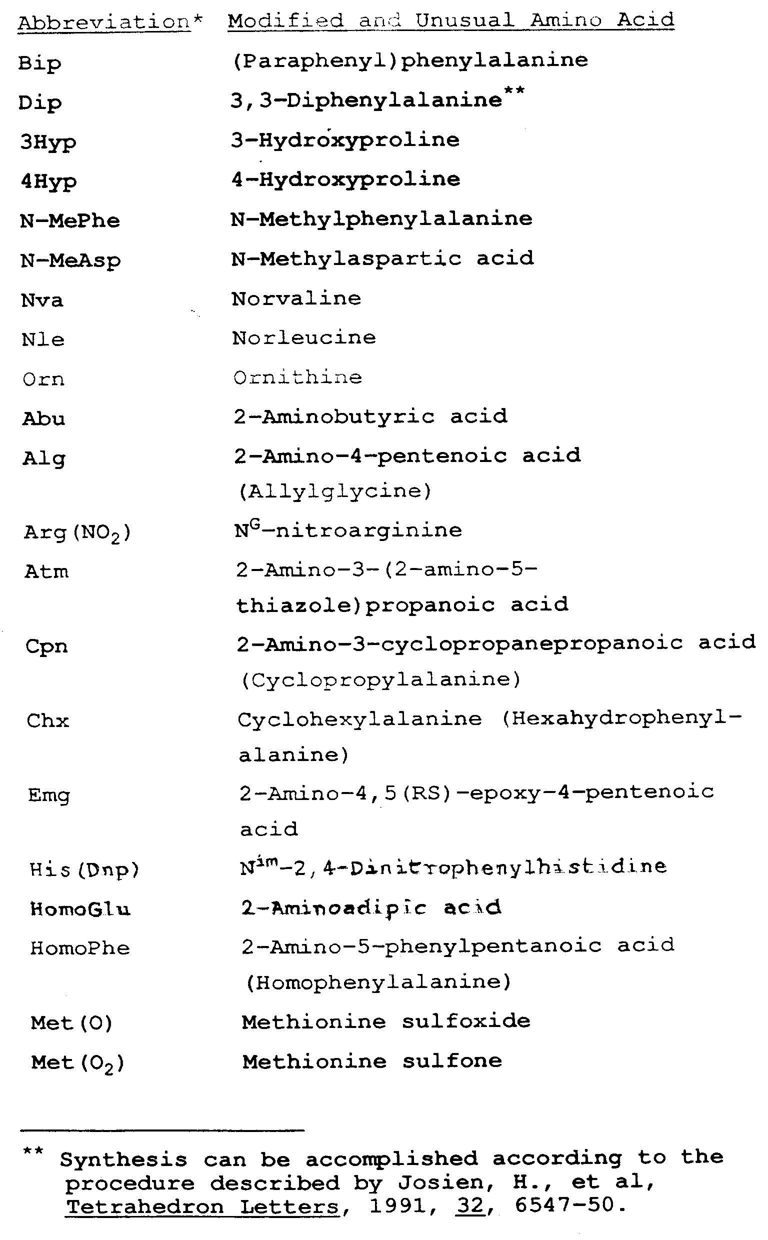

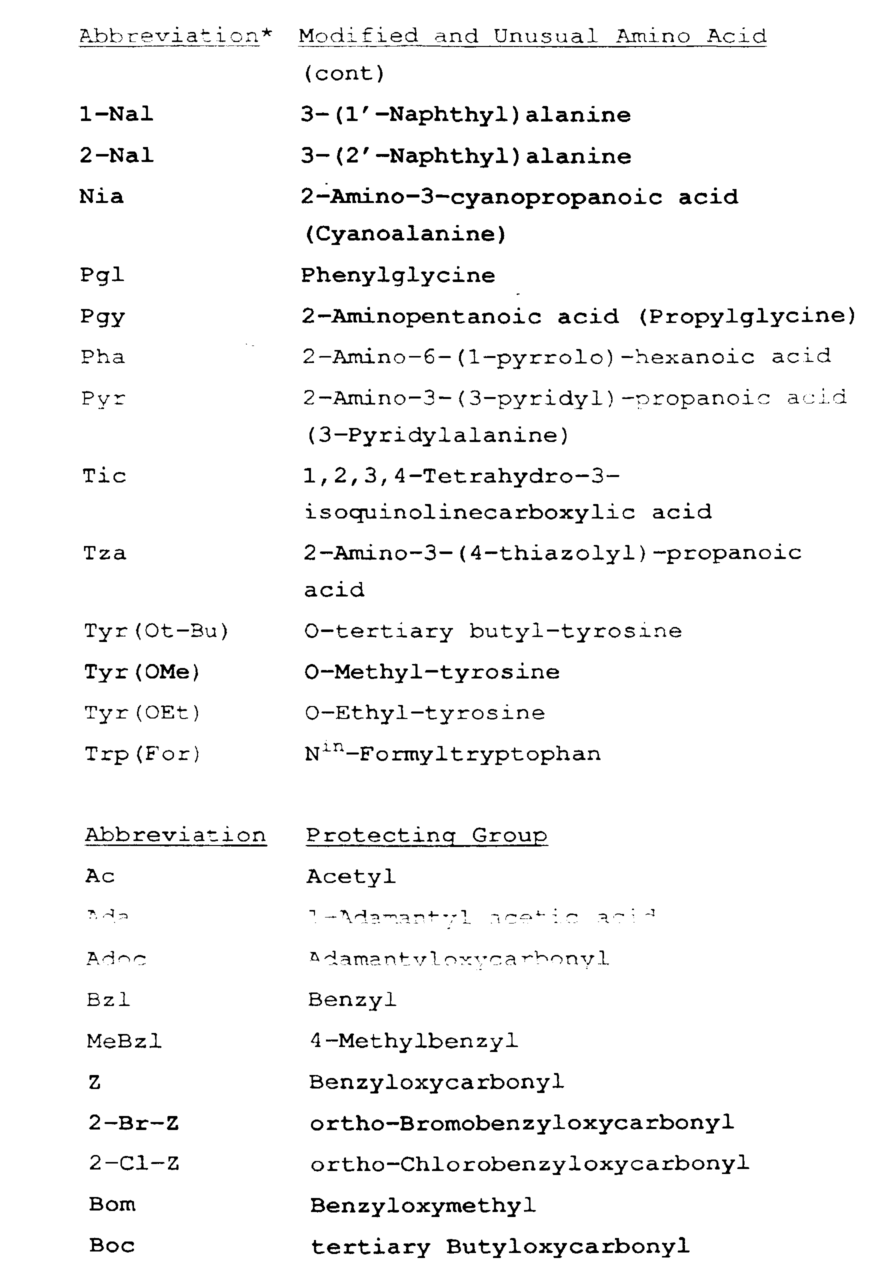

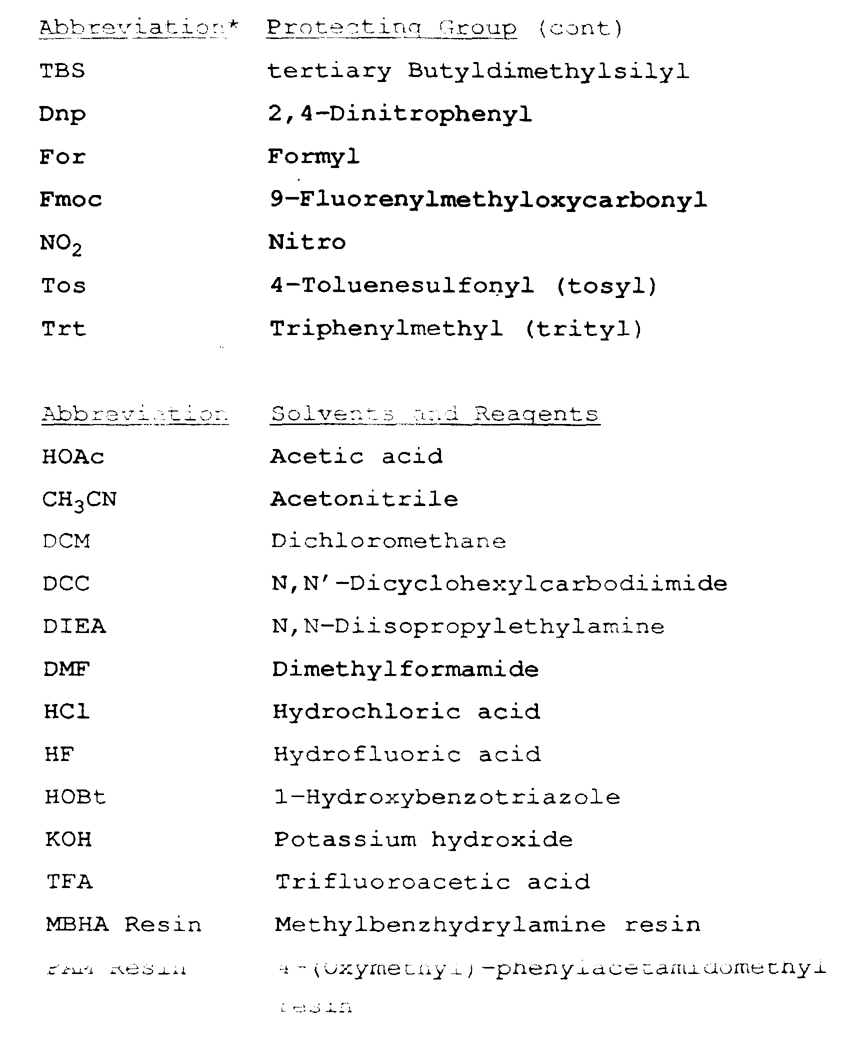

The compounds of Formula I are capable of further forming both pharmaceutically acceptable acid addition and/or base salts. All of these forms are within the scope of the present invention.

Pharmaceutically acceptable acid addition salts of the compounds of Formula I include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge, S. M., et al, "Pharmaceutical Salts," Journal of Pharmaceutical Science, 66, pp. 1-19 (1977)).

The acid addition salts of said basic compounds are prepared by contacting the free base form with a sufficient amount of the desired acid to produce the salt in the conventional manner. Preferably a peptide of Formula I can be converted to an acidic salt by treating with an aqueous solution of the desired base, such that the resulting pH is less than 4. The solution can be passed through a C18 cartridge to absorb the peptide, washed with copious amounts of water, the peptide eluted with a polar organic solvent such as, for example, methanol, acetonitrile, and the like, and isolated by concentrating under reduced pressure followed by lyophilization. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge, S. M., et al., "Pharmaceutical Salts," Journal of Pharmaceutical Science, 66, pp. 1-19 (1977)).

The base addition salts of said acidic compounds are prepared by contacting the free acid form with a sufficient amount of the desired base to produce the salt in the conventional manner. Preferably, a peptide of Formula I can be converted to a base salt by treating with an aqueous solution of the desired base, such that the resulting pH is greater than 9. The solution can be passed through a C18 cartridge to absorb the peptide, washed with copious amounts of water, the peptide eluted with a polar organic solvent such as, for example, methanol, acetonitrile and the like, and isolated by concentrating under reduced pressure followed by lyophilization. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present invention.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms as well as the appropriate mixtures thereof.

A preferred compound of Formula I is one wherein
AA¹ is
   D-Dip,
   D-Bip,
   D-His,
   D-His(Dnp),
   D-2-Nal,
   D-1-Nal,
   D-Phe,
   D-Pgl,
   D-Tyr,
   D-Tyr(OMe),
   D-Tyr(OEt),
   D-Tyr(OtBu),
   D-Trp,
   D-Trp(For),
   D-Tic,
   D-Tza,
   D-Pyr,
   Ac-D-Dip,
   Ac-D-Bip,
   Ac-D-His,
   Ac-D-His(Dnp),
   Ac-D-2-Nal,
   Ac-D-1-Nal,
   Ac-D-Phe,
   Ac-D-Pgl,
   Ac-D-Tyr,
   Ac-D-Tyr(OMe),
   Ac-D-Tyr(OEt),
   Ac-D-Tyr(OtBu),
   Ac-D-Trp,
   Ac-D-Trp(For),
   Ac-D-Tic,
   Ac-D-Tza,
   Ac-D-Pyr,
   Ada-D-Dip,
   Ada-D-Bip,
   Ada-D-His,
   Ada-D-His(Dnp),
   Ada-D-2-Nal,
   Ada-D-1-Nal,
   Ada-D-Phe,
   Ada-D-Pgl,
   Ada-D-Tyr,
   Ada-D-Tyr(OMe),
   Ada-D-Tyr(OEt),
   Ada-D-Tyr(OtBu),
   Ada-D-Trp,
   Ada-D-Trp(For),
   Ada-D-Tic,
   Ada-D-Tza,
   Ada-D-Pyr,
   Adoc-D-Dip,
   Adoc-D-Bip,
   Adoc-D-His,
   Adoc-D-His(Dnp),
   Adoc-D-2-Nal,
   Adoc-D-1-Nal,
   Adoc-D-Phe,
   Adoc-D-Pgl,
   Adoc-D-Tyr,
   Adoc-D-Tyr(OMe),
   Adoc-D-Tyr(OEt).
   Adoc-D-Tyr(OtBu),
   Adoc-D-Trp,
   Adoc-D-Trp(For),
   Adoc-D-Tic,
   Adoc-D-Tza,
   Adoc-D-Pyr,
   Boc-D-Dip,
   Boc-D-Bip,
   Boc-D-His,
   Boc-D-His(Dnp),
   Boc-D-2-Nal,
   Boc-D-1-Nal,
   Boc-D-Phe,
   Boc-D-Pgl,
   Boc-D-Tyr,
   Boc-D-Tyr(OMe),
   Boc-D-Tyr(OEt),
   Boc-D-Tyr(OtBu),
   Boc-D-Trp,
   Boc-D-Trp(For),
   Boc-D-Tic,
   Boc-D-Tza,
   Boc-D-Pyr,
   Z-D-Dip,
   Z-D-Bip,
   Z-D-His,
   Z-D-His(Dnp),
   Z-D-2-Nal,
   Z-D-1-Nal,
   Z-D-Phe,
   Z-D-Pgl,
   Z-D-Tyr,
   Z-D-Tyr(OMe),
   Z-D-Tyr(OEt),
   Z-D-Tyr(OtBu),
   Z-D-Trp,
   Z-D-Trp(For),
   Z-D-Tic,
   Z-D-Tza,
   Z-D-Pyr,
   Fmoc-D-Dip,
   Fmoc-D-Bip,
   Fmoc-D-His,
   Fmoc-D-His(Dnp),
   Fmoc-D-2-Nal,
   Fmoc-D-1-Nal,
   Fmoc-D-Phe,
   Fmoc-D-Pgl,
   Fmoc-D-Tyr,
   Fmoc-D-Tyr(OMe),
   Fmoc-D-Tyr(OEt),
   Fmoc-D-Tyr(OtBu),
   Fmoc-D-Trp,
   Fmoc-D-Trp(For),
   Fmoc-D-Tic,
   Fmoc-D-Tza,
   Fmoc-D-Pyr, or
AA² is Ala,
   Alg,
   Arg
   Asn,
   Asp,
   Dab,
   Glu,
   Gln,
   Gly,
   homoArg,
   homoGlu,
   homoLys,
   Ile,
   Leu,
   D-Leu,
   Lys,
   Met,
   Met(O),
   Met(O₂),
   Nva,
   Nle,
   Orn,
   Phe,
   Tyr,
   Val, or
   AA² is absent;
AA³ is Asn,
   Asp,
   D-Asp,
   N-MeAsp,
   Glu,
   Gln,
   homoPhe,
   Phe,
   Tyr, or
   AA³ is absent;
AA⁴ is Ala,
   Chx,
   Gly,
   Glu,
   Ile,
   D-Ile,
   Leu,
   Nleu,
   Nval,
   Val, or
   AA⁴ is absent;
AA⁵ is Ala,
   Chx,
   Gly,
   Ile,
   D-Ile,
   Leu,
   Nleu,
   Nval,
   Val, or
   AA⁵ is absent; and
AA⁶ is 2-Nal,
   1-Nal,
   Pyr,
   Trp,
   Tyr(OMe),
   Tyr(OEt),
   Tyr(Ot-Bu),
   Tyr,
   Trp-Gly,
   Trp-Asp,
   Trp(For),
   Dip,
   Phe,
   Bza, 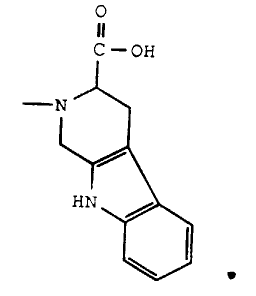 Particularly valuable are:
   D-Phe-Leu-Asp-Ile-Ile-Trp;
   D-His(Dnp)-Leu-Asp-Ile-Ile-Trp;
   D-Trp-Leu-Asp-Ile-Ile-Trp;
   D-Tyr-Leu-Asp-Ile-Ile-Trp;
   D-Tyr(OMe)-Leu-Asp-Ile-Ile-Trp;
   D-Tyr(OEt)-Leu-Asp-Ile-Ile-Trp;
   D-2-Nal-Leu-Asp-Ile-Ile-Trp;
   D-1-Nal-Leu-Asp-Ile-Ile-Trp;
   D-Pgl-Leu-Asp-Ile-Ile-Trp;
   D-Pyr-Leu-Asp-Ile-Ile-Trp;
   D-Tic-Leu-Asp-Ile-Ile-Trp;
   D-Dip-Leu-Asp-Ile-Ile-Trp;
   D-Bip-Leu-Asp-Ile-Ile-Trp;
   Ac-D-Phe-Leu-Asp-Ile-Ile-Trp;
   Ac-D-His(Dnp)-Leu-Asp-Ile-Ile-Trp;
   Ac-D-Trp-Leu-Asp-Ile-Ile-Trp;
   Ac-D-Tyr-Leu-Asp-Ile-Ile-Trp;
   Ac-D-Tyr(OMe)-Leu-Asp-Ile-Ile-Trp;
   Ac-D-Tyr(OEt)-Leu-Asp-Ile-Ile-Trp;
   Ac-D-2-Nal-Leu-Asp-Ile-Ile-Trp;
   Ac-D-1-Nal-Leu-Asp-Ile-Ile-Trp;
   Ac-D-Pgl-Leu-Asp-Ile-Ile-Trp;
   Ac-D-Pyr-Leu-Asp-Ile-Ile-Trp;
   Ac-D-Tic-Leu-Asp-Ile-Ile-Trp;
   Ac-D-Dip-Leu-Asp-Ile-Ile-Trp;
   Ac-D-Bip-Leu-Asp-Ile-Ile-Trp;
   Fmoc-D-Phe-Leu-Asp-Ile-Ile-Trp;
   Fmoc-D-His-Leu-Asp-Ile-Ile-Trp;
   Fmoc-D-Trp-Leu-Asp-Ile-Ile-Trp;
   Fmoc-D-Tyr-Leu-Asp-Ile-Ile-Trp;
   Fmoc-D-Tyr(OMe)-Leu-Asp-Ile-Ile-Trp;
   Fmoc-D-Tyr(OEt)-Leu-Asp-Ile-Ile-Trp;
   Fmoc-D-2-Nal-Leu-Asp-Ile-Ile-Trp;
   Fmoc-D-1-Nal-Leu-Asp-Ile-Ile-Trp;
   Fmoc-D-Dip-Leu-Asp-Ile-Ile-Trp;
   Fmoc-D-Bip-Leu-Asp-Ile-Ile-Trp;
   Ada-D-Phe-Leu-Asp-Ile-Ile-Trp;
   Ada-D-His-Leu-Asp-Ile-Ile-Trp;
   Ada-D-Trp-Leu-Asp-Ile-Ile-Trp;
   Ada-D-Tyr-Leu-Asp-Ile-Ile-Trp;
   Ada-D-Tyr(OMe)-Leu-Asp-Ile-Ile-Trp;
   Ada-D-Tyr(OEt)-Leu-Asp-Ile-Ile-Trp;
   Ada-D-2-Nal-Leu-Asp-Ile-Ile-Trp;
   Ada-D-1-Nal-Leu-Asp-Ile-Ile-Trp;
   Ada-D-Dip-Leu-Asp-Ile-Ile-Trp;
   Ada-D-Bip-Leu-Asp-Ile-Ile-Trp;
   Ac-D-Phe-D-Leu-Asp-Ile-Ile-Trp;
   Ac-D-His-D-Leu-Asp-Ile-Ile-Trp;
   Ac-D-Trp-D-Leu-Asp-Ile-Ile-Trp;
   Ac-D-Tyr-D-Leu-Asp-Ile-Ile-Trp;
   Ac-D-Tyr(OMe)-D-Leu-Asp-Ile-Ile-Trp;
   Ac-D-Tyr(OEt)-D-Leu-Asp-Ile-Ile-Trp;
   Ac-D-2-Nal-D-Leu-Asp-Ile-Ile-Trp;
   Ac-D-1-Nal-D-Leu-Asp-Ile-Ile-Trp;
   Ac-D-Dip-D-Leu-Asp-Ile-Ile-Trp;
   Ac-D-Bip-D-Leu-Asp-Ile-Ile-Trp;
   Ac-D-Phe-Ile-Asp-Ile-Ile-Trp;
   Ac-D-His-Ile-Asp-Ile-Ile-Trp;
   Ac-D-Trp-Ile-Asp-Ile-Ile-Trp;
   Ac-D-Tyr-Ile-Asp-Ile-Ile-Trp;
   Ac-D-Tyr(OMe)-Ile-Asp-Ile-Ile-Trp;
   Ac-D-Tyr(OEt)-Ile-Asp-Ile-Ile-Trp;
   Ac-D-2-Nal-Ile-Asp-Ile-Ile-Trp;
   Ac-D-1-Nal-Ile-Asp-Ile-Ile-Trp;
   Ac-D-Dip-Ile-Asp-Ile-Ile-Trp;
   Ac-D-Bip-Ile-Asp-Ile-Ile-Trp;
   Ac-D-Phe-Val-Asp-Ile-Ile-Trp;
   Ac-D-His-Val-Asp-Ile-Ile-Trp;
   Ac-D-Trp-Val-Asp-Ile-Ile-Trp;
   Ac-D-Tyr-Val-Asp-Ile-Ile-Trp;
   Ac-D-Tyr(OMe)-Val-Asp-Ile-Ile-Trp;
   Ac-D-Tyr(OEt)-Val-Asp-Ile-Ile-Trp;
   Ac-D-2-Nal-Val-Asp-Ile-Ile-Trp;
   Ac-D-1-Nal-Val-Asp-Ile-Ile-Trp;
   Ac-D-Dip-Val-Asp-Ile-Ile-Trp;
   Ac-D-Bip-Val-Asp-Ile-Ile-Trp;
   Ac-DPhe-Dab-Asp-Ile-Ile-Trp;
   Ac-DHis-Dab-Asp-Ile-Ile-Trp;
   Ac-DTrp-Dab-Asp-Ile-Ile-Trp;
   Ac-DTyr(OMe)-Dab-Asp-Ile-Ile-Trp;
   Ac-DTyr(OEt)-Dab-Asp-Ile-Ile-Trp;
   Ac-D2Nal-Dab-Asp-Ile-Ile-Trp;
   Ac-D3Nal-Dab-Asp-Ile-Ile-Trp;
   Ac-DDip-Dab-Asp-Ile-Ile-Trp;
   Ac-DBip-Dab-Asp-Ile-Ile-Trp;
   Ac-DPhe-Arg-Asp-Ile-Ile-Trp;
   Ac-DHis-Arg-Asp-Ile-Ile-Trp;
   Ac-DTrp-Arg-Asp-Ile-Ile-Trp;
   Ac-DTyr(OMe)-Arg-Asp-Ile-Ile-Trp;
   Ac-DTyr(OEt)-Arg-Asp-Ile-Ile-Trp;
   Ac-D2Nal-Arg-Asp-Ile-Ile-Trp;
   Ac-D3Nal-Arg-Asp-Ile-Ile-Trp;
   Ac-DDip-Arg-Asp-Ile-Ile-Trp;
   Ac-DBip-Arg-Asp-Ile-Ile-Trp;
   Ac-DPhe-hLys-Asp-Ile-Ile-Trp;
   Ac-DHis-hLys-Asp-Ile-Ile-Trp;
   Ac-DTrp-hLys-Asp-Ile-Ile-Trp;
   Ac-DTyr(OMe)-hLys-Asp-Ile-Ile-Trp;
   Ac-DTyr(OEt)-hLys-Asp-Ile-Ile-Trp;
   Ac-D2Nal-hLys-Asp-Ile-Ile-Trp;
   Ac-D3Nal-hLys-Asp-Ile-Ile-Trp;
   Ac-DDip-hLys-Asp-Ile-Ile-Trp;
   Ac-DBip-hLys-Asp-Ile-Ile-Trp;
   Ac-DPhe-hGlu-Asp-Ile-Ile-Trp;
   Ac-DHis-Glu-Asp-Ile-Ile-Trp;
   Ac-DTrp-Glu-Asp-Ile-Ile-Trp;
   Ac-DTyr(OMe)-Glu-Asp-Ile-Ile-Trp;
   Ac-DTyr(OEt)-Glu-Asp-Ile-Ile-Trp;
   Ac-D2Nal-Glu-Asp-Ile-Ile-Trp;
   Ac-D3Nal-Glu-Asp-Ile-Ile-Trp;
   Ac-DDip-Glu-Asp-Ile-Ile-Trp;
   Ac-DBip-Glu-Asp-Ile-Ile-Trp;
   Ac-DPhe-hGlu-Asp-Ile-Ile-Trp;
   Ac-DHis-hGlu-Asp-Ile-Ile-Trp;
   Ac-DTrp-hGlu-Asp-Ile-Ile-Trp;
   Ac-DTyr(OMe)-hGlu-Asp-Ile-Ile-Trp;
   Ac-DTyr(OEt)-hGlu-Asp-Ile-Ile-Trp;
   Ac-D2Nal-hGlu-Asp-Ile-Ile-Trp;
   Ac-D3Nal-hGlu-Asp-Ile-Ile-Trp;
   Ac-DDip-hGlu-Asp-Ile-Ile-Trp;
   Ac-DBip-hGlu-Asp-Ile-Ile-Trp;
   Ac-DPhe-Asp-Asp-Ile-Ile-Trp;
   Ac-DHis-Asp-Asp-Ile-Ile-Trp;
   Ac-DTrp-Asp-Asp-Ile-Ile-Trp;
   Ac-DTyr(OMe)-Asp-Asp-Ile-Ile-Trp;
   Ac-DTyr(OEt)-Asp-Asp-Ile-Ile-Trp;
   Ac-D2Nal-Asp-Asp-Ile-Ile-Trp;
   Ac-D3Nal-Asp-Asp-Ile-Ile-Trp;
   Ac-DDip-Asp-Asp-Ile-Ile-Trp;
   Ac-DBip-Asp-Asp-Ile-Ile-Trp;
   Ac-D-Phe-Lys-Asp-Ile-Ile-Trp;
   Ac-D-His-Lys-Asp-Ile-Ile-Trp;
   Ac-D-Trp-Lys-Asp-Ile-Ile-Trp;
   Ac-D-Tyr-Lys-Asp-Ile-Ile-Trp;
   Ac-D-Tyr(OMe)-Lys-Asp-Ile-Ile-Trp;
   Ac-D-Tyr(OEt)-Lys-Asp-Ile-Ile-Trp;
   Ac-D-2-Nal-Lys-Asp-Ile-Ile-Trp;
   Ac-D-1-Nal-Lys-Asp-Ile-Ile-Trp;
   Ac-D-Phe-Orn-Asp-Ile-Ile-Trp;
   Ac-D-His-Orn-Asp-Ile-Ile-Trp;
   Ac-D-Trp-Orn-Asp-Ile-Ile-Trp;
   Ac-D-Tyr-Orn-Asp-Ile-Ile-Trp;
   Ac-D-Tyr(OMe)-Orn-Asp-Ile-Ile-Trp;
   Ac-D-Tyr(OEt)-Orn-Asp-Ile-Ile-Trp;
   Ac-D-2-Nal-Orn-Asp-Ile-Ile-Trp;
   Ac-D-1-Nal-Orn-Asp-Ile-Ile-Trp;
   Ac-D-Phe-Gln-Asp-Ile-Ile-Trp;
   Ac-D-His-Gln-Asp-Ile-Ile-Trp;
   Ac-D-Trp-Gln-Asp-Ile-Ile-Trp;
   Ac-D-Tyr-Gln-Asp-Ile-Ile-Trp;
   Ac-D-Tyr(OMe)-Gln-Asp-Ile-Ile-Trp;
   Ac-D-Tyr(OEt)-Gln-Asp-Ile-Ile-Trp;
   Ac-D-2-Nal-Gln-Asp-Ile-Ile-Trp;
   Ac-D-1-Nal-Gln-Asp-Ile-Ile-Trp;
   Ac-D-Phe-Leu-Glu-Ile-Ile-Trp;
   Ac-D-His-Leu-Glu-Ile-Ile-Trp;
   Ac-D-Trp-Leu-Glu-Ile-Ile-Trp;
   Ac-D-Tyr-Leu-Glu-Ile-Ile-Trp;
   Ac-D-Tyr(OMe)-Leu-Glu-Ile-Ile-Trp;
   Ac-D-Tyr(OEt)-Leu-Glu-Ile-Ile-Trp;
   Ac-D-2-Nal-Leu-Glu-Ile-Ile-Trp;
   Ac-D-1-Nal-Leu-Glu-Ile-Ile-Trp;
   Ac-D-Phe-Leu-Asn-Ile-Ile-Trp;
   Ac-D-His-Leu-Asn-Ile-Ile-Trp;
   Ac-D-Trp-Leu-Asn-Ile-Ile-Trp;
   Ac-D-Tyr-Leu-Asn-Ile-Ile-Trp;
   Ac-D-Tyr(OMe)-Leu-Asn-Ile-Ile-Trp;
   Ac-D-Tyr(OEt)-Leu-Asn-Ile-Ile-Trp;
   Ac-D-2-Nal-Leu-Asn-Ile-Ile-Trp;
   Ac-D-1-Nal-Leu-Asn-Ile-Ile-Trp;
   Ac-D-Dip-Leu-Asn-Ile-Ile-Trp;
   Ac-D-Bip-Leu-Asn-Ile-Ile-Trp;
   Ac-DPhe-Leu-Phe-Ile-Ile-Trp;
   Ac-DHis-Leu-Phe-Ile-Ile-Trp;
   Ac-DTrp-Leu-Phe-Ile-Ile-Trp;
   Ac-DTyr(OMe)-Leu-Phe-Ile-Ile-Trp;
   Ac-DTyr(OEt)-Leu-Phe-Ile-Ile-Trp;
   Ac-D2Nal-Leu-Phe-Ile-Ile-Trp;
   Ac-D3Nal-Leu-Phe-Ile-Ile-Trp;
   Ac-DDip-Leu-Phe-Ile-Ile-Trp;
   Ac-DBip-Leu-Phe-Ile-Ile-Trp;
   Ac-D-Phe-Glu-Asp-Ile-Ile-Trp;
   Ac-D-Phe-Leu-Asp-Val-Ile-Trp;
   Ac-D-His-Leu-Asp-Val-Ile-Trp;
   Ac-D-Trp-Leu-Asp-Val-Ile-Trp;
   Ac-D-Tyr-Leu-Asp-Val-Ile-Trp;
   Ac-D-Tyr(OMe)-Leu-Asp-Val-Ile-Trp;
   Ac-D-Tyr(OEt)-Leu-Asp-Val-Ile-Trp;
   Ac-D-2-Nal-Leu-Asp-Val-Ile-Trp;
   Ac-D-1-Nal-Leu-Asp-Val-Ile-Trp;
   Ac-D-Dip-Leu-Asp-Val-Ile-Trp;
   Ac-D-Bip-Leu-Asp-Val-Ile-Trp;
   Ac-D-Phe-Leu-Asp-Chx-Ile-Trp;
   Ac-D-His-Leu-Asp-Chx-Ile-Trp;
   Ac-D-Trp-Leu-Asp-Chx-Ile-Trp;
   Ac-D-Tyr-Leu-Asp-Chx-Ile-Trp;
   Ac-D-Tyr(OMe)-Leu-Asp-Chx-Ile-Trp;
   Ac-D-Tyr(OEt)-Leu-Asp-Chx-Ile-Trp;
   Ac-D-2-Nal-Leu-Asp-Chx-Ile-Trp;
   Ac-D-1-Nal-Leu-Asp-Chx-Ile-Trp;
   Ac-D-Dip-Leu-Asp-Chx-Ile-Trp;
   Ac-D-Bip-Leu-Asp-Chx-Ile-Trp;
   Ac-D-Phe-Leu-Asp-D-Ile-Ile-Trp;
   Ac-D-His-Leu-Asp-D-Ile-Ile-Trp;
   Ac-D-Trp-Leu-Asp-D-Ile-Ile-Trp;
   Ac-D-Tyr-Leu-Asp-D-Ile-Ile-Trp;
   Ac-D-Tyr(OMe)-Leu-Asp-D-Ile-Ile-Trp;
   Ac-D-Tyr(OEt)-Leu-Asp-D-Ile-Ile-Trp;
   Ac-D-2-Nal-Leu-Asp-D-Ile-Ile-Trp;
   Ac-D-1-Nal-Leu-Asp-D-Ile-Ile-Trp;
   Ac-D-Dip-Leu-Asp-D-Ile-Ile-Trp;
   Ac-D-Bip-Leu-Asp-D-Ile-Ile-Trp;
   Ac-D-Phe-Leu-Asp-Ile-D-Ile-Trp;
   Ac-D-His-Leu-Asp-Ile-D-Ile-Trp;
   Ac-D-Trp-Leu-Asp-Ile-D-Ile-Trp;
   Ac-D-Tyr-Leu-Asp-Ile-D-Ile-Trp;
   Ac-D-Tyr(OMe)-Leu-Asp-Ile-D-Ile-Trp;
   Ac-D-Tyr(OEt)-Leu-Asp-Ile-D-Ile-Trp;
   Ac-D-2-Nal-Leu-Asp-Ile-D-Ile-Trp;
   Ac-D-1-Nal-Leu-Asp-Ile-D-Ile-Trp;
   Ac-D-Dip-Leu-Asp-Ile-D-Ile-Trp;
   Ac-D-Bip-Leu-Asp-Ile-D-Ile-Trp;
   Ac-D-Phe-Leu-Asp-Ile-Val-Trp;
   Ac-D-His-Leu-Asp-Ile-Val-Trp;
   Ac-D-Trp-Leu-Asp-Ile-Val-Trp;
   Ac-D-Tyr-Leu-Asp-Ile-Val-Trp;
   Ac-D-Tyr(OMe)-Leu-Asp-Ile-Val-Trp;
   Ac-D-Tyr(OEt)-Leu-Asp-Ile-Val-Trp;
   Ac-D-2-Nal-Leu-Asp-Ile-Val-Trp;
   Ac-D-1-Nal-Leu-Asp-Ile-Val-Trp;
   Ac-D-Dip-Leu-Asp-Ile-Val-Trp;
   Ac-D-Bip-Leu-Asp-Ile-Val-Trp;
   Ac-D-Phe-Leu-Asp-Ile-Chx-Trp;
   Ac-D-His-Leu-Asp-Ile-Chx-Trp;
   Ac-D-Trp-Leu-Asp-Ile-Chx-Trp;
   Ac-D-Tyr-Leu-Asp-Ile-Chx-Trp;
   Ac-D-Tyr(OMe)-Leu-Asp-Ile-Chx-Trp;
   Ac-D-Tyr(OEt)-Leu-Asp-Ile-Chx-Trp;
   Ac-D-2-Nal-Leu-Asp-Ile-Chx-Trp;
   Ac-D-1-Nal-Leu-Asp-Ile-Chx-Trp;
   Ac-D-Dip-Leu-Asp-Ile-Chx-Trp;
   Ac-D-Bip-Leu-Asp-Ile-Chx-Trp;
   Ac-D-Phe-Leu-Asp-Ile-Ile-2-Nal;
   Ac-D-His-Leu-Asp-Ile-Ile-2-Nal;
   Ac-D-Trp-Leu-Asp-Ile-Ile-2-Nal;
   Ac-D-Tyr-Leu-Asp-Ile-Ile-2-Nal;
   Ac-D-Tyr(OMe)-Leu-Asp-Ile-Ile-2-Nal;
   Ac-D-Tyr(OEt)-Leu-Asp-Ile-Ile-2-Nal;
   Ac-D-2-Nal-Leu-Asp-Ile-Ile-2-Nal;
   Ac-D-1-Nal-Leu-Asp-Ile-Ile-2-Nal;
   Ac-D-Dip-Leu-Asp-Ile-Ile-2-Nal;
   Ac-D-Bip-Leu-Asp-Ile-Ile-2-Nal;
   Ac-D-Phe-Leu-Asp-Ile-Ile-1-Nal;
   Ac-D-His-Leu-Asp-Ile-Ile-1-Nal;
   Ac-D-Trp-Leu-Asp-Ile-Ile-1-Nal;
   Ac-D-Tyr-Leu-Asp-Ile-Ile-1-Nal;
   Ac-D-Tyr(OMe)-Leu-Asp-Ile-Ile-1-Nal;
   Ac-D-Tyr(OEt)-Leu-Asp-Ile-Ile-1-Nal;
   Ac-D-2-Nal-Leu-Asp-Ile-Ile-1-Nal;
   Ac-D-1-Nal-Leu-Asp-Ile-Ile-1-Nal;
   Ac-D-Dip-Leu-Asp-Ile-Ile-1-Nal;
   Ac-D-Bip-Leu-Asp-Ile-Ile-1-Nal;
   Ac-D-His-Leu-D-Asp-Ile-D-Ile-Trp;
   Ac-D-Phe-Leu-D-Asp-Ile-D-Ile-Trp;
   Ac-D-Bip-Leu-D-Asp-Ile-D-Ile-Trp;
   Ac-D-Dip-Leu-D-Asp-Ile-D-Ile-Trp;
   Ac-D-2-Nal-Leu-D-Asp-Ile-D-Ile-Trp;
   Ac-D-1-Nal-Leu-D-Asp-Ile-D-Ile-Trp;
   Ac-D-Trp-Leu-D-Asp-Ile-D-Ile-Trp;
   Ac-D-Dip-Asn-Ile-Ile-Trp;
   Ac-D-Dip-Phe-Ile-Ile-Trp;
   Ac-D-Dip-Ile-Ile-Trp;
   Ac-D-Dip-Asp-Ile-Ile-Trp;
   Ac-D(NMe)-Dip-Leu-Asp-Ile-Ile-Trp;
   Ac-D-Dip-Leu-Asp-Ile-Ile-(NMe)Trp;
   Ac-D-Dip-Leu-Asp-Ile-(NMe)-Ile-Trp;
   Ac-D-Dip-Leu-Asp-(NMe)Ile-Ile-Trp;
   Ac-D-Dip-Leu-(NMe)Asp-Ile-Ile-Trp;
   Ac-D-Dip-(NMe)Leu-Asp-Ile-Ile-Trp;
   Ac-D-Phe-Asp-Ile-Ile-Trp;
   Ac-D-His-Asp-Ile-Ile-Trp;
   Ac-D-Trp-Asp-Ile-Ile-Trp;
   Ac-D-Tyr-Asp-Ile-Ile-Trp;
   Ac-D-Tyr(OMe)-Asp-Ile-Ile-Trp;
   Ac-D-Tyr(OEt)-Asp-Ile-Ile-Trp;
   Ac-D-2-Nal-Asp-Ile-Ile-Trp;
   Ac-D-1-Nal-Asp-Ile-Ile-Trp;
   Ada-D-Phe-Asp-Ile-Ile-Trp;
   Ada-D-His-Asp-Ile-Ile-Trp;
   Ada-D-Trp-Asp-Ile-Ile-Trp;
   Ada-D-Tyr-Asp-Ile-Ile-Trp;
   Ada-D-Tyr(OMe)-Asp-Ile-Ile-Trp;
   Ada-D-Tyr(OEt)-Asp-Ile-Ile-Trp;
   Ada-D-2-Nal-Asp-Ile-Ile-Trp;
   Ada-D-1-Nal-Asp-Ile-Ile-Trp;
   Ada-D-Dip-Asp-Ile-Ile-Trp;
   Ada-D-Bip-Asp-Ile-Ile-Trp;
   Ac-D-Phe-Asp-Ile-Ile-2-Nal;
   Ac-D-Phe-Asp-Ile-Ile-1-Nal;
   Ac-D-His-Asp-Ile-Ile-2-Nal;
   Ac-D-His-Asp-Ile-Ile-1-Nal;
   Ac-D-Tyr-Asp-Ile-Ile-2-Nal;
   Ac-D-Tyr-Asp-Ile-Ile-1-Nal;
   Ac-D-Trp-Asp-Ile-Ile-2-Nal;
   Ac-D-Trp-Asp-Ile-Ile-1-Nal;
   Ac-D-Dip-Asp-Ile-Ile-2-Nal;
   Ac-D-Dip-Asp-Ile-Ile-1-Nal;
   Ac-D-Bip-Asp-Ile-Ile-2-Nal;
   Ac-D-Bip-Asp-Ile-Ile-1-Nal;
   Ac-D-Phe-Leu-Asp-Ile-Trp;
   Ac-D-His-Leu-Asp-Ile-Trp;
   Ac-D-Tyr-Leu-Asp-Ile-Trp;
   Ac-D-Dip-Leu-Asp-Ile-Trp;
   Ac-D-Trp-Leu-Asp-Ile-Trp;
   Ac-DPhe-Leu-Asp-Ile-Ile-Trp-Gly;
   Ac-DHis-Leu-Asp-Ile-Ile-Trp-Gly;
   Ac-DTrp-Leu-Asp-Ile-Ile-Trp-Gly;
   Ac-DTyr(OMe)-Leu-Asp-Ile-Ile-Trp-Gly;
   Ac-DTyr(OEt)-Leu-Asp-Ile-Ile-Trp-Gly;
   Ac-D2Nal-Leu-Asp-Ile-Ile-Trp-Gly;
   Ac-D3Nal-Leu-Asp-Ile-Ile-Trp-Gly;
   Ac-DDip-Leu-Asp-Ile-Ile-Trp-Gly;
   Ac-DBip-Leu-Asp-Ile-Ile-Trp-Gly;
   Ac-DPhe-Leu-Asp-Ile-Ile-Trp-Asp;
   Ac-DHis-Leu-Asp-Ile-Ile-Trp-Asp;
   Ac-DTrp-Leu-Asp-Ile-Ile-Trp-Asp;
   Ac-DTyr(OMe)-Leu-Asp-Ile-Ile-Trp-Asp;
   Ac-DTyr(OEt)-Leu-Asp-Ile-Ile-Trp-Asp;
   Ac-D2Nal-Leu-Asp-Ile-Ile-Trp-Asp;
   Ac-D3Nal-Leu-Asp-Ile-Ile-Trp-Asp;
   Ac-DDip-Leu-Asp-Ile-Ile-Trp-Asp; and
   Ac-DBip-Leu-Asp-Ile-Ile-Trp-Asp-Asp;
   or a pharmaceutically acceptable acid or base addition salt thereof.

The compounds of Formula I are valuable antagonists of endothelin. The tests employed indicate that compounds of Formula I possess endothelin antagonist activity. Thus, the compounds of Formula I were tested for their ability to inhibit [¹²⁵I]-ET-1([¹²⁵I]-Endothelin-1) binding in a receptor assay. The binding of the compounds of Formula I is determined by incubation (37°C, 2 hours) of a compound of Formula I with [¹²⁵I]-ET-1 and the tissue (rat heart ventricle (10 µg)) in 50 mM Tris(hydroxymethyl)aminomethane hydrochloride (Tris-HCl) (pH 7.4), 5 mM ethylenediamine tetraacetic acid (EDTA), 2 mM ethylene glycol bis(β-aminoethyl ether)N,N,N',N'-tetraacetic acid (EGTA), 100 µM phenylmethylsulfonyl fluoride (PMSF), and 100 µM bacitracin containing protease inhibitors (total volume of 0.5 mL). IC₅₀ values are calculated by weighing nonlinear regression curve-fitting to the mass-action (Langmuir) equation. The functional activity of compounds of Formula I is determined in Rat-1 cells by measuring intracellular levels of second messengers. Thus, cells were prelabeled with [³H]-inositol and endothelin-stimulated accumulation of total [³H]-inositol phosphates in the presence of Li⁺ is monitored using anion exchange chromatography as described by Muldoon, L. L., et al, Journal of Biological Chemistry, Volume 264, pages 8529-8536 (1989) and Dudley, D. T., et al, Molecular Pharmacology, Volume 38, pages 370-377 (1990). Antagonist activity is assessed as the ability of added compounds to reduce endothelin-stimulated inositol phosphate accumulation. Antagonist activity was also measured by the ability of added compounds to reduce endothelin-stimulated arachidonic acid release in cultured vascular smooth muscle cells as (AAR) described in Reynolds, E., Mok, K., Faseb J., 1991, 5, A1066. The data in the table show the endothelin antagonist activity of representative compounds of Formula I.

**TABLE 1**

| Biological Activity of Compounds of Formula I | | | | |
|---|---|---|---|---|
| Example Number | Compound | Binding Assay in Rat Heart Ventricle IC₅₀ (µM) or % Inhibition | IP (Inositol Phosphate) Accumulation, IC₅₀ (µM) or % Inhibition | AAR IC₅₀ (µM) |
| 1 | Ac-D-Phe-Leu-Asp-Ile-Ile-Trp | 0.72 | 0.86 | |
| 4 | D-1-Nal-Leu-Asp-Ile-Ile-Trp | 8.98 | 53% @ 50 µM | |
| 5 | Ac-D-1-Nal-Leu-Asp-Ile-Ile-Trp | 1.63 | 0.63 | 1.9 |
| 6 | Ac-D-Phe-Leu-Asp-Ile-Trp | 24.5 | | |
| 7 | Ac-D-His-Leu-D-Asp-Ile-D-Ile-Trp | 6.03 | | |
| 8 | Ac-D-Phe-Orn-Asp-Ile-Ile-Trp | 0.68 | 0.43 | |
| 9 | Ac-D-Phe-Glu-Asp-Ile-Ile-Trp | 0.74 | | 0.60 |
| 10 | Ac-D-Tyr-Leu-Asp-Ile-Ile-Trp | 0.70 | 0.43 | 0.25 |
| 11 | Ac-D-Phe-Asp-Ile-Ile-Trp | 2.15 | | |
| 12 | Fmoc-D-Phe-Leu-Asp-Ile-Ile-Trp | 0.43 | | |
| 13 | Ac-D-Dip-Leu-Asp-Ile-Ile-Trp | 0.015 | | 0.07 |
| 16 | Ac-D-Dip-Leu-Phe-Ile-Ile-Trp | 0.047 | | 1.8 |
| 17 | Ac-D-Dip-Leu-Asp-Ile-Lys-Tsp | 24.6% @ 10 µM | | |
| 18 | Ac-D-Dip-Leu-Asp-Ile-Glu-Trp | 37.0% @ 10 µM | | |
| 19 | Ac-D-Dip-Leu-Asp-Glu-Ile-Trp | 0.015 | | |
| 20 | Ac-D-Dip-Glu-Asp-Ile-Ile-Trp | 0.085 | | |
| 21 | Ac-D-Dip-Orn-Asp-Ile-Ile-Trp | 79% @ 0.05 µM | | 0.02 |
| 23 | Ac-D-Dip-D-Leu-Asp-Ile-Ile-Trp | 41.3% @ 10 µM | | |

### General Method for Preparing Compounds of Formula I

The compounds of Formula I may be prepared by solid phase peptide synthesis on a peptide synthesizer, for example, an Applied Biosystems 430A peptide synthesizer using activated esters or anhydrides of N-alpha-Boc protected amino acids, on PAM or MBHA resins. Additionally, the compounds of Formula I may also be prepared by conventional solution peptide synthesis. Amino acid side chains are protected as follows; Bzl(Asp, Glu, Ser), 2-Cl-Z(Lys), 2-Br-Z(Tyr), Bom(His), For(Trp), and MeBzl(Cys). Each peptide resin (1.0 g) is cleaved with 9 ml of HF and 1 mL of anisole or p-cresol as a scavenger (60 minutes, 0°C). The peptide resin is washed with cyclohexane, extracted with 30% aqueous HOAc, followed by glacial HOAc, concentrated under reduced pressure, and lyophilized. (A peptide containing For(Trp) is dissolved in 0°C, the pH is adjusted to 12.5 with 1N KOH (2 minutes), neutralized with glacial HOAc, desalted on C₁₈ (as described below), and lyophilized. The crude peptide is purified by preparative reversed phase high performance liquid chromatography (RP-HPLC) on a C₁₈ column (2.2 x 25.0 cm, 15.0 mL/min) with a linear gradient of 0.1% TFA in water to 0.1% TFA in acetonitrile and lyophilized. The homogeneity and composition of the resulting peptide is verified by RP-HPLC, capillary electrophoresis, thin layer chromatography (TLC), proton nuclear magnetic resonance spectrometry (NMR), and fast atom bombardment mass spectrometry (FAB-MS).

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Also, the compounds of the present invention can be administered by inhalation, for example, intranasally. Additionally, the compounds of the present invention can be administered transdermally. It will be obvious to those skilled in the art that the following dosage forms may comprise as the active component, either a compound of Formula I or a corresponding pharmaceutically acceptable salt of a compound of Formula I.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form.

The quantity of active component in a unit dose preparation may be varied or adjusted from 0.1 mg to 100 mg preferably 0.5 mg to 100 mg according to the particular application and the potency of the active component. The composition can, if desired, also contain other compatible therapeutic agents.

In therapeutic use as antagonist of endothelin, the compounds utilized in the pharmaceutical method of this invention are administered at the initial dosage of about 0.01 mg to about 20 mg per kilogram daily. A daily dose range of about 0.01 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day, if desired.

The following nonlimiting examples illustrate the inventors' preferred methods for preparing the compounds of the invention.

### EXAMPLE 1

### Ac-D-Phe-Leu-Asp-Ile-Ile-Trp

The linear hexapeptide is prepared by standard solid phase synthetic peptide methodology utilizing a Boc/benzyl strategy (Stewart, J. M. and Young, J. D., Solid Phase Peptide Synthesis, Pierce Chemical Co., Rockford, IL, 1984). All protected amino acids and reagents are obtained from commercial sources and are not further purified. The protected peptide resin is prepared on an Applied Biosystems 430A Peptide Synthesizer, utilizing protocols supplied for a dicyclohexylcarbodiimide mediated coupling scheme (Standard 1.0, Version 1.40). Starting with 0.560 g of N-α-Boc-Trp(For)-PAM resin (0.88 meq/g, 0.43 meq of Boc-Trp(For) total) the protected peptide is prepared by the stepwise coupling of the following amino acids (in order of addition): N-α-Boc-D-Phe, N-α-Boc-Leu·H₂O, N-α-Boc-Asp(Bzl), and N-α-Boc-Ile·0.5 H₂O. A typical cycle for the coupling of an individual amino acid residue is illustrated below (reproduced from the ABI manual):

All the single couple RV cycles conform to the following pattern:
1) 33% TFA in DCM for 80 seconds
2) 50% TFA in DCM for 18.5 minutes
3) Three DCM washes
4) 10% DIEA in DMF for 1 minute
5) 10% DIEA in DMF for 1 minute
6) Five DMF washes
7) Coupling period
8) five DCM washes

After the coupling of N-α-Boc-D-Phe, the Boc group is removed with the end-NH₂ cycle and the free amine is acetylated with N-acetylimidazole (1.0 g, 120 minutes) in 20 mL of dichloromethane (DCM). The resin is washed with DCM (3 x 20 mL) and dried under reduced pressure (0.878 g).

The peptide is liberated from the solid support, and the carboxylate of aspartic acid deprotected by treatment with anhydrous hydrogen fluoride (9.0 mL), anisole (1.0 mL), and dimethyl sulfide (0.5 mL) (60 minutes, 0°C). After removing the hydrogen fluoride under a stream of nitrogen, the resin is washed with diethyl ether (3 x 30 mL) and extracted with 20% HOAc in water (3 x 30 mL) and glacial HOAc (2 x 30 mL). The aqueous extractions are combined, concentrated under reduced pressure, and lyophilized (320 mg). To remove the formyl protecting group, the crude peptide is suspended in 50 mL of aqueous 0.1 N KOH at 0°C for 2 minutes. The pH of the solution is adjusted to less than 4.0 with 10% HCl/H₂O and passed through a C 18 (60 cc) cartridge. The cartridge is washed with H₂O (50 mL), eluted with 0.1% TFA, 70% CH₃CN in H₂O, the eluants combined, concentrated under reduced pressure (10 mL), diluted with H₂O, and lyophilized to yield 153 mg of a white powder. The crude peptide is dissolved in 4.0 mL of 50% TFA/H₂O, filtered through a 0.4 µM syringe filter, and chromatographed on a Vydac 218TP 1022 column (2.2 x 25.0 cm, 15.0 mL/min, A: 0.1% TFA/H₂O, B: 0.1% TFA/CH₃CN, Gradient; 0% B for 10 minutes, 10% to 50% B over 120 minutes). Individual fractions are collected and combined based upon analysis by analytical HPLC. The combined fractions are concentrated under reduced pressure (10 mL), diluted with H₂O (50 mL), and lyophilized (14.8 mg). The homogeneity and structure of the resulting peptide is confirmed by analytical HPLC, capillary zone electrophoresis, Proton Nuclear Magnetic Resonance Spectroscopy (H¹-NMR) and Fast Atom Bombardment Mass Spectroscopy (FAB-MS), MH⁺ 848.4.

In a process analogous to Example 1 using the appropriate amino acids, the corresponding compounds of Formula I are prepared as follows:

### EXAMPLE 2

D-2-Nal-Leu-Asp-Ile-Ile-Trp; FAB-MS, MH⁺ 856.3.

### EXAMPLE 3

Ac-D-2-Nal-Leu-Asp-Ile-Ile-Trp; FAB-MS, MH⁺ 898.5.

### EXAMPLE 4

D-1-Nal-Leu-Asp-Ile-Ile-Trp; MH⁺ 856.3, MNa⁺ 878.2.

### EXAMPLE 5

Ac-D-1-Nal-Leu-Asp-Ile-Ile-Trp; MH⁺ 898.5, MNa⁺ 920.5.

### EXAMPLE 6

Ac-D-Phe-Leu-Asp-Ile-Trp; MH⁺ 735.5, MNa⁺ 757.8.

### EXAMPLE 7

Ac-D-His-Leu-D-Asp-Ile-D-Ile-Trp; MH⁺ 838.5, MNa⁺ 860.40.

### EXAMPLE 8

Ac-D-Phe-Orn-Asp-Ile-Ile-Trp; MH⁺ 849.1, MNa⁺ 871.0.

### EXAMPLE 9

Ac-D-Phe-Glu-Asp-Ile-Ile-Trp; MH⁺ 864.1, MNa⁺ 886.0.

### EXAMPLE 10

Ac-D-Tyr-Leu-Asp-Ile-Ile-Trp; MH⁺ 864.0, MNa⁺ 886.3.

### EXAMPLE 11

Ac-D-Phe-Asp-Ile-Ile-Trp; MH⁺ 735.1, MNa⁺ 757.3.

### EXAMPLE 12

Fmoc-D-Phe-Leu-Asp-Ile-Ile-Trp; MH⁺ 1028.1, MNa⁺ 1050.3.

### EXAMPLE 13

Ac-D-Dip-Leu-Asp-Ile-Ile-Trp; FAB-MS, MNa⁺ 946.6.

### EXAMPLE 14

Ac-D-Dip-Ile-Ile-Trp; FAB-MS, MH⁺ 696.5, MNa⁺ 718.5.

### EXAMPLE 15

Ac-D-Dip-Asp-Ile-Ile-Trp; FAB-MS, MH⁺ 810.4, MNa⁺ 833.5.

### EXAMPLE 16

Ac-D-Dip-Leu-Phe-Ile-Ile-Trp; FAB-MS, MNa⁺ 978.3.

### EXAMPLE 17

Ac-D-Dip-Leu-Asp-Ile-Lys-Trp; FAB-MS, MH⁺ 939.6.

### EXAMPLE 18

Ac-D-Dip-Leu-Asp-Ile-Glu-Trp; FAB-MS, MH⁺ 940.9, MNa⁺ 963.3.

### EXAMPLE 19

Ac-D-Dip-Leu-Asp-Glu-Ile-Trp; FAB-MS, MH⁺ 938.2.

### EXAMPLE 20

Ac-D-Dip-Glu-Asp-Ile-Ile-Trp; FAB-MS, MH⁺ 940.5.

### EXAMPLE 21

Ac-D-Dip-Orn-Asp-Ile-Ile-Trp; FAB-MS, MH⁺ 925.1.

### EXAMPLE 22

Ac-D-Dip-Leu-Asp(NMe)-Ile-Ile-Trp; FAB-MS, MNa⁺ 960.7.

### EXAMPLE 23

Ac-D-Dip-D-Leu-Asp-Ile-Ile-Trp; FAB-MS, MH⁺ 924.12, M⁺Na 946.0.

### EXAMPLE 24

### N-Methyl-glucamine salt of Ac-D-Phe-Leu-Asp-Ile-Ile-Trp

A saturated solution of N-methyl-glucamine in water is prepared, diluted with water (1:10), chilled to 0°C, and 10 mL of the solution is added to 20.45 mg of Ac-D-Phe-Leu-Asp-Ile-Ile-Trp (Example 1) with stirring. The pH of the solution is greater than 9. After 10 minutes, the solution is passed through a C18 cartridge, washed with water (100 mL), and the absorbed peptide is eluted with methanol (50 mL), concentrated under reduced pressure, resuspended in water (50 mL), and lyophilized (three times) to give the title compound.

## Claims

1. A compound of Formula I
AA¹-AA²-AA³-AA⁴-AA⁵-AA⁶
in which
AA¹ is 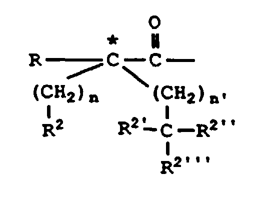 wherein
R is
hydrogen,
C₁-C₁₂ alkyl,
C₂-C₁₂ alkenyl,
C₂-C₁₂ alkynyl,
C₃-C₁₂ cycloalkyl,
C₃-C₁₂ cycloalkyl C₁-C₁₂alkyl,
an aromatic radical which is a phenyl groups a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 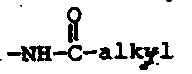 wherein alkyl is as defined above, 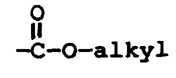 wherein alkyl is as defined above, 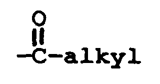 wherein alkyl is as defined above, or aryl, or
heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 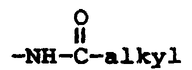 wherein alkyl is as defined above, 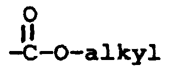 wherein alkyl is as defined above, 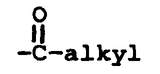 wherein alkyl is as defined above or phenyl,
fluorenylmethyl, 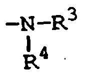 wherein R³ and R⁴ are each the same or different and each is
hydrogen,
C₁-C₁₂ alkyl,
C₂-C₁₂ alkenyl,
C₂-C₁₂ alkynyl,
C₃-C₁₂ cycloalkyl,
C₃-C₁₂ cycloalkyl C₁-C₁₂alkyl,
an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 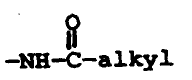 wherein alkyl is as defined above, 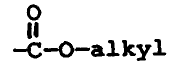 wherein alkyl is as defined above, 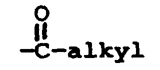 wherein alkyl is as defined above, or aryl, or
heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isozazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or
7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 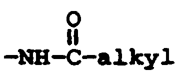 wherein alkyl is as defined above, 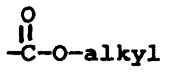 wherein alkyl is as defined above, 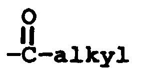 wherein alkyl is as defined above or phenyl, or
fluoroenylmethyl, 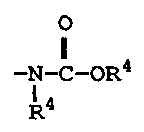 wherein R³ and R⁴ are each the same or different and each is as defined above, and R^{2'}, R^{2''}, and R^{2'''} are each the same or different and each is
hydrogen,
C₁-C₁₂ alkyl,
an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 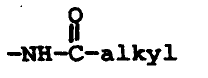 wherein alkyl is as defined above, 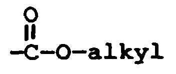 wherein alkyl is as defined above, 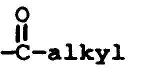 wherein alkyl is as defined above, or aryl, or
heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or
4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 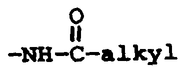 wherein alkyl is as defined above, 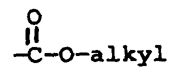 wherein alkyl is as defined above, 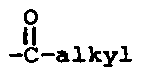 wherein alkyl is as defined above or phenyl, with the proviso that at least one of R², R^{2'}, and R^{2''} is aryl or heteroaryl as defined above, and R^{2'''} is hydrogen or methyl,
R² is hydrogen or methyl,
n is zero, and
n' is zero or an integer of 1, 2, or 3,
AA² is 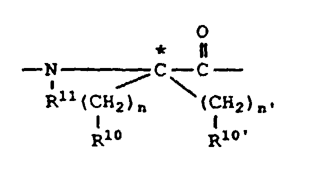 wherein
R¹¹ is hydrogen or methyl,
n is zero,
R¹⁰ is hydrogen or methyl,
n' is zero or an integer of 1, 2, 3, or 4, and
R¹⁰ is C₁-C₁₂ alkyl,
OH, 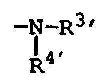 wherein R^{3'} and R^{4'} are each the same or different and each is
hydrogen,
C₁-C₁₂ alkyl, or
an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 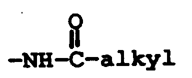 wherein alkyl is as defined above, 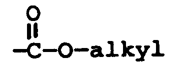 wherein alkyl is as defined above, 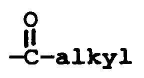 wherein alkyl is as defined above, or aryl, 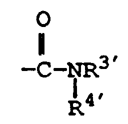 wherein R^{3'} and R^{4'} are as defined above, 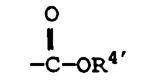 wherein R^{4'} is as defined above;
AA³ is 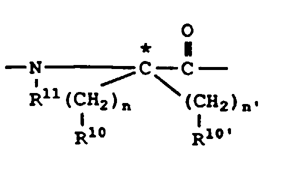 wherein
R¹¹ is hydrogen or methyl,
n is zero,
R¹⁰ is hydrogen or methyl,
n'' is zero or an integer of 1, 2, or 3, and
R^{11'} is C₁-C₁₂ alkyl,
an aromatic radical which is a phenyl group, a benzyl group, a naphthyl group, a biphenyl group, a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 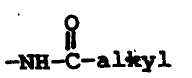 wherein alkyl is as defined above, 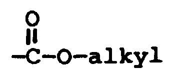 wherein alkyl is as defined above, 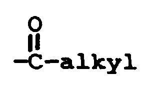 wherein alkyl is as defined above, or aryl, 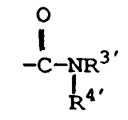 wherein R^{3'} and R^{4'} are as defined above, 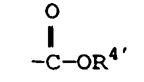 wherein R^{4'} is as defined above,
AA⁴ and AA⁵ are each 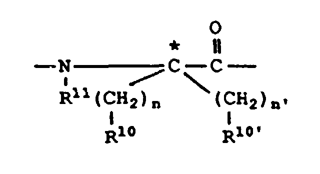 wherein
R¹¹ is hydrogen or methyl,
n is zero,
R¹⁰ is hydrogen or methyl,
n' is zero, and
R^{10'} is C₁-C₁₂ alkyl,
or C₃-C₁₂ cycloalkyl,
AA⁶ is 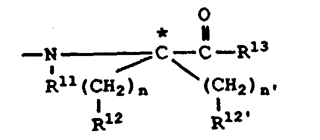 wherein
R¹¹ is hydrogen or methyl,
n is zero,
R¹² is hydrogen, or methyl,
n' is zero or an integer of 1, 2, of 3,
R^{12'} is
an aromatic radical which is a phenyl groups a benzyl group, a naphthyl group, a biphenyl groups a pyrenyl group, an anthracenyl group, or a fluorenyl group, unsubstituted or substituted by 1 to 4 substituents selected from alkyl as defined above, alkoxy as defined above, thioalkoxy as defined above, hydroxy, thiol, nitro, halogen, amino, 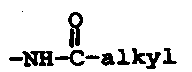 wherein alkyl is as defined above, 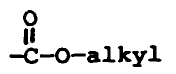 wherein alkyl is as defined above, 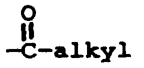 wherein alkyl is as defined above, or aryl, or
heteroaromatic radical which is 2- or 3-thienyl, 2- or 3-furanyl, 2-or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2,3-triazolyl, tetrazolyl, 2-, 3-, or 4-pyridinyl, 3-, 4-, or 5-pyridazinyl, 2-pyrazinyl, 2-, 4-, or 5-pyrimidinyl, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, or 2-, 4-, 5-, 6-, or 7-benzoxazolyl, 2-, 4-, 5-, 6-, or 7-benzimidazolyl, 2-, 4-, 5-, 6-, or 7-benzothiazolyl, unsubstituted or substituted by 1 to 2 substituents selected from alkyl as defined above, aryl as defined above, alkoxy as defined above, thioalkoxy as defined above, hydroxy, thiol, nitro, halogen, formyl, amino, 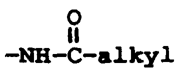 wherein alkyl is as defined above, 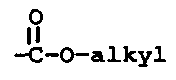 wherein alkyl is as defined above, 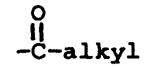 wherein alkyl is as defined above or phenyl,
R¹³ is
-(CH₂)ₙ-CO₂H wherein n is zero or an integer of 1, 2, 3, 4, 5, or 6,
-(CH₂)ₙ-OH wherein n is zero or an integer of 1, 2, 3, 4, 5, or 6, or 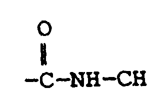 wherein R¹⁴ is hydrogen or
-CH₂CO₂H,
stereochemistry at 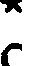 in AA¹ is D,
stereochemistry at 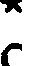 in AA², AA³, AA⁴, or AA⁵ is D or L and
stereochemistry at 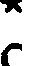 in AA⁶ is L.

2. A compound according to Claim 1 in which
AA¹ is
D-Dip,
D-Bip,
D-His,
D-His(Dnp),
D-2-Nal,
D-1-Nal,
D-Phe,
D-Pgl,
D-Tyr,
D-Tyr(OMe),
D-Tyr(OEt),
D-Tyr(OtBu),
D-Trp,
D-Trp(For),
D-Tic,
D-Tza,
D-Pyr,
Ac-D-Dip,
Ac-D-Bip,
Ac-D-His,
Ac-D-His(Dnp),
Ac-D-2-Nal,
Ac-D-1-Nal,
Ac-D-Phe,
Ac-D-Pgl,
Ac-D-Tyr,
Ac-D-Tyr(OMe),
Ac-D-Tyr(OEt),
Ac-D-Tyr(OtBu),
Ac-D-Trp,
Ac-D-Trp(For),
Ac-D-Tic,
Ac-D-Tza,
Ac-D-Pyr,
Ada-D-Dip,
Ada-D-Bip,
Ada-D-His,
Ada-D-His(Dnp),
Ada-D-2-Nal,
Ada-D-1-Nal,
Ada-D-Phe,
Ada-D-Pgl,
Ada-D-Tyr,
Ada-D-Tyr(OMe),
Ada-D-Tyr(OEt),
Ada-D-Tyr(OtBu),
Ada-D-Trp,
Ada-D-Trp(For),
Ada-D-Tic,
Ada-D-Tza,
Ada-D-Pyr,
Adoc-D-Dip,
Adoc-D-Bip,
Adoc-D-His,
Adoc-D-His(Dnp),
Adoc-D-2-Nal,
Adoc-D-1-Nal,
Adoc-D-Phe,
Adoc-D-Pgl,
Adoc-D-Tyr,
Adoc-D-Tyr(OMe),
Adoc-D-Tyr(OEt),
Adoc-D-Tyr(OtBu),
Adoc-D-Trp,
Adoc-D-Trp(For),
Adoc-D-Tic,
Adoc-D-Tza,
Adoc-D-Pyr,
Boc-D-Dip,
Boc-D-Bip,
Boc-D-His,
Boc-D-His(Dnp),
Boc-D-2-Nal,
Boc-D-1-Nal,
Boc-D-Phe,
Boc-D-Pgl,
Boc-D-Tyr,
Boc-D-Tyr(OMe),
Boc-D-Tyr(OEt),
Boc-D-Tyr(OtBu),
Boc-D-Trp,
Boc-D-Trp(For),
Boc-D-Tic,
Boc-D-Tza,
Boc-D-Pyr,
Z-D-Dip,
Z-D-Bip,
Z-D-His,
Z-D-His(Dnp),
Z-D-2-Nal,
Z-D-1-Nal,
Z-D-Phe,
Z-D-Pgl,
Z-D-Tyr,
Z-D-Tyr(OMe),
Z-D-Tyr(OEt),
Z-D-Tyr(OtBu),
Z-D-Trp,
Z-D-Trp(For),
Z-D-Tic,
Z-D-Tza,
Z-D-Pyr,
Fmoc-D-Dip,
Fmoc-D-Bip,
Fmoc-D-His,
Fmoc-D-His(Dnp),
Fmoc-D-2-Nal,
Fmoc-D-1-Nal,
Fmoc-D-Phe,
Fmoc-D-Pgl,
Fmoc-D-Tyr,
Fmoc-D-Tyr(OMe),
Fmoc-D-Tyr(OEt),
Fmoc-D-Tyr(OtBu),
Fmoc-D-Trp,
Fmoc-D-Trp(For),
Fmoc-D-Tic,
Fmoc-D-Tza,
Fmoc-D-Pyr, or
AA² is Ala,
Alg,
Arg
Asn,
Asp,
Dab,
Glu,
Gln,
Gly,
homoArg,
homoGlu,
homoLys,
Ile,
Leu,
D-Leu,
Lys,
Met,
Met(O),
Met(O₂),
Nva,
Nle,
Orn,
Phe,
Tyr,
Val, or
AA² is absent;
AA³ is Asn,
Asp,
D-Asp,
N-MeAsp,
Glu,
Gln,
homoPhe,
Phe,
Tyr, or
AA³ is absent;
AA⁴ is Ala,
Chx,
Gly,
Glu,
Ile,
D-Ile,
Leu,
Nleu,
Nval,
Val, or
AA⁴ is absent;
AA⁵ is Ala,
Chx,
Gly,
Ile,
D-Ile,
Leu,
Nleu,
Nval,
Val, or
AA⁵ is absent; and
AA⁶ is 2-Nal,
1-Nal,
Pyr,
Trp,
Tyr(OMe),
Tyr(OEt),
Tyr(Ot-Bu),
Tyr,
Trp-Gly,
Trp-Asp,
Trp(For),
Dip,
Phe,
Bza, or

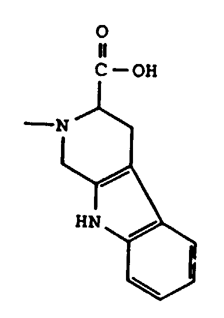

3. A compound according to Claim 2 selected from the groups consisting of:
D-Phe-Leu-Asp-Ile-Ile-Trp;
D-His(Dnp)-Leu-Asp-Ile-Ile-Trp;
D-Trp-Leu-Asp-Ile-Ile-Trp;
D-Tyr-Leu-Asp-Ile-Ile-Trp;
D-Tyr(OMe)-Leu-Asp-Ile-Ile-Trp;
D-Tyr(OEt)-Leu-Asp-Ile-Ile-Trp;
D-2-Nal-Leu-Asp-Ile-Ile-Trp;
D-1-Nal-Leu-Asp-Ile-Ile-Trp;
D-Pgl-Leu-Asp-Ile-Ile-Trp;
D-Pyr-Leu-Asp-Ile-Ile-Trp;
D-Tic-Leu-Asp-Ile-Ile-Trp;
D-Dip-Leu-Asp-Ile-Ile-Trp;
D-Bip-Leu-Asp-Ile-Ile-Trp;
Ac-D-Phe-Leu-Asp-Ile-Ile-Trp;
Ac-D-His(Dnp)-Leu-Asp-Ile-Ile-Trp;
Ac-D-Trp-Leu-Asp-Ile-Ile-Trp;
Ac-D-Tyr-Leu-Asp-Ile-Ile-Trp;
Ac-D-Tyr(OMe)-Leu-Asp-Ile-Ile-Trp;
Ac-D-Tyr(OEt)-Leu-Asp-Ile-Ile-Trp;
Ac-D-2-Nal-Leu-Asp-Ile-Ile-Trp;
Ac-D-1-Nal-Leu-Asp-Ile-Ile-Trp;
Ac-D-Pgl-Leu-Asp-Ile-Ile-Trp;
Ac-D-Pyr-Leu-Asp-Ile-Ile-Trp;
Ac-D-Tic-Leu-Asp-Ile-Ile-Trp;
Ac-D-Dip-Leu-Asp-Ile-Ile-Trp;
Ac-D-Bip-Leu-Asp-Ile-Ile-Trp;
Fmoc-D-Phe-Leu-Asp-Ile-Ile-Trp;
Fmoc-D-His-Leu-Asp-Ile-Ile-Trp;
Fmoc-D-Trp-Leu-Asp-Ile-Ile-Trp;
Fmoc-D-Tyr-Leu-Asp-Ile-Ile-Trp;
Fmoc-D-Tyr(OMe)-Leu-Asp-Ile-Ile-Trp;
Fmoc-D-Tyr(OEt)-Leu-Asp-Ile-Ile-Trp;
Fmoc-D-2-Nal-Leu-Asp-Ile-Ile-Trp;
Fmoc-D-1-Nal-Leu-Asp-Ile-Ile-Trp;
Fmoc-D-Dip-Leu-Asp-Ile-Ile-Trp;
Fmoc-D-Bip-Leu-Asp-Ile-Ile-Trp;
Ada-D-Phe-Leu-Asp-Ile-Ile-Trp;
Ada-D-His-Leu-Asp-Ile-Ile-Trp;
Ada-D-Trp-Leu-Asp-Ile-Ile-Trp;
Ada-D-Tyr-Leu-Asp-Ile-Ile-Trp;
Ada-D-Tyr(OMe)-Leu-Asp-Ile-Ile-Trp;
Ada-D-Tyr(OEt)-Leu-Asp-Ile-Ile-Trp;
Ada-D-2-Nal-Leu-Asp-Ile-Ile-Trp;
Ada-D-1-Nal-Leu-Asp-Ile-Ile-Trp;
Ada-D-Dip-Leu-Asp-Ile-Ile-Trp;
Ada-D-Bip-Leu-Asp-Ile-Ile-Trp;
Ac-D-Phe-D-Leu-Asp-Ile-Ile-Trp;
Ac-D-His-D-Leu-Asp-Ile-Ile-Trp;
Ac-D-Trp-D-Leu-Asp-Ile-Ile-Trp;
Ac-D-Tyr-D-Leu-Asp-Ile-Ile-Trp;
Ac-D-Tyr(OMe)-D-Leu-Asp-Ile-Ile-Trp;
Ac-D-Tyr(OEt)-D-Leu-Asp-Ile-Ile-Trp;
Ac-D-2-Nal-D-Leu-Asp-Ile-Ile-Trp;
Ac-D-1-Nal-D-Leu-Asp-Ile-Ile-Trp;
Ac-D-Dip-D-Leu-Asp-Ile-Ile-Trp;
Ac-D-Bip-D-Leu-Asp-Ile-Ile-Trp;
Ac-D-Phe-Ile-Asp-Ile-Ile-Trp;
Ac-D-His-Ile-Asp-Ile-Ile-Trp;
Ac-D-Trp-Ile-Asp-Ile-Ile-Trp;
Ac-D-Tyr-Ile-Asp-Ile-Ile-Trp;
Ac-D-Tyr(OMe)-Ile-Asp-Ile-Ile-Trp;
Ac-D-Tyr(OEt)-Ile-Asp-Ile-Ile-Trp;
Ac-D-2-Nal-Ile-Asp-Ile-Ile-Trp;
Ac-D-1-Nal-Ile-Asp-Ile-Ile-Trp;
Ac-D-Dip-Ile-Asp-Ile-Ile-Trp;
Ac-D-Bip-Ile-Asp-Ile-Ile-Trp;
Ac-D-Phe-Val-Asp-Ile-Ile-Trp;
Ac-D-His-Val-Asp-Ile-Ile-Trp;
Ac-D-Trp-Val-Asp-Ile-Ile-Trp;
Ac-D-Tyr-Val-Asp-Ile-Ile-Trp;
Ac-D-Tyr(OMe)-Val-Asp-Ile-Ile-Trp;
Ac-D-Tyr(OEt)-Val-Asp-Ile-Ile-Trp;
Ac-D-2-Nal-Val-Asp-Ile-Ile-Trp;
Ac-D-1-Nal-Val-Asp-Ile-Ile-Trp;
Ac-D-Dip-Val-Asp-Ile-Ile-Trp;
Ac-D-Bip-Val-Asp-Ile-Ile-Trp;
Ac-DPhe-Dab-Asp-Ile-Ile-Trp;
Ac-DHis-Dab-Asp-Ile-Ile-Trp;
Ac-DTrp-Dab-Asp-Ile-Ile-Trp;
Ac-DTyr(OMe)-Dab-Asp-Ile-Ile-Trp;
Ac-DTyr(OEt)-Dab-Asp-Ile-Ile-Trp;
Ac-D2Nal-Dab-Asp-Ile-Ile-Trp;
Ac-D3Nal-Dab-Asp-Ile-Ile-Trp;
Ac-DDip-Dab-Asp-Ile-Ile-Trp;
Ac-DBip-Dab-Asp-Ile-Ile-Trp;
Ac-DPhe-Arg-Asp-Ile-Ile-Trp;
Ac-DHis-Arg-Asp-Ile-Ile-Trp;
Ac-DTrp-Arg-Asp-Ile-Ile-Trp;
Ac-DTyr(OMe)-Arg-Asp-Ile-Ile-Trp;
Ac-DTyr(OEt)-Arg-Asp-Ile-Ile-Trp;
Ac-D2Nal-Arg-Asp-Ile-Ile-Trp;
Ac-D3Nal-Arg-Asp-Ile-Ile-Trp;
Ac-DDip-Arg-Asp-Ile-Ile-Trp;
Ac-DBip-Arg-Asp-Ile-Ile-Trp;
Ac-DPhe-hLys-Asp-Ile-Ile-Trp;
Ac-DHis-hLys-Asp-Ile-Ile-Trp;
Ac-DTrp-hLys-Asp-Ile-Ile-Trp;
Ac-DTyr(OMe)-hLys-Asp-Ile-Ile-Trp;
Ac-DTyr(OEt)-hLys-Asp-Ile-Ile-Trp;
Ac-D2Nal-hLys-Asp-Ile-Ile-Trp;
Ac-D3Nal-hLys-Asp-Ile-Ile-Trp;
Ac-DDip-hLys-Asp-Ile-Ile-Trp;
Ac-DBip-hLys-Asp-Ile-Ile-Trp;
Ac-DPhe-hGlu-Asp-Ile-Ile-Trp;
Ac-DHis-Glu-Asp-Ile-Ile-Trp;
Ac-DTrp-Glu-Asp-Ile-Ile-Trp;
Ac-DTyr(OMe)-Glu-Asp-Ile-Ile-Trp;
Ac-DTyr(OEt)-Glu-Asp-Ile-Ile-Trp;
Ac-D2Nal-Glu-Asp-Ile-Ile-Trp;
Ac-D3Nal-Glu-Asp-Ile-Ile-Trp;
Ac-DDip-Glu-Asp-Ile-Ile-Trp;
Ac-DBip-Glu-Asp-Ile-Ile-Trp;
Ac-DPhe-hGlu-Asp-Ile-Ile-Trp;
Ac-DHis-hGlu-Asp-Ile-Ile-Trp;
Ac-DTrp-hGlu-Asp-Ile-Ile-Trp;
Ac-DTyr(OMe)-hGlu-Asp-Ile-Ile-Trp;
Ac-DTyr(OEt)-hGlu-Asp-Ile-Ile-Trp;
Ac-D2Nal-hGlu-Asp-Ile-Ile-Trp;
Ac-D3Nal-hGlu-Asp-Ile-Ile-Trp;
Ac-DDip-hGlu-Asp-Ile-Ile-Trp;
Ac-DBip-hGlu-Asp-Ile-Ile-Trp;
Ac-DPhe-Asp-Asp-Ile-Ile-Trp;
Ac-DHis-Asp-Asp-Ile-Ile-Trp;
Ac-DTrp-Asp-Asp-Ile-Ile-Trp;
Ac-DTyr(OMe)-Asp-Asp-Ile-Ile-Trp;
Ac-DTyr(OEt)-Asp-Asp-Ile-Ile-Trp;
Ac-D2Nal-Asp-Asp-Ile-Ile-Trp;
Ac-D3Nal-Asp-Asp-Ile-Ile-Trp;
Ac-DDip-Asp-Asp-Ile-Ile-Trp;
Ac-DBip-Asp-Asp-Ile-Ile-Trp;
Ac-D-Phe-Lys-Asp-Ile-Ile-Trp;
Ac-D-His-Lys-Asp-Ile-Ile-Trp;
Ac-D-Trp-Lys-Asp-Ile-Ile-Trp;
Ac-D-Tyr-Lys-Asp-Ile-Ile-Trp;
Ac-D-Tyr(OMe)-Lys-Asp-Ile-Ile-Trp;
Ac-D-Tyr(OEt)-Lys-Asp-Ile-Ile-Trp;
Ac-D-2-Nal-Lys-Asp-Ile-Ile-Trp;
Ac-D-1-Nal-Lys-Asp-Ile-Ile-Trp;
Ac-D-Phe-Orn-Asp-Ile-Ile-Trp;
Ac-D-His-Orn-Asp-Ile-Ile-Trp;
Ac-D-Trp-Orn-Asp-Ile-Ile-Trp;
Ac-D-Tyr-Orn-Asp-Ile-Ile-Trp;
Ac-D-Tyr(OMe)-Orn-Asp-Ile-Ile-Trp;
Ac-D-Tyr(OEt)-Orn-Asp-Ile-Ile-Trp;
Ac-D-2-Nal-Orn-Asp-Ile-Ile-Trp;
Ac-D-1-Nal-Orn-Asp-Ile-Ile-Trp;
Ac-D-Phe-Gln-Asp-Ile-Ile-Trp;
Ac-D-His-Gln-Asp-Ile-Ile-Trp;
Ac-D-Trp-Gln-Asp-Ile-Ile-Trp;
Ac-D-Tyr-Gln-Asp-Ile-Ile-Trp;
Ac-D-Tyr(OMe)-Gln-Asp-Ile-Ile-Trp;
Ac-D-Tyr(OEt)-Gln-Asp-Ile-Ile-Trp;
Ac-D-2-Nal-Gln-Asp-Ile-Ile-Trp;
Ac-D-1-Nal-Gln-Asp-Ile-Ile-Trp;
Ac-D-Phe-Leu-Glu-Ile-Ile-Trp;
Ac-D-His-Leu-Glu-Ile-Ile-Trp;
Ac-D-Trp-Leu-Glu-Ile-Ile-Trp;
Ac-D-Tyr-Leu-Glu-Ile-Ile-Trp;
Ac-D-Tyr(OMe)-Leu-Glu-Ile-Ile-Trp;
Ac-D-Tyr(OEt)-Leu-Glu-Ile-Ile-Trp;
Ac-D-2-Nal-Leu-Glu-Ile-Ile-Trp;
Ac-D-1-Nal-Leu-Glu-Ile-Ile-Trp;
Ac-D-Phe-Leu-Asn-Ile-Ile-Trp;
Ac-D-His-Leu-Asn-Ile-Ile-Trp;
Ac-D-Trp-Leu-Asn-Ile-Ile-Trp;
Ac-D-Tyr-Leu-Asn-Ile-Ile-Trp;
Ac-D-Tyr(OMe)-Leu-Asn-Ile-Ile-Trp;
Ac-D-Tyr(OEt)-Leu-Asn-Ile-Ile-Trp;
Ac-D-2-Nal-Leu-Asn-Ile-Ile-Trp;
Ac-D-1-Nal-Leu-Asn-Ile-Ile-Trp;
Ac-D-Dip-Leu-Asn-Ile-Ile-Trp;
Ac-D-Bip-Leu-Asn-Ile-Ile-Trp;
Ac-DPhe-Leu-Phe-Ile-Ile-Trp;
Ac-DHis-Leu-Phe-Ile-Ile-Trp;
Ac-DTrp-Leu-Phe-Ile-Ile-Trp;
Ac-DTyr(OMe)-Leu-Phe-Ile-Ile-Trp;
Ac-DTyr(OEt)-Leu-Phe-Ile-Ile-Trp;
Ac-D2Nal-Leu-Phe-Ile-Ile-Trp;
Ac-D3Nal-Leu-Phe-Ile-Ile-Trp;
Ac-DDip-Leu-Phe-Ile-Ile-Trp;
Ac-DBip-Leu-Phe-Ile-Ile-Trp;
Ac-D-Phe-Glu-Asp-Ile-Ile-Trp;
Ac-D-Phe-Leu-Asp-Val-Ile-Trp;
Ac-D-His-Leu-Asp-Val-Ile-Trp;
Ac-D-Trp-Leu-Asp-Val-Ile-Trp;
Ac-D-Tyr-Leu-Asp-Val-Ile-Trp;
Ac-D-Tyr(OMe)-Leu-Asp-Val-Ile-Trp;
Ac-D-Tyr(OEt)-Leu-Asp-Val-Ile-Trp;
Ac-D-2-Nal-Leu-Asp-Val-Ile-Trp;
Ac-D-1-Nal-Leu-Asp-Val-Ile-Trp;
Ac-D-Dip-Leu-Asp-Val-Ile-Trp;
Ac-D-Bip-Leu-Asp-Val-Ile-Trp;
Ac-D-Phe-Leu-Asp-Chx-Ile-Trp;
Ac-D-His-Leu-Asp-Chx-Ile-Trp;
Ac-D-Trp-Leu-Asp-Chx-Ile-Trp;
Ac-D-Tyr-Leu-Asp-Chx-Ile-Trp;
Ac-D-Tyr(OMe)-Leu-Asp-Chx-Ile-Trp;
Ac-D-Tyr(OEt)-Leu-Asp-Chx-Ile-Trp;
Ac-D-2-Nal-Leu-Asp-Chx-Ile-Trp;
Ac-D-1-Nal-Leu-Asp-Chx-Ile-Trp;
Ac-D-Dip-Leu-Asp-Chx-Ile-Trp;
Ac-D-Bip-Leu-Asp-Chx-Ile-Trp;
Ac-D-Phe-Leu-Asp-D-Ile-Ile-Trp;
Ac-D-His-Leu-Asp-D-Ile-Ile-Trp;
Ac-D-Trp-Leu-Asp-D-Ile-Ile-Trp;
Ac-D-Tyr-Leu-Asp-D-Ile-Ile-Trp;
Ac-D-Tyr(OMe)-Leu-Asp-D-Ile-Ile-Trp;
Ac-D-Tyr(OEt)-Leu-Asp-D-Ile-Ile-Trp;
Ac-D-2-Nal-Leu-Asp-D-Ile-Ile-Trp;
Ac-D-1-Nal-Leu-Asp-D-Ile-Ile-Trp;
Ac-D-Dip-Leu-Asp-D-Ile-Ile-Trp;
Ac-D-Bip-Leu-Asp-D-Ile-Ile-Trp;
Ac-D-Phe-Leu-Asp-Ile-D-Ile-Trp;
Ac-D-His-Leu-Asp-Ile-D-Ile-Trp;
Ac-D-Trp-Leu-Asp-Ile-D-Ile-Trp;
Ac-D-Tyr-Leu-Asp-Ile-D-Ile-Trp;
Ac-D-Tyr(OMe)-Leu-Asp-Ile-D-Ile-Trp;
Ac-D-Tyr(OEt)-Leu-Asp-Ile-D-Ile-Trp;
Ac-D-2-Nal-Leu-Asp-Ile-D-Ile-Trp;
Ac-D-1-Nal-Leu-Asp-Ile-D-Ile-Trp;
Ac-D-Dip-Leu-Asp-Ile-D-Ile-Trp;
Ac-D-Bip-Leu-Asp-Ile-D-Ile-Trp;
Ac-D-Phe-Leu-Asp-Ile-Val-Trp;
Ac-D-His-Leu-Asp-Ile-Val-Trp;
Ac-D-Trp-Leu-Asp-Ile-Val-Trp;
Ac-D-Tyr-Leu-Asp-Ile-Val-Trp;
Ac-D-Tyr(OMe)-Leu-Asp-Ile-Val-Trp;
Ac-D-Tyr(OEt)-Leu-Asp-Ile-Val-Trp;
Ac-D-2-Nal-Leu-Asp-Ile-Val-Trp;
Ac-D-1-Nal-Leu-Asp-Ile-Val-Trp;
Ac-D-Dip-Leu-Asp-Ile-Val-Trp;
Ac-D-Bip-Leu-Asp-Ile-Val-Trp;
Ac-D-Phe-Leu-Asp-Ile-Chx-Trp;
Ac-D-His-Leu-Asp-Ile-Chx-Trp;
Ac-D-Trp-Leu-Asp-Ile-Chx-Trp;
Ac-D-Tyr-Leu-Asp-Ile-Chx-Trp;
Ac-D-Tyr(OMe)-Leu-Asp-Ile-Chx-Trp;
Ac-D-Tyr(OEt)-Leu-Asp-Ile-Chx-Trp;
Ac-D-2-Nal-Leu-Asp-Ile-Chx-Trp;
Ac-D-1-Nal-Leu-Asp-Ile-Chx-Trp;
Ac-D-Dip-Leu-Asp-Ile-Chx-Trp;
Ac-D-Bip-Leu-Asp-Ile-Chx-Trp;
Ac-D-Phe-Leu-Asp-Ile-Ile-2-Nal;
Ac-D-His-Leu-Asp-Ile-Ile-2-Nal;
Ac-D-Trp-Leu-Asp-Ile-Ile-2-Nal;
Ac-D-Tyr-Leu-Asp-Ile-Ile-2-Nal;
Ac-D-Tyr(OMe)-Leu-Asp-Ile-Ile-2-Nal;
Ac-D-Tyr(OEt)-Leu-Asp-Ile-Ile-2-Nal;
Ac-D-2-Nal-Leu-Asp-Ile-Ile-2-Nal;
Ac-D-1-Nal-Leu-Asp-Ile-Ile-2-Nal;
Ac-D-Dip-Leu-Asp-Ile-Ile-2-Nal;
Ac-D-Bip-Leu-Asp-Ile-Ile-2-Nal;
Ac-D-Phe-Leu-Asp-Ile-Ile-1-Nal;
Ac-D-His-Leu-Asp-Ile-Ile-1-Nal;
Ac-D-Trp-Leu-Asp-Ile-Ile-1-Nal;
Ac-D-Tyr-Leu-Asp-Ile-Ile-1-Nal;
Ac-D-Tyr(OMe)-Leu-Asp-Ile-Ile-1-Nal;
Ac-D-Tyr(OEt)-Leu-Asp-Ile-Ile-1-Nal;
Ac-D-2-Nal-Leu-Asp-Ile-Ile-1-Nal;
Ac-D-1-Nal-Leu-Asp-Ile-Ile-1-Nal;
Ac-D-Dip-Leu-Asp-Ile-Ile-1-Nal;
Ac-D-Bip-Leu-Asp-Ile-Ile-1-Nal;
Ac-D-His-Leu-D-Asp-Ile-D-Ile-Trp;
Ac-D-Phe-Leu-D-Asp-Ile-D-Ile-Trp;
Ac-D-Bip-Leu-D-Asp-Ile-D-Ile-Trp;
Ac-D-Dip-Leu-D-Asp-Ile-D-Ile-Trp;
Ac-D-2-Nal-Leu-D-Asp-Ile-D-Ile-Trp;
Ac-D-1-Nal-Leu-D-Asp-Ile-D-Ile-Trp;
Ac-D-Trp-Leu-D-Asp-Ile-D-Ile-Trp;
Ac-D-Dip-Asn-Ile-Ile-Trp;
Ac-D-Dip-Phe-Ile-Ile-Trp;
Ac-D-Dip-Ile-Ile-Trp;
Ac-D-Dip-Asp-Ile-Ile-Trp;
Ac-D(NMe)-Dip-Leu-Asp-Ile-Ile-Trp;
Ac-D-Dip-Leu-Asp-Ile-Ile-(NMe)Trp;
Ac-D-Dip-Leu-Asp-Ile-(NMe)Ile-Trp;
Ac-D-Dip-Leu-Asp-(NMe)Ile-Ile-Trp;
Ac-D-Dip-Leu-(NMe)Asp-Ile-Ile-Trp;
Ac-D-Dip-(NMe)Leu-Asp-Ile-Ile-Trp;
Ac-D-Phe-Asp-Ile-Ile-Trp;
Ac-D-His-Asp-Ile-Ile-Trp;
Ac-D-Trp-Asp-Ile-Ile-Trp;
Ac-D-Tyr-Asp-Ile-Ile-Trp;
Ac-D-Tyr(OMe)-Asp-Ile-Ile-Trp;
Ac-D-Tyr(OEt)-Asp-Ile-Ile-Trp;
Ac-D-2-Nal-Asp-Ile-Ile-Trp;
Ac-D-1-Nal-Asp-Ile-Ile-Trp;
Ada-D-Phe-Asp-Ile-Ile-Trp;
Ada-D-His-Asp-Ile-Ile-Trp;
Ada-D-Tip-Asp-Ile-Ile-Trp;
Ada-D-Tyr-Asp-Ile-Ile-Trp;
Ada-D-Tyr(OMe)-Asp-Ile-Ile-Trp;
Ada-D-Tyr(OEt)-Asp-Ile-Ile-Trp;
Ada-D-2-Nal-Asp-Ile-Ile-Trp;
Ada-D-1-Nal-Asp-Ile-Ile-Trp;
Ada-D-Dip-Asp-Ile-Ile-Trp;
Ada-D-Bip-Asp-Ile-Ile-Trp;
Ac-D-Phe-Asp-Ile-Ile-2-Nal;
Ac-D-Phe-Asp-Ile-Ile-1-Nal;
Ac-D-His-Asp-Ile-Ile-2-Nal;
Ac-D-His-Asp-Ile-Ile-1-Nal;
Ac-D-Tyr-Asp-Ile-Ile-2-Nal;
Ac-D-Tyr-Asp-Ile-Ile-1-Nal;
Ac-D-Trp-Asp-Ile-Ile-2-Nal;
Ac-D-Trp-Asp-Ile-Ile-1-Nal;
Ac-D-Dip-Asp-Ile-Ile-2-Nal;
Ac-D-Dip-Asp-Ile-Ile-1-Nal;
Ac-D-Bip-Asp-Ile-Ile-2-Nal;
Ac-D-Bip-Asp-Ile-Ile-1-Nal;
Ac-D-Phe-Leu-Asp-Ile-Trp;
Ac-D-His-Leu-Asp-Ile-Trp;
Ac-D-Tyr-Leu-Asp-Ile-Trp;
Ac-D-Dip-Leu-Asp-Ile-Trp;
Ac-D-Trp-Leu-Asp-Ile-Trp;
Ac-DPhe-Leu-Asp-Ile-Ile-Trp-Gly;
Ac-DHis-Leu-Asp-Ile-Ile-Trp-Gly;
Ac-DTrp-Leu-Asp-Ile-Ile-Trp-Gly;
Ac-DTyr(OMe)-Leu-Asp-Ile-Ile-Trp-Gly;
Ac-DTyr(OEt)-Leu-Asp-Ile-Ile-Trp-Gly;
Ac-D2Nal-Leu-Asp-Ile-Ile-Trp-Gly;
Ac-D3Nal-Leu-Asp-Ile-Ile-Trp-Gly;
Ac-DDip-Leu-Asp-Ile-Ile-Trp-Gly;
Ac-DBip-Leu-Asp-Ile-Ile-Trp-Gly;
Ac-DPhe-Leu-Asp-Ile-Ile-Trp-Asp;
Ac-DHis-Leu-Asp-Ile-Ile-Trp-Asp;
Ac-DTrp-Leu-Asp-Ile-Ile-Trp-Asp;
Ac-DTyr(OMe)-Leu-Asp-Ile-Ile-Trp-Asp;
Ac-DTyr(OEt)-Leu-Asp-Ile-Ile-Trp-Asp;
Ac-D2Nal-Leu-Asp-Ile-Ile-Trp-Asp;
Ac-D3Nal-Leu-Asp-Ile-Ile-Trp-Asp;
Ac-DDip-Leu-Asp-Ile-Ile-Trp-Asp; and
Ac-DBip-Leu-Asp-Ile-Ile-Trp-Asp-Asp.

4. A compound according to Claim 3 selected from the group consisting of:
Ac-D-Phe-Leu-Asp-Ile-Ile-Trp;
D-2-Nal-Leu-Asp-Ile-Ile-Trp;
Ac-D-2-Nal-Leu-Asp-Ile-Ile-Trp;
D-1-Nal-Leu-Asp-Ile-Ile-Trp;
Ac-D-Phe-Leu-Asp-Ile-Trp;
Ac-D-His-Leu-D-Asp-Ile-D-Ile-Trp;
Ac-D-Phe-Orn-Asp-Ile-Ile-Trp;
Ac-D-Phe-Glu-Asp-Ile-Ile-Trp;
Ac-D-Tyr-Leu-Asp-Ile-Ile-Trp;
Ac-D-Phe-Asp-Ile-Ile-Trp;
Fmoc-D-Phe-Leu-Asp-Ile-Ile-Trp;
Ac-D-Dip-Leu-Asp-Ile-Ile-Trp;
Ac-D-Dip-Ile-Ile-Trp;
Ac-D-Dip-Asp-Ile-Ile-Trp;
Ac-D-Dip-Leu-Phe-Ile-Ile-Trp;
Ac-D-Dip-Leu-Asp-Ile-Lys-Trp;
Ac-D-Dip-Leu-Asp-Ile-Glu-Trp;
Ac-D-Dip-Leu-Asp-Glu-Ile-Trp;
Ac-D-Dip-Glu-Asp-Ile-Ile-Trp;
Ac-D-Dip-Orn-Asp-Ile-Ile-Trp;
Ac-D-Dip-Leu-Asp(N-Me)-Ile-Ile-Trp; and
Ac-D-Dip-D-Leu-Asp-Ile-Ile-Trp.

5. A pharmaceutical composition comprising a compound according to Claims 1 to 4 in admixture with a pharmaceutically acceptable excipient, diluent, or carrier.

6. Use of a compound according to Claims 1 to 4 for the preparation of a pharmaceutical composition useful for treating hypertension, metabolic and endocrine disorders, congestive heart failure, myocardial infarction, endotoxic shock, subarachnoid hemorrhage, arrhythmias, asthma, acute renal failure, preeclampsia, diabetes and neurological disorders and for inhibiting elevated level of endothelin.

7. A process of preparing a compound according to claims 1 to 4 of Formula I
AA¹-AA²-AA³-AA⁴-AA⁵-AA⁶
wherein AA¹, AA², AA³, AA⁴, AA⁵ and AA⁶ have the above meaning or a pharmaceutically acceptable salt thereof comprising sequential stepwise coupling of the amino acids selected from AA¹, AA², AA³, AA⁴, AA⁵, or AA⁶ to the preceding amino acid using conventional peptide synthesis methodology and after conventional deprotection to afford a compound of Formula I and, if desired, converting a compound of Formula I to a pharmaceutically acceptable salt of a compound of Formula I by conventional methodology and, if further desired, converting the obtained pharmaceutically acceptably salt of a compound of Formula I to a compound of Formula I by conventional methodology.

## Patentansprüche

1. Verbindung der Formel I
AA¹-AA²-AA³-AA⁴-AA⁵-AA⁶
in welcher
AA¹ steht für 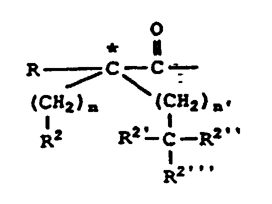 worin
R bedeutet:
Wasserstoff,
C₁-C₁₂-Alkyl,
C₂-C₁₂-Alkenyl,
C₂-C₁₂-Alkynyl,
C₃-C₁₂-Cycloalkyl,
C₃-C₁₂-Cycloalkyl-C₁-C₁₂-Alkyl,
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 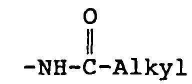 mit Alkyl wie vorstehend definiert, 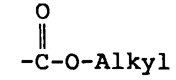 mit Alkyl wie vorstehend definiert, 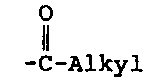 mit Alkyl wie vorstehend definiert, oder Aryl, oder
ein heteroaromatisches Radikal, bestehend aus einem Rest 2- oder 3-Thienyl, 2- oder 3-Furanyl, 2- oder 3-Pyrrolyl, 2-, 4- oder 5-Imidazolyl, 3-, 4 oder 5-Pyrazolyl, 2-, 4- oder 5-Thiazolyl, 3-, 4 oder 5-Isothiazolyl, 2-, 4- oder 5-Oxazolyl, 3-, 4 oder 5-Isoxazolyl, 3- oder 5-1,2,4,-Triazolyl, 4- oder 5-1,2,3-Triazolyl, Tetrazolyl, 2-, 3- oder 4-Pyridinyl, 3-, 4 oder 5-Pyridazinyl, 2-Pyrazinyl, 2-, 4- oder 5-Pyrimidinyl, 2-, 3-, 4-, 5-, 6-, 7- oder 8-Chinolinyl, 1-, 3-, 4-, 5-, 6-, 7- oder 8-Isochinolinyl, 2-, 3-, 4-, 5-, 6- oder 7-Indolyl, 2-, 3-, 4-, 5-, 6- oder 7-Benzo[b]thienyl, 2-, 4-, 5-, 6- oder 7-Benzoxazolyl, 2-, 4-, 5-, 6- oder 7-Benzothiazolyl,
der unsubstituiert ist oder substituiert ist mit 1 bis 2 Substituenten, die ausgewählt sind aus Alkyl wie vorstehend definiert, Aryl wie vorstehend definiert, Alkoxy wie vorstehend definiert, Thioalkoxy wie vorstehend definiert, Hydroxy, Thiol, Nitro, Halogen, Formyl, Amino, 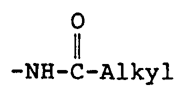 mit Alkyl wie vorstehend definiert, 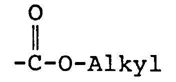 mit Alkyl wie vorstehend definiert, 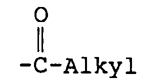 mit Alkyl wie vorstehend definiert, oder Phenyl, oder Fluorenylmethyl, 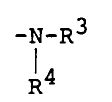
worin
R³ und R⁴ gleich oder verschieden sind und jedes davon bedeutet:
Wasserstoff,
C₁-C₁₂-Alkyl,
C₂-C₁₂-Alkenyl,
C₂-C₁₂-Alkynyl,
C₃-C₁₂-Cycloalkyl,
C₃-C₁₂-Cycloalkyl-C₁-C₁₂-Alkyl,
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 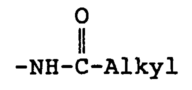 mit Alkyl
wie vorstehend definiert, 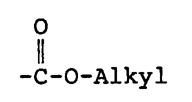 mit Alkyl
wie vorstehend definiert, 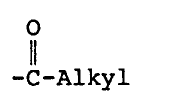 mit Alkyl
wie vorstehend definiert,
oder Aryl, oder
ein heteroaromatisches Radikal, bestehend aus einem Rest 2- oder 3-Thienyl, 2- oder 3-Furanyl, 2- oder 3-Pyrrolyl, 2-, 4- oder 5-Imidazolyl, 3-, 4 oder 5-Pyrazolyl, 2-, 4- oder 5-Thiazolyl, 3-, 4 oder 5-Isothiazolyl, 2-, 4- oder 5-Oxazolyl, 3-, 4 oder 5-Isoxazolyl, 3- oder 5-1,2,4,-Triazolyl, 4- oder 5-1,2,3-Triazolyl, Tetrazolyl, 2-, 3- oder 4-Pyridinyl, 3-, 4 oder 5-Pyridazinyl, 2-Pyrazinyl, 2-, 4- oder 5-Pyrimidinyl, 2-, 3-, 4-, 5-, 6-, 7- oder 8-Chinolinyl, 1-, 3-, 4-, 5-, 6-, 7- oder 8-Isochinolinyl, 2-, 3-, 4-, 5-, 6- oder 7-Indolyl, 2-, 3-, 4-, 5-, 6- oder 7-Benzo[b]thienyl, 2-, 4-, 5-, 6- oder 7-Benzoxazolyl, 2-, 4-, 5-, 6-oder 7-Benzothiazolyl,
der unsubstituiert ist oder substituiert ist mit 1 bis 2 Substituenten, die ausgewählt sind aus Alkyl wie vorstehend definiert, Aryl wie vorstehend definiert, Alkoxy wie vorstehend definiert, Thioalkoxy wie vorstehend definiert, Hydroxy, Thiol, Nitro, Halogen, Formyl, Amino, 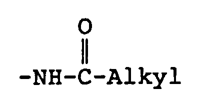 mit Alkyl
wie vorstehend definiert, 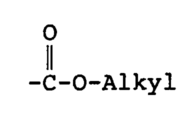 mit Alkyl
wie vorstehend definiert, 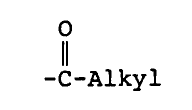 mit Alkyl
wie vorstehend definiert,
oder Phenyl, oder Fluorenylmethyl, 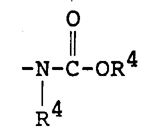
worin
R³ und R⁴ gleich oder verschieden sind und jedes davon wie vorstehend definiert ist, und worin R^{2'}, R^{2''}, R^{2'''} gleich oder verschieden sind und jedes davon bedeutet:
Wasserstoff,
C₁-C₁₂-Alkyl,
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 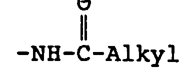 mit Alkyl
wie vorstehend definiert, 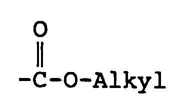 mit Alkyl
wie vorstehend definiert, 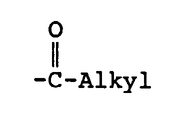 mit Alkyl
wie vorstehend definiert,
oder Aryl, oder
ein heteroaromatisches Radikal, bestehend aus einem Rest 2- oder 3-Thienyl, 2- oder 3-Furanyl, 2-oder 3-Pyrrolyl, 2-, 4- oder 5-Imidazolyl, 3-, 4 oder 5-Pyrazolyl, 2-, 4- oder 5-Thiazolyl, 3-, 4 oder 5-Isothiazolyl, 2-, 4- oder 5-Oxazolyl, 3-, 4 oder 5-Isoxazolyl, 3- oder 5-1,2,4,-Triazolyl, 4- oder 5-1,2,3-Triazolyl, Tetrazolyl, 2-, 3- oder 4-Pyridinyl, 3-, 4 oder 5-Pyridazinyl, 2-Pyrazinyl, 2-, 4- oder 5-Pyrimidinyl, 2-, 3-, 4-, 5-, 6-, 7- oder 8-Chinolinyl, 1-, 3-, 4-, 5-, 6-, 7- oder 8-Isochinolinyl, 2-, 3-, 4-, 5-, 6- oder 7-Indolyl, 2-, 3-, 4-, 5-, 6- oder 7-Benzo[b]thienyl, 2-, 4-, 5-, 6- oder 7-Benzoxazolyl, 2-, 4-, 5-, 6- oder 7-Benzothiazolyl,
der unsubstituiert ist oder substituiert ist mit 1 bis 2 Substituenten, die ausgewählt sind aus Alkyl wie vorstehend definiert, Aryl wie vorstehend definiert, Alkoxy wie vorstehend definiert, Thioalkoxy wie vorstehend definiert, Hydroxy, Thiol, Nitro, Halogen, Formyl, Amino, 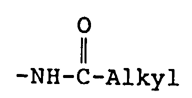 mit Alkyl
wie vorstehend definiert, 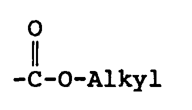 mit Alkyl
wie vorstehend definiert, 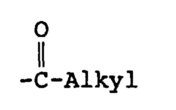 mit Alkyl
wie vorstehend definiert,
oder Phenyl,
mit der Massgabe, dass mindestens eines von R^{2'}, R^{2''}, R^{2'''} für Wasserstoff oder Methyl steht,
R² für Wasserstoff oder Methyl steht,
n für Null steht, und
n' für Null oder die natürliche Zahl 1, 2 oder 3 steht,
AA² steht für 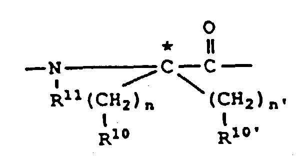 worin
R¹¹ für Wasserstoff oder Methyl steht,
n für Null steht,
R¹⁰ für Wasserstoff oder Methyl steht,
n' für Null oder die natürliche Zahl 1, 2, 3 oder 4 steht, und
R^{10'} für C₁-C₁₂-Alkyl steht,
oder OH, 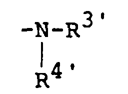 worin
R^{3'} und R^{4'} gleich oder verschieden sind und jedes davon bedeutet:
Wasserstoff,
C₁-C₁₂-Alkyl, oder
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 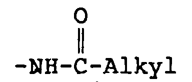 mit Alkyl wie vorstehend definiert, 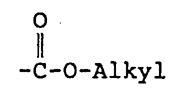 mit Alkyl wie vorstehend definiert, 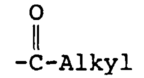 mit Alkyl wie vorstehend definiert, oder Aryl, oder 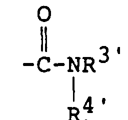 mit R^{3'} und R^{4'} wie vorstehend definiert, 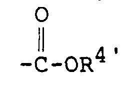 mit R^{4'} wie vorstehend definiert;
AA³ steht für 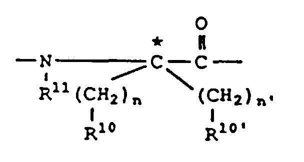 worin
R¹¹ für Wasserstoff oder Methyl steht,
n für Null steht,
R¹⁰ für Wasserstoff oder Methyl steht,
n' für Null oder die natürliche Zahl 1, 2 oder 3 steht, und
R^{10'} für C₁-C₁₂-Alkyl steht,
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 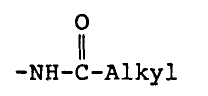 mit Alkyl
wie vorstehend definiert, 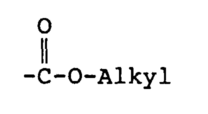 mit Alkyl
wie vorstehend definiert, 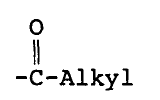 mit Alkyl
wie vorstehend definiert,
oder Aryl, oder 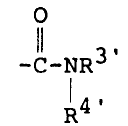 mit R^{3'} und R^{4'} wie vorstehend definiert, 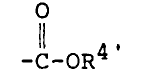 mit R^{4'} wie vorstehend definiert;
AA⁴ und AA⁵ stehen, jedes davon, für 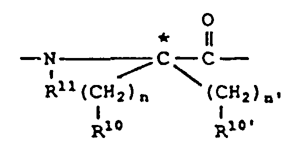 worin
R¹¹ für Wasserstoff oder Methyl steht,
n für Null steht,
R¹⁰ für Wasserstoff oder Methyl steht,
n' für Null steht, und
R^{10'} für C₁-C₁₂-Alkyl oder C₁-C₁₂-Cycloalkyl steht,
AA⁶ steht für 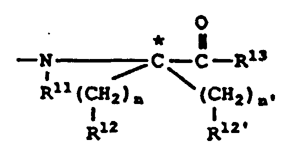 worin
R¹¹ für Wasserstoff oder Methyl steht,
n für Null steht,
R¹² für Wasserstoff oder Methyl steht,
n' für Null oder die natürliche Zahl 1, 2 oder 3 steht, und
R^{12'} steht für
ein aromatisches Radikal, bestehend aus einem Rest Phenyl, Benzyl, Naphthyl, Biphenyl, Pyrenyl, Anthracenyl oder Fluorenyl,
der unsubstituiert ist oder substituiert ist mit 1 bis 4 Substituenten, die ausgewählt sind aus Alkyl wie vorstehend definiert, Alkoxy wie vorstehend definiert, Thioalkoxy wie vorstehend definiert, Hydroxy, Thiol, Nitro, Halogen, Amino, 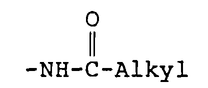 mit Alkyl
wie vorstehend definiert, 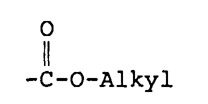 mit Alkyl
wie vorstehend definiert, 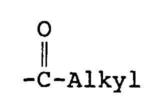 mit Alkyl
wie vorstehend definiert,
oder Aryl, oder
ein heteroaromatisches Radikal, bestehend aus einem Rest 2- oder 3-Thienyl, 2- oder 3-Furanyl, 2- oder 3-Pyrrolyl, 2-, 4- oder 5-Imidazolyl, 3-, 4 oder 5-Pyrazolyl, 2-, 4- oder 5-Thiazolyl, 3-, 4 oder 5-Isothiazolyl, 2-, 4- oder 5-Oxazolyl, 3-, 4 oder 5-Isoxazolyl, 3- oder 5-1,2,4,-Triazolyl, 4- oder 5-1,2,3-Triazolyl, Tetrazolyl, 2-, 3- oder 4-Pyridinyl, 3-, 4 oder 5-Pyridazinyl, 2-Pyrazinyl, 2-, 4- oder 5-Pyrimidinyl, 2-, 3-, 4-, 5-, 6-, 7- oder 8-Chinolinyl, 1-, 3-, 4-, 5-, 6-, 7- oder 8-Isochinolinyl, 2-, 3-, 4-, 5-, 6- oder 7-Indolyl, 2-, 3-, 4-, 5-, 6- oder 7-Benzo[b]thienyl, 2-, 4-, 5-, 6- oder 7-Benzoxazolyl, 2-, 4-, 5-, 6- oder 7-Benzothiazolyl,
der unsubstituiert ist oder substituiert ist mit 1 bis 2 Substituenten, die ausgewählt sind aus Alkyl wie vorstehend definiert, Aryl wie vorstehend definiert, Alkoxy wie vorstehend definiert, Thioalkoxy wie vorstehend definiert, Hydroxy, Thiol, Nitro, Halogen, Formyl, Amino, 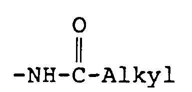 mit Alkyl
wie vorstehend definiert, 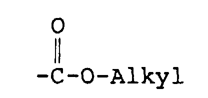 mit Alkyl
wie vorstehend definiert, 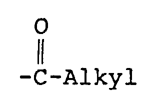 mit Alkyl
wie vorstehend definiert,
oder Phenyl,
R¹³ steht für
-(CH₂)ₙ-CO₂H,
worin n für Null oder die natürliche Zahl 1, 2, 3, 4, 5 oder 6 steht,
-(CH₂)ₙ-OH,
worin n für Null oder die natürliche Zahl 1, 2, 3, 4, 5 oder 6 steht, oder 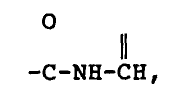 worin R¹⁴ für Wasserstoff oder
-(CH₂)-CO₂H steht,
wobei
am 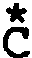 in AA¹ die Stereochemie D ist,
am 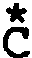 in AA², AA³, AA⁴ oder AA⁵ die Stereochemie D oder L ist, und
am 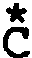 in AA⁶ die Stereochemie L ist.

2. Verbindung nach Anspruch 1, mit
AA¹ in der Bedeutung:
D-Dip,
D-Bip,
D-His,
D-His(Dnp),
D-2-Nal,
D-1-Nal,
D-Phe,
D-Pgl,
D-Tyr,
D-Tyr(OMe),
D-Tyr(OEt),
D-Tyr(OtBu),
D-Trp,
D-Trp(For),
D-Tic,
D-Tza,
D-Pyr,
Ac-D-Dip,
Ac-D-Bip,
Ac-D-His,
Ac-D-His(Dnp),
Ac-D-2-Nal,
Ac-D-1-Nal,
Ac-D-Phe,
Ac-D-Pgl,
Ac-D-Tyr,
Ac-D-Tyr(OMe),
Ac-D-Tyr(OEt),
Ac-D-Tyr(OtBu),
Ac-D-Trp,
Ac-D-Trp(For),
Ac-D-Tic,
Ac-D-Tza,
Ac-D-Pyr,
Ada-D-Dip,
Ada-D-Bip,
Ada-D-His,
Ada-D-His(Dnp),
Ada-D-2-Nal,
Ada-D-1-Nal,
Ada-D-Phe,
Ada-D-Pgl,
Ada-D-Tyr,
Ada-D-Tyr(OMe),
Ada-D-Tyr(OEt),
Ada-D-Tyr(OtBu),
Ada-D-Trp,
Ada-D-Trp(For),
Ada-D-Tic,
Ada-D-Tza,
Ada-D-Pyr,
Adoc-D-Dip,
Adoc-D-Bip,
Adoc-D-His,
Adoc-D-His(Dnp),
Adoc-D-2-Nal,
Adoc-D-1-Nal,
Adoc-D-Phe,
Adoc-D-Pgl,
Adoc-D-Tyr,
Adoc-D-Tyr(OMe),
Adoc-D-Tyr(OEt),
Adoc-D-Tyr(OtBu),
Adoc-D-Trp,
Adoc-D-Trp(For),
Adoc-D-Tic,
Adoc-D-Tza,
Adoc-D-Pyr,
Boc-D-Dip,
Boc-D-Bip,
Boc-D-His,
Boc-D-His(Dnp),
Boc-D-2-Nal,
Boc-D-1-Nal,
Boc-D-Phe,
Boc-D-Pgl,
Boc-D-Tyr,
Boc-D-Tyr(OMe),
Boc-D-Tyr(OEt),
Boc-D-Tyr(OtBu),
Boc-D-Trp,
Boc-D-Trp(For),
Boc-D-Tic,
Boc-D-Tza,
Boc-D-Pyr,
Z-D-Dip,
Z-D-Bip,
Z-D-His,
Z-D-His(Dnp),
Z-D-2-Nal,
Z-D-1-Nal,
Z-D-Phe,
Z-D-Pgl,
Z-D-Tyr,
Z-D-Tyr(OMe),
Z-D-Tyr(OEt),
Z-D-Tyr(OtBu),
Z-D-Trp,
Z-D-Trp(For),
Z-D-Tic,
Z-D-Tza,
Z-D-Pyr,
Fmoc-D-Dip,
Fmoc-D-Bip,
Fmoc-D-His,
Fmoc-D-His(Dnp),
Fmoc-D-2-Nal,
Fmoc-D-1-Nal,
Fmoc-D-Phe,
Fmoc-D-Pgl,
Fmoc-D-Tyr,
Fmoc-D-Tyr(OMe),
Fmoc-D-Tyr(OEt),
Fmoc-D-Tyr(OtBu),
Fmoc-D-Trp,
Fmoc-D-Trp(For),
Fmoc-D-Tic,
Fmoc-D-Tza,
Fmoc-D-Pyr, oder
AA² in der Bedeutung:
Ala,
Arg,
Asn,
Asp,
Dab,
Glu,
Gln,
Gly,
homoArg,
homoGlu,
homoLys,
Ile,
Leu,
D-Leu,
Lys,
Met,
Met(O),
Met(O₂),
Nva,
Nle,
Orn,
Phe,
Tyr,
Val, oder
AA² nicht vorhanden;
AA³ in der Bedeutung:
Asn,
Asp,
D-Asp,
N-MeAsp,
Glu,
Gln,
homoPhe,
Phe,
Tyr,oder
AA³ nicht vorhanden;
AA⁴ in der Bedeutung:
Ala,
Chx,
Gly,
Glu,
Ile,
D-Ile,
Leu,
Nleu,
Nval,
Val, oder
AA⁴ nicht vorhanden;
AA⁵ in der Bedeutung:
Ala,
Chx,
Gly,
Ile,
D-Ile,
Leu,
Nleu,
Nval,
Val, oder
AA⁵ nicht vorhanden;
AA⁶ in der Bedeutung:
2-Nal,
1-Nal,
Pyr,
Trp,
Tyr(OMe),
Tyr(OEt),
Tyr(OtBu),
Tyr,
Trp-Gly,
Trp-Asp,
Trp(For),
Dip,
Phe,
Bza, oder

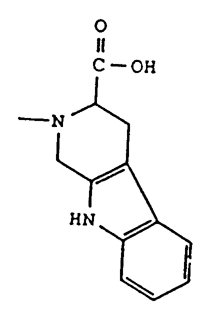

3. Verbindung nach Anspruch 1, ausgewählt aus der Gruppe, die gebildet ist von:
D-Phe-Leu-Asp-Ile-Ile-Trp;
D-His(Dnp)-Leu-Asp-Ile-Ile-Trp;
D-Trp-Leu-Asp-Ile-Ile-Trp;
D-Tyr-Leu-Asp-Ile-Ile-Trp;
D-Tyr(OMe)-Leu-Asp-Ile-Ile-Trp;
D-Tyr(OEt)-Leu-Asp-Ile-Ile-Trp;
D-2-Nal-Leu-Asp-Ile-Ile-Trp;
D-1-Nal-Leu-Asp-Ile-Ile-Trp;
D-Pgl-Leu-Asp-Ile-Ile-Trp;
D-Pyr-Leu-Asp-Ile-Ile-Trp;
D-Tic-Leu-Asp-Ile-Ile-Trp;
D-Dip-Leu-Asp-Ile-Ile-Trp;
D-Bip-Leu-Asp-Ile-Ile-Trp;
Ac-D-Phe-Leu-Asp-Ile-Ile-Trp;
Ac-D-His(Dnp)-Leu-Asp-Ile-Ile-Trp;
Ac-D-Trp-Leu-Asp-Ile-Ile-Trp;
Ac-D-Tyr-Leu-Asp-Ile-Ile-Trp;
Ac-D-Tyr(OMe)-Leu-Asp-Ile-Ile-Trp;
Ac-D-Tyr(OEt)-Leu-Asp-Ile-Ile-Trp;
Ac-D-2-Nal-Leu-Asp-Ile-Ile-Trp;
Ac-D-1-Nal-Leu-Asp-Ile-Ile-Trp;
Ac-D-Pgl-Leu-Asp-Ile-Ile-Trp;
Ac-D-Pyr-Leu-Asp-Ile-Ile-Trp;
Ac-D-Tic-Leu-Asp-Ile-Ile-Trp;
Ac-D-Dip-Leu-Asp-Ile-Ile-Trp;
Ac-D-Bip-Leu-Asp-Ile-Ile-Trp;
Fmoc-D-Phe-Leu-Asp-Ile-Ile-Trp;
Fmoc-D-His-Leu-Asp-Ile-Ile-Trp;
Fmoc-D-Trp-Leu-Asp-Ile-Ile-Trp;
Fmoc-D-Tyr-Leu-Asp-Ile-Ile-Trp;
Fmoc-D-Tyr(OMe)-Leu-Asp-Ile-Ile-Trp;
Fmoc-D-Tyr(OEt)-Leu-Asp-Ile-Ile-Trp;
Fmoc-D-2-Nal-Leu-Asp-Ile-Ile-Trp;
Fmoc-D-1-Nal-Leu-Asp-Ile-Ile-Trp;
Fmoc-D-Dip-Leu-Asp-Ile-Ile-Trp;
Fmoc-D-Bip-Leu-Asp-Ile-Ile-Trp;
Ada-D-Phe-Leu-Asp-Ile-Ile-Trp;
Ada-D-His-Leu-Asp-Ile-Ile-Trp;
Ada-D-Trp-Leu-Asp-Ile-Ile-Trp;
Ada-D-Tyr-Leu-Asp-Ile-Ile-Trp;
Ada-D-Tyr(OMe)-Leu-Asp-Ile-Ile-Trp;
Ada-D-Tyr(OEt)-Leu-Asp-Ile-Ile-Trp;
Ada-D-2-Nal-Leu-Asp-Ile-Ile-Trp;
Ada-D-1-Nal-Leu-Asp-Ile-Ile-Trp;
Ada-D-Dip-Leu-Asp-Ile-Ile-Trp;
Ada-D-Bip-Leu-Asp-Ile-Ile-Trp;
Ac-D-Phe-D-Leu-Asp-Ile-Ile-Trp;
Ac-D-His-D-Leu-Asp-Ile-Ile-Trp;
Ac-D-Trp-D-Leu-Asp-Ile-Ile-Trp;
Ac-D-Tyr-D-Leu-Asp-Ile-Ile-Trp;
Ac-D-Tyr(OMe)-D-Leu-Asp-Ile-Ile-Trp;
Ac-D-Tyr(OEt)-D-Leu-Asp-Ile-Ile-Trp;
Ac-D-2-Nal-D-Leu-Asp-Ile-Ile-Trp;
Ac-D-1-Nal-D-Leu-Asp-Ile-Ile-Trp;
Ac-D-Dip-D-Leu-Asp-Ile-Ile-Trp;
Ac-D-Bip-D-Leu-Asp-Ile-Ile-Trp;
Ac-D-Phe-Ile-Asp-Ile-Ile-Trp;
Ac-D-His-Ile-Asp-Ile-Ile-Trp;
Ac-D-Trp-Ile-Asp-Ile-Ile-Trp;
Ac-D-Tyr-Ile-Asp-Ile-Ile-Trp;
Ac-D-Tyr(OMe)-Ile-Asp-Ile-Ile-Trp;
Ac-D-Tyr(OEt)-Ile-Asp-Ile-Ile-Trp;
Ac-D-2-Nal-Ile-Asp-Ile-Ile-Trp;
Ac-D-1-Nal-Ile-Asp-Ile-Ile-Trp;
Ac-D-Dip-Ile-Asp-Ile-Ile-Trp;
Ac-D-Bip-Ile-Asp-Ile-Ile-Trp;
Ac-D-Phe-Val-Asp-Ile-Ile-Trp;
Ac-D-His-Val-Asp-Ile-Ile-Trp;
Ac-D-Trp-Val-Asp-Ile-Ile-Trp;
Ac-D-Tyr-Val-Asp-Ile-Ile-Trp;
Ac-D-Tyr(OMe)-Val-Asp-Ile-Ile-Trp;
Ac-D-Tyr(OEt)-Val-Asp-Ile-Ile-Trp;
Ac-D-2-Nal-Val-Asp-Ile-Ile-Trp;
Ac-D-1-Nal-Val-Asp-Ile-Ile-Trp;
Ac-D-Dip-Val-Asp-Ile-Ile-Trp;
Ac-D-Bip-Val-Asp-Ile-Ile-Trp;
Ac-DPhe-Dab-Asp-Ile-Ile-Trp;
Ac-DHis-Dab-Asp-Ile-Ile-Trp;
Ac-DTrp-Dab-Asp-Ile-Ile-Trp;
Ac-DTyr(OMe)-Dab-Asp-Ile-Ile-Trp;
Ac-DTyr(OEt)-Dab-Asp-Ile-Ile-Trp;
Ac-D2Nal-Dab-Asp-Ile-Ile-Trp;
Ac-D3Nal-Dab-Asp-Ile-Ile-Trp;
AC-DDip-Dab-Asp-Ile-Ile-Trp;
Ac-DBip-Dab-Asp-Ile-Ile-Trp;
Ac-DPhe-Arg-Asp-Ile-Ile-Trp;
Ac-DHis-Arg-Asp-Ile-Ile-Trp;
Ac-DTrp-Arg-Asp-Ile-Ile-Trp;
Ac-DTyr(OMe)-Arg-Asp-Ile-Ile-Trp;
Ac-DTyr(OEt)-Arg-Asp-Ile-Ile-Trp;
Ac-D2Nal-Arg-Asp-Ile-Ile-Trp;
Ac-D3Nal-Arg-Asp-Ile-Ile-Trp;
Ac-DDip-Arg-Asp-Ile-Ile-Trp;
Ac-DBip-Arg-Asp-Ile-Ile-Trp;
Ac-DPhe-hLys-Asp-Ile-Ile-Trp;
Ac-DHis-hLys-Asp-Ile-Ile-Trp;
Ac-DTrp-hLys-Asp-Ile-Ile-Trp;
Ac-DTyr(OMe)-hLys-Asp-Ile-Ile-Trp;
Ac-DTyr(OEt)-hLys-Asp-Ile-Ile-Trp;
Ac-D2Nal-hLys-Asp-Ile-Ile-Trp;
Ac-D3Nal-hLys-Asp-Ile-Ile-Trp;
Ac-DDip-hLys-Asp-Ile-Ile-Trp;
Ac-DBip-hLys-Asp-Ile-Ile-Trp;
Ac-DPhe-Glu-Asp-Ile-Ile-Trp;
Ac-DHis-Glu-Asp-Ile-Ile-Trp;
Ac-DTrp-Glu-Asp-Ile-Ile-Trp;
Ac-DTyr(OMe)-Glu-Asp-Ile-Ile-Trp;
Ac-DTyr(OEt)-Glu-Asp-Ile-Ile-Trp;
Ac-D2Nal-Glu-Asp-Ile-Ile-Trp;
Ac-D3Nal-Glu-Asp-Ile-Ile-Trp;
Ac-DDip-Glu-Asp-Ile-Ile-Trp;
Ac-DBip-Glu-Asp-Ile-Ile-Trp;
Ac-DPhe-hGlu-Asp-Ile-Ile-Trp;
Ac-DHis-hGlu-Asp-Ile-Ile-Trp;
Ac-DTrp-hGlu-Asp-Ile-Ile-Trp;
Ac-DTyr(OMe)-hGlu-Asp-Ile-Ile-Trp;
Ac-DTyr(OEt)-hGlu-Asp-Ile-Ile-Trp;
Ac-D2Nal-hGlu-Asp-Ile-Ile-Trp;
Ac-D3Nal-hGlu-Asp-Ile-Ile-Trp;
Ac-DDip-hGlu-Asp-Ile-Ile-Trp;
Ac-DBip-hGlu-Asp-Ile-Ile-Trp;
Ac-DPhe-Asp-Asp-Ile-Ile-Trp;
Ac-DHis-Asp-Asp-Ile-Ile-Trp;
Ac-DTrp-Asp-Asp-Ile-Ile-Trp;
Ac-DTyr(OMe)-Asp-Asp-Ile-Ile-Trp;
Ac-DTyr(OEt)-Asp-Asp-Ile-Ile-Trp;
Ac-D2Nal-Asp-Asp-Ile-Ile-Trp;
Ac-D3Nal-Asp-Asp-Ile-Ile-Trp;
Ac-DDip-Asp-Asp-Ile-Ile-Trp;
Ac-DBip-Asp-Asp-Ile-Ile-Trp;
Ac-D-Phe-Lys-Asp-Ile-Ile-Trp;
Ac-D-His-Lys-Asp-Ile-Ile-Trp;
Ac-D-Trp-Lys-Asp-Ile-Ile-Trp;
Ac-D-Tyr-Lys-Asp-Ile-Ile-Trp;
Ac-D-Tyr(OMe)-Lys-Asp-Ile-Ile-Trp;
Ac-D-Tyr(OEt)-Lys-Asp-Ile-Ile-Trp;
Ac-D-2-Nal-Lys-Asp-Ile-Ile-Trp;
Ac-D-1-Nal-Lys-Asp-Ile-Ile-Trp;
Ac-D-Phe-Orn-Asp-Ile-Ile-Trp;
Ac-D-His-Orn-Asp-Ile-Ile-Trp;
Ac-D-Trp-Orn-Asp-Ile-Ile-Trp;
Ac-D-Tyr-Orn-Asp-Ile-Ile-Trp;
Ac-D-Tyr(OMe)-Orn-Asp-Ile-Ile-Trp;
Ac-D-Tyr(OEt)-Orn-Asp-Ile-Ile-Trp;
Ac-D-2-Nal-Orn-Asp-Ile-Ile-Trp;
Ac-D-1-Nal-Orn-Asp-Ile-Ile-Trp;
Ac-D-Phe-Gln-Asp-Ile-Ile-Trp;
Ac-D-His-Gln-Asp-Ile-Ile-Trp;
Ac-D-Trp-Gln-Asp-Ile-Ile-Trp;
Ac-D-Tyr-Gln-Asp-Ile-Ile-Trp;
Ac-D-Tyr(OMe)-Gln-Asp-Ile-Ile-Trp;
Ac-D-Tyr(OEt)-Gln-Asp-Ile-Ile-Trp;
Ac-D-2-Nal-Gln-Asp-Ile-Ile-Trp;
Ac-D-1-Nal-Gln-Asp-Ile-Ile-Trp;
Ac-D-Phe-Leu-Glu-Ile-Ile-Trp;
Ac-D-His-Leu-Glu-Ile-Ile-Trp;
Ac-D-Trp-Leu-Glu-Ile-Ile-Trp;
Ac-D-Tyr-Leu-Glu-Ile-Ile-Trp;
Ac-D-Tyr(OMe)-Leu-Glu-Ile-Ile-Trp;
Ac-D-Tyr(OEt)-Leu-Glu-Ile-Ile-Trp;
Ac-D-2-Nal-Leu-Glu-Ile-Ile-Trp;
Ac-D-1-Nal-Leu-Glu-Ile-Ile-Trp;
Ac-D-Phe-Leu-Asn-Ile-Ile-Trp;
Ac-D-His-Leu-Asn-Ile-Ile-Trp;
Ac-D-Trp-Leu-Asn-Ile-Ile-Trp;
Ac-D-Tyr-Leu-Asn-Ile-Ile-Trp;
Ac-D-Tyr(OMe)-Leu-Asn-Ile-Ile-Trp;
Ac-D-Tyr(OEt)-Leu-Asn-Ile-Ile-Trp;
Ac-D-2-Nal-Leu-Asn-Ile-Ile-Trp;
Ac-D-1-Nal-Leu-Asn-Ile-Ile-Trp;
Ac-D-Dip-Leu-Asn-Ile-Ile-Trp;
Ac-D-Bip-Leu-Asn-Ile-Ile-Trp;
Ac-DPhe-Leu-Phe-Ile-Ile-Trp;
Ac-DHis-Leu-Phe-Ile-Ile-Trp;
Ac-DTrp-Leu-Phe-Ile-Ile-Trp;
Ac-DTyr(OMe)-Leu-Phe-Ile-Ile-Trp;
Ac-DTyr(OEt)-Leu-Phe-Ile-Ile-Trp;
Ac-D2Nal-Leu-Phe-Ile-Ile-Trp;
Ac-D3Nal-Leu-Phe-Ile-Ile-Trp;
Ac-DDip-Leu-Phe-Ile-Ile-Trp;
Ac-DBip-Leu-Phe-Ile-Ile-Trp;
Ac-D-Phe-Glu-Asn-Ile-Ile-Trp;
Ac-D-Phe-Leu-Asp-Val-Ile-Trp;
Ac-D-His-Leu-Asp-Val-Ile-Trp;
Ac-D-Trp-Leu-Asp-Val-Ile-Trp;
Ac-D-Tyr-Leu-Asp-Val-Ile-Trp;
Ac-D-Tyr(OMe)-Leu-Asp-Val-Ile-Trp;
Ac-D-Tyr(OEt)-Leu-Asp-Val-Ile-Trp;
Ac-D-2-Nal-Leu-Asp-Val-Ile-Trp;
Ac-D-1-Nal-Leu-Asp-Val-Ile-Trp;
Ac-D-Dip-Leu-Asp-Val-Ile-Trp;
Ac-D-Bip-Leu-Asp-Val-Ile-Trp;
Ac-D-Phe-Leu-Asp-Chx-Ile-Trp;
Ac-D-His-Leu-Asp-Chx-Ile-Trp;
Ac-D-Trp-Leu-Asp-Chx-Ile-Trp;
Ac-D-Tyr-Leu-Asp-Chx-Ile-Trp;
Ac-D-Tyr(OMe)-Leu-Asp-Chx-Ile-Trp;
Ac-D-Tyr(OEt)-Leu-Asp-Chx-Ile-Trp;
Ac-D-2-Nal-Leu-Asp-Chx-Ile-Trp;
Ac-D-1-Nal-Leu-Asp-Chx-Ile-Trp;
Ac-D-Dip-Leu-Asp-Chx-Ile-Trp;
Ac-D-Bip-Leu-Asp-Chx-Ile-Trp;
Ac-D-Phe-Leu-Asp-D-Ile-Ile-Trp;
Ac-D-His-Leu-Asp-D-Ile-Ile-Trp;
Ac-D-Trp-Leu-Asp-D-Ile-Ile-Trp;
Ac-D-Tyr-Leu-Asp-D-Ile-Ile-Trp;
Ac-D-Tyr(OMe)-Leu-Asp-D-Ile-Ile-Trp;
Ac-D-Tyr(OEt)-Leu-Asp-D-Ile-Ile-Trp;
Ac-D-2-Nal-Leu-Asp-D-Ile-Ile-Trp;
Ac-D-1-Nal-Leu-Asp-D-Ile-Ile-Trp;
Ac-D-Dip-Leu-Asp-D-Ile-Ile-Trp;
Ac-D-Bip-Leu-Asp-D-Ile-Ile-Trp;
Ac-D-Phe-Leu-Asp-Ile-D-Ile-Trp;
Ac-D-His-Leu-Asp-Ile-D-Ile-Trp;
Ac-D-Trp-Leu-Asp-Ile-D-Ile-Trp;
Ac-D-Tyr-Leu-Asp-Ile-D-Ile-Trp;
Ac-D-Tyr(OMe)-Leu-Asp-Ile-D-Ile-Trp;
Ac-D-Tyr(OEt)-Leu-Asp-Ile-D-Ile-Trp;
Ac-D-2-Nal-Leu-Asp-Ile-D-Ile-Trp;
Ac-D-1-Nal-Leu-Asp-Ile-D-Ile-Trp;
Ac-D-Dip-Leu-Asp-Ile-D-Ile-Trp;
Ac-D-Bip-Leu-Asp-Ile-D-Ile-Trp;
Ac-D-Phe-Leu-Asp-Ile-Val-Trp;
Ac-D-His-Leu-Asp-Ile-Val-Trp;
Ac-D-Trp-Leu-Asp-Ile-Val-Trp;
Ac-D-Tyr-Leu-Asp-Ile-Val-Trp;
Ac-D-Tyr(OMe)-Leu-Asp-Ile-Val-Trp;
Ac-D-Tyr(OEt)-Leu-Asp-Ile-Val-Trp;
Ac-D-2-Nal-Leu-Asp-Ile-Val-Trp;
Ac-D-1-Nal-Leu-Asp-Ile-Val-Trp;
Ac-D-Dip-Leu-Asp-Ile-Val-Trp;
Ac-D-Bip-Leu-Asp-Ile-Val-Trp;
Ac-D-Phe-Leu-Asp-Ile-Chx-Trp;
Ac-D-His-Leu-Asp-Ile-Chx-Trp;
Ac-D-Trp-Leu-Asp-Ile-Chx-Trp;
Ac-D-Tyr-Leu-Asp-Ile-Chx-Trp;
Ac-D-Tyr(OMe)-Leu-Asp-Ile-Chx-Trp;
Ac-D-Tyr(OEt)-Leu-Asp-Ile-Chx-Trp;
Ac-D-2-Nal-Leu-Asp-Ile-Chx-Trp;
Ac-D-1-Nal-Leu-Asp-Ile-Chx-Trp;
Ac-D-Dip-Leu-Asp-Ile-Chx-Trp;
Ac-D-Bip-Leu-Asp-Ile-Chx-Trp;
Ac-D-Phe-Leu-Asp-Ile-Ile-2-Nal;
Ac-D-His-Leu-Asp-Ile-Ile-2-Nal;
Ac-D-Trp-Leu-Asp-Ile-Ile-2-Nal;
Ac-D-Tyr-Leu-Asp-Ile-Ile-2-Nal;
Ac-D-Tyr(OMe)-Leu-Asp-Ile-Ile-2-Nal;
Ac-D-Tyr(OEt)-Leu-Asp-Ile-Ile-2-Nal;
Ac-D-2-Nal-Leu-Asp-Ile-Ile-2-Nal;
Ac-D-1-Nal-Leu-Asp-Ile-Ile-2-Nal;
Ac-D-Dip-Leu-Asp-Ile-Ile-2-Nal;
Ac-D-Bip-Leu-Asp-Ile-Ile-2-Nal;
Ac-D-Phe-Leu-Asp-Ile-Ile-1-Nal;
Ac-D-His-Leu-Asp-Ile-Ile-1-Nal;
Ac-D-Trp-Leu-Asp-Ile-Ile-1-Nal;
Ac-D-Tyr-Leu-Asp-Ile-Ile-1-Nal;
Ac-D-Tyr(OMe)-Leu-Asp-Ile-Ile-1-Nal;
Ac-D-Tyr(OEt)-Leu-Asp-Ile-Ile-1-Nal;
Ac-D-2-Nal-Leu-Asp-Ile-Ile-1-Nal;
Ac-D-1-Nal-Leu-Asp-Ile-Ile-1-Nal;
Ac-D-Dip-Leu-Asp-Ile-Ile-1-Nal;
Ac-D-Bip-Leu-Asp-Ile-Ile-1-Nal;
Ac-D-His-Leu-D-Asp-Ile-D-Ile-Trp;
Ac-D-Phe-Leu-D-Asp-Ile-D-Ile-Trp;
Ac-D-Bip-Leu-D-Asp-Ile-D-Ile-Trp;
Ac-D-Dip-Leu-D-Asp-Ile-D-Ile-Trp;
Ac-D-2-Nal-Leu-D-Asp-Ile-D-Ile-Trp;
Ac-D-1-Nal-Leu-D-Asp-Ile-D-Ile-Trp;
Ac-D-Trp-Leu-D-Asp-Ile-D-Ile-Trp;
Ac-D-Dip-Asn-Ile-Ile-Trp;
Ac-D-Dip-Phe-Ile-Ile-Trp;
Ac-D-Dip-Ile-Ile-Trp;
Ac-D-Dip-Asp-Ile-Ile-Trp;
Ac-D(Nme)-Dip-Leu-Asp-Ile-Ile-Trp;
Ac-D-Dip-Leu-Asp-Ile-Ile-(Nme)Trp;
Ac-D-Dip-Leu-Asp-Ile-(Nme)Ile-Trp;
Ac-D-Dip-Leu-Asp-(Nme)Ile-Ile-Trp;
Ac-D-Dip-Leu-(Nme)Asp-Ile-Ile-Trp;
Ac-D-Dip-(Nme)Leu-Asp-Ile-Ile-Trp;
Ac-D-Phe-Asp-Ile-Ile-Trp;
Ac-D-His-Asp-Ile-Ile-Trp;
Ac-D-Trp-Asp-Ile-Ile-Trp;
Ac-D-Tyr-Asp-Ile-Ile-Trp;
Ac-D-Tyr(OMe)-Asp-Ile-Ile-Trp;
Ac-D-Tyr(OEt)-Asp-Ile-Ile-Trp;
Ac-D-2-Nal-Asp-Ile-Ile-Trp;
Ac-D-1-Nal-Asp-Ile-Ile-Trp;
Ada-D-Phe-Asp-Ile-Ile-Trp;
Ada-D-His-Asp-Ile-Ile-Trp;
Ada-D-Trp-Asp-Ile-Ile-Trp;
Ada-D-Tyr-Asp-Ile-Ile-Trp;
Ada-D-Tyr(OMe)-Asp-Ile-Ile-Trp;
Ada-D-Tyr(OEt)-Asp-Ile-Ile-Trp;
Ada-D-2-Nal-Asp-Ile-Ile-Trp;
Ada-D-1-Nal-Asp-Ile-Ile-Trp;
Ada-D-Dip-Asp-Ile-Ile-Trp;
Ada-D-Bip-Asp-Ile-Ile-Trp;
Ac-D-Phe-Asp-Ile-Ile-2-Nal;
Ac-D-Phe-Asp-Ile-Ile-1-Nal;
Ac-D-His-Asp-Ile-Ile-2-Nal;
Ac-D-His-Asp-Ile-Ile-1-Nal;
Ac-D-Tyr-Asp-Ile-Ile-2-Nal;
Ac-D-Tyr-Asp-Ile-Ile-1-Nal;
Ac-D-Trp-Asp-Ile-Ile-2-Nal;
Ac-D-Trp-Asp-Ile-Ile-1-Nal;
Ac-D-Dip-Asp-Ile-Ile-2-Nal;
Ac-D-Dip-Asp-Ile-Ile-1-Nal;
Ac-D-Bip-Asp-Ile-Ile-2-Nal;
Ac-D-Bip-Asp-Ile-Ile-1-Nal;
Ac-D-Phe-Leu-Asp-Ile-Trp;
Ac-D-His-Leu-Asp-Ile-Trp;
Ac-D-Tyr-Leu-Asp-Ile-Trp;
Ac-D-Dip-Leu-Asp-Ile-Trp;
Ac-D-Trp-Leu-Asp-Ile-Trp;
Ac-DPhe-Leu-Asp-Ile-Ile-Trp-Gly;
Ac-DHis-Leu-Asp-Ile-Ile-Trp-Gly;
Ac-DTrp-Leu-Asp-Ile-Ile-Trp-Gly;
Ac-DTyr(OMe)-Leu-Asp-Ile-Ile-Trp-Gly;
Ac-DTyr(OEt)-Leu-Asp-Ile-Ile-Trp-Gly;
Ac-D2Nal-Leu-Asp-Ile-Ile-Trp-Gly;
Ac-D3Nal-Leu-Asp-Ile-Ile-Trp-Gly;
Ac-DDip-Leu-Asp-Ile-Ile-Trp-Gly;
Ac-DBip-Leu-Asp-Ile-Ile-Trp-Gly;
Ac-DPhe-Leu-Asp-Ile-Ile-Trp-Asp;
Ac-DHis-Leu-Asp-Ile-Ile-Trp-Asp;
Ac-DTrp-Leu-Asp-Ile-Ile-Trp-Asp;
Ac-DTyr(OMe)-Leu-Asp-Ile-Ile-Trp-Asp;
Ac-DTyr(OEt)-Leu-Asp-Ile-Ile-Trp-Asp;
Ac-D2Nal-Leu-Asp-Ile-Ile-Trp-Asp;
Ac-D3Nal-Leu-Asp-Ile-Ile-Trp-Asp;
Ac-DDip-Leu-Asp-Ile-Ile-Trp-Asp;
Ac-DBip-Leu-Asp-Ile-Ile-Trp-Asp.

4. Verbindung nach Anspruch 3, ausgewählt aus der Gruppe, die gebildet ist von:
Ac-D-Phe-Leu-Asp-Ile-Ile-Trp;
D-2-Nal-Leu-Asp-Ile-Ile-Trp;
Ac-D-2-Nal-Leu-Asp-Ile-Ile-Trp;
D-1-Nal-Leu-Asp-Ile-Ile-Trp;
Ac-D-Phe-Leu-Asp-Ile-Trp;
Ac-D-His-Leu-D-Asp-Ile-D-Ile-Trp;
Ac-D-Phe-Orn-Asp-Ile-Ile-Trp;
Ac-D-Phe-Glu-Asp-Ile-Ile-Trp;
Ac-D-Tyr-Leu-Asp-Ile-Ile-Trp;
Ac-D-Phe-Asp-Ile-Ile-Trp;
Fmoc-D-Phe-Leu-Asp-Ile-Ile-Trp;
Ac-D-Dip-Leu-Asp-Ile-Ile-Trp;
Ac-D-Dip-Ile-Ile-Trp;
Ac-D-Dip-Asp-Ile-Ile-Trp;
Ac-D-Dip-Leu-Phe-Ile-Ile-Trp;
Ac-D-Dip-Leu-Asp-Ile-Lys-Trp;
Ac-D-Dip-Leu-Asp-Ile-Glu-Trp;
Ac-D-Dip-Leu-Asp-Glu-Ile-Trp;
Ac-D-Dip-Glu-Asp-Ile-Ile-Trp;
Ac-D-Dip-Orn-Asp-Ile-Ile-Trp;
Ac-D-Dip-Leu-Asp(N-Me)-Ile-Ile-Trp; und
Ac-D-Dip-D-Leu-Asp-Ile-Ile-Trp.

5. Arzneimittelzusammensetzung, umfassend eine Verbindung nach Anspruch 1 bis 4 im Gemisch mit einem pharmazeutisch annehmbaren Füller, Verdünner oder Träger.

6. Verwendung einer Verbindung nach Anspruch 1 bis 4 zur Herstellung einer Arzneimittelzusammensetzung, die verwendbar ist zur Behandlung von erhöhtem Blutdruck, Metabolismus- und endokrinen Störungen, kongestivem Herzversagen, Myokardinfektion, endotoxischem Schock, subarachnoidaler Hämorrhagie, Arrhythmien, Asthma, akutem Nierenversagen, Präeklampsie, Diabetes und neurologischen Störungen, und zur Hemmung von erhöhtem Endothelinspiegel.

7. Verfahren zur Herstellung einer Verbindung nach Anspruch 1 bis 4 der Formel I
AA¹-AA²-AA³-AA⁴-AA⁵-AA⁶
in welcher AA¹, AA², AA³, AA⁴, AA⁵ und AA⁶ die im vorstehenden angegebene Bedeutung haben, oder eines pharmazeutisch annehmbaren Salzes davon, umfassend die aufeinanderfolgende schrittweise Kopplung der aus AA¹, AA², AA³, AA⁴, AA⁵ oder AA⁶ ausgewählten Aminosäuren an die vorangehende Aminosäure unter Verwendung von herkömmlicher Peptidsynthesen-Methodologie, was nach herkömmlicher Schutzgruppenentfernung eine Verbindung der Formel I ergibt, und falls erwünscht die Konversion einer Verbindung der Formel I zu einem pharmazeutisch annehmbaren Salz einer Verbindung der Formel I durch herkömmliche Methodologie, und falls weiter erwünscht die Konversion des so erhaltenen pharmazeutisch annehmbaren Salzes einer Verbindung der Formel I zu einer Verbindung der Formel I durch herkömmliche Methodologie.

## Revendications

1. Un composé de formule I
AA¹-AA²-AA³-AA⁴-AA⁵-AA⁵-AA⁶
où
AA¹ est 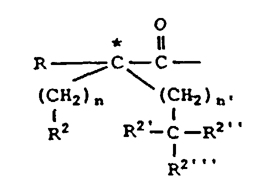
dans laquelle R est un hydrogène, un alkyle en C₁-C₁₂, un alkényle en C₂-C₁₂, un alkynyle en C₂-C₁₂, un cycloalkyle en C₃-C₁₂, un alkyle en C₁-C₁₂ cycloalkyle en C₃-C₁₂, un radical aromatique qui est un groupe phényle, un groupe benzyle, un groupe naphtyle, un groupe biphényle, un groupe pyrényle, un groupe anthracényle, ou un groupe fluorényle, non substitués ou substitués par 1 à 4 substituants choisis parmi les alkyles tels que définis ci-dessus, alkoxy tels que définis ci-dessus, thioalkoxy que définis ci-dessus, hydroxy, thiol, nitro, halogène, amino, 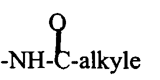 où alkyle est tel que défini ci-dessus, 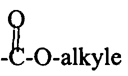 où alkyle est tel que défini ci-dessus, 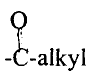 où alkyle est tel que défini ci-dessus, ou aryle, ou bien un radical hétéroatomique qui est 2- ou 3-thiényle, 2- ou 3-furanyle, 2- ou 3-pyrrolyle, 2-, 4-, ou 5-imidazolyle, 3-, 4-, ou 5 pyrazolyle, 2-, 4-, ou 5-thiazolyle, 3-, 4-, ou 5-isothiazolyle, 2-, 4-, ou 5-oxazolyle, 3-, 4-, ou 5-isoxazolyle, 3-, ou 5-1, 2, 4-triazolyle, 4- ou 5-1,2,3-triazolyle, tétrazolyle, 2-, 3-, ou 4-pyridinyle, 3-, 4-, ou 5-pyridazinyle, 2-pyrazinyle, 2-, 4-, ou 5-pyrimidinyle, 2-, 3-, 4-, 5-, 6-, 7-, ou 8-quinolinyle, 1-, 3-, 4-, 5-, 6-, 7-, ou 8-isoquinolinyle, 2-, 3-, 4-, 5-, 6-, ou 7-indolyle, 2-, 3-, 4-, 5-, 6-, ou 7-benzo[b]thiényle, ou 2-, 4-, 5-, 6-, ou 7-benzoxazolyle, ou 2-, 4-, 5-, 6-, ou 7-benzimidazolyle, 2-, 4-, 5-, 6-, ou 7-benzothiazolyle, non substitués ou substitués par un ou deux substituants choisis parmi les alkyles tels que définis ci-dessus, aryles tel que défini ci-dessus, alkoxy tel que défini ci-dessus, thialkoxy tel que défini ci-dessus, hydroxy, thiol, nitro, halogène, formyle, amino, 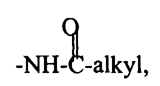 où alkyl est tel que défini ci-dessus, 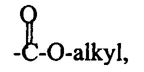 où alkyl est tel que défini ci-dessus, 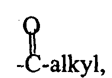 où alkyl est tel que défini ci-dessus ou phényle ou fluorénylméthyle, 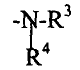 où R³ et R⁴ sont identiques ou différents et sont chacun un hydrogène,
un alkyle en C₁-C₂, un alkényle en C₂-C₁₂, un alkynyle en C₂-C₁₂,
un cycloalkyl en C₃-C₁₂, alkyl en C₁-C₁₂, cycloalkyle en C₃-C₁₂, un radical aromatique qui est un groupe phényle, un groupe benzyle, un groupe naphtyle, un groupe biphényle, un groupe pyrényle, un groupe anthracényle, ou un groupe fluorényle, non substitués ou substitués par 1 à 4 substituants choisis parmi les alkyles tels que définis ci-dessus, alkoxy tel que défini ci-dessus, thialkoxy tel que défini ci-dessus, hydroxy, thiol, nitro, halogène, amino, 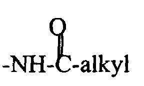 où alkyl est tel que défini ci-dessus, 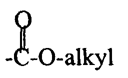 où alkyl est tel que défini ci-dessus, 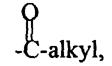 où alkyl est tel que défini ci-dessus ou aryle, ou
un radical hétéroatomique qui est le 2- ou 3-thiényle, 2- ou 3-furanyle, 2- ou 3-pyrrolyle, 2-, 4-, ou 5-imidazolyle, 3-, 4-, ou 5 pyrazolyle, 2-, 4-, ou 5-thiazolyle, 3-, 4-, ou 5-isothiazolyle, 2-, 4-, ou 5-oxazolyle, 3-, 4-, ou 5-isoxazolyle, 3-, ou 5-1, 2, 4-triazolyle, 4- ou 5-1,2,3-triazolyle, tétrazolyle, 2-, 3-, ou 4-pyridinyle, 3-, 4-, ou 5-pyridazinyle, 2-pyrazinyle, 2-, 4-, ou 5-pyrimidinyle, 2-, 3-, 4-, 5-, 6-, 7-, ou 8-quinolinyle, 1-, 3-, 4-, 5-, 6-, 7-, ou 8-isoquinolinyle, 2-, 3-, 4-, 5-, 6-, ou 7-indolyle, 2-, 3-, 4-, 5-, 6-, ou 7-benzo[b]thiényle, ou 2-, 4-, 5-, 6-, ou 7-benzoxazolyle, ou 2-, 4-, 5-, 6-, ou 7-benzimidazolyle, 2-, 4-, 5-, 6-, ou 7-benzothaizolyle, 2-, 4-, 5-, 6-, ou 7-benzothiazolyle, non substitués ou substitués par 1 ou 2 substituants choisis parmi les alkyles tel que défini ci-dessus, aryles tel que défini ci-dessus, alkoxy tel que défini ci-dessus, thialkoxy tel que défini ci- dessus, hydroxy, thiol, nitro, halogène, formyle, amino, 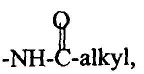 où alkyl est tel que défini ci-dessus, 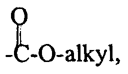 où alkyl est tel que défini ci-dessus, 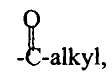 où alkyl est tel que défini ci-dessus ou phényle, ou fluorénylméthyle, 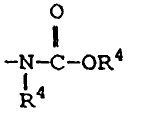 où R³ et R⁴ sont identiques ou différents et sont chacun tels que définis ci-dessus, et R^{2'}, R^{2''} et R^{2'''} sont identiques ou différents les uns et les autres et sont chacun l'hydrogène,
un alkyle en C₁-C₁₂,
un radical aromatique qui est un groupe phényle, un groupe benzyle, un groupe naphtyle, un groupe biphényle, un groupe pyrényle, un groupe anthracényle, ou un groupe fluorényle, non substitués ou substitués par 1 à 4 substituants choisis parmi les alkyle tel que défini ci-dessus, alkoxy tel que défini ci-dessus, thialkoxy tel que défini ci-dessus, hydroxy, thiol, nitro, halogène, amino, 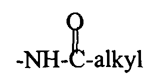 où alkyl est tel que défini ci-dessus, 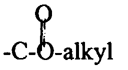 où alkyl est tel que défini ci-dessus, 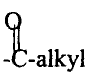 où alkyle est tel que défini ci-dessus,
ou aryle ou
un radical hétéroaromatique qui est un 2- ou 3-thiényle, 2- ou 3-furanyle, 2- ou 3-pyrrolyle, 2-, 4-, ou 5-imidazolyle, 3-, 4- ou 5-pyrazolyle, 2-, 4- ou 5-thiazolyle, 3-, 4- ou 5-isothiazolyle, 2-, 4- ou 5-oxazolyle, 3-, 4- ou 5-isoxazolyle, 3- ou 5-1,2,4-triazolyle, 4- ou 5-1,2,3-triazolyle, tétrazolyle, 2-, 3- ou 4-pyridinyle, 3-, 4- ou 5-pyridazinyle, 2-pyrazinyle, 2-, 4- ou 5-pyrimidinyle, 2-, 3-, 4-, 5-, 6-, 7- ou 8-quinolinyle, 1-, 3-, 4-, 5-, 6-, 7- ou 8-isoquinolinyle, 2-, 3-, 4-, 5-, 6- ou 7-indolyle, 2-, 3-, 4-, 5-, 6- ou 7-benzo[b]thiényle ou 2-, 4-, 5-, 6- ou 7-benzoxazolyle, 2-, 4-, 5-, 6- ou 7-benzimidazolyle, 2-, 4-, 5-, 6- ou 7-benzothiazolyle, non substitués ou substitués par 1 ou 2 substituants choisis parmi alkyle comme défini ci-dessus, aryle comme défini ci-dessus, alcoxy comme défini ci-dessus, thioalcoxy comme défini ci-dessus, hydroxy, thiol, nitro, halogène, formyle, amino, 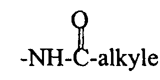 où alkyle est comme défini ci-dessus, 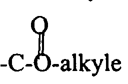 où alkyle est comme défini ci-dessus, 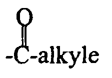 où alkyle est comme défini ci-dessus ou phényle,
avec cette condition qu'au moins un des R², R^{2'} et R^{2''} est un aryle ou un hétéroaryle tel que défini ci-dessus et R^{2'''} est un hydrogène ou un méthyle,
R² est l'hydrogène ou un méthyle,
n est égal à zéro, et
n' est un nombre égal à zéro ou un nombre entier égal à 1, 2 ou 3,
AA² est : 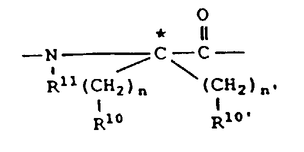 dans laquelle :
R¹¹ est un hydrogène ou un méthyle,
n est égal à zéro,
R¹⁰ est un hydrogène ou un méthyle,
n' est égal à zéro ou un est un nombre entier valant 1, 2, 3 ou 4, et
R¹⁰ est un alkyle en C₁-C₁₂,
OH, 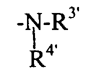 où R^{3'} et R^{4'} sont identiques ou différents et sont chacun un hydrogène,
un alkyle en C₁-C₁₂ ou bien un radical aromatique qui est un groupe phényle, un groupe benzyle, un groupe naphtyle, un groupe biphényle, un groupe pyrényle, un groupe anthracényle, ou un groupe fluorényle, non substitué ou substitué par 1 à 4 substituants choisis parmi les alkyle comme définis ci-dessus, alcoxy comme défini ci-dessus, thioalcoxy comme défini ci-dessus, hydroxy, thiol, nitro, halogène, amino, 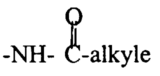 où alkyle est comme défini ci-dessus, 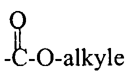 où alkyle est comme défini ci-dessus, 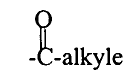 où alkyle est comme défini ci-dessus ou aryle, 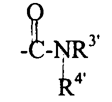 où R^{3'} et R^{4'} sont comme définis ci-dessus, 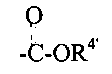 où R^{4'} est comme défini ci-dessus ;
AA³ est : 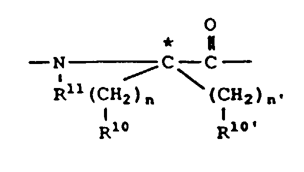 dans laquelle :
R¹¹ est un hydrogène ou un méthyle,
n est égal à zéro,
R¹⁰ est un hydrogène ou un méthyle,
n'' est égal à zéro ou est un nombre entier valant 1, 2 ou 3, et
R^{11'} est un alkyle en C₁-C₁₂, un radical aromatique qui est un groupe phényle, un groupe benzyle, un groupe naphtyle, un groupe biphényle, un groupe pyrényle, un groupe anthracényle ou un groupe fluorényle, non substitués ou substitués par 1 à 4 substituants choisis parmi les alkyles comme défini ci-dessus, alcoxy comme défini ci-dessus, thioalcoxy comme défini ci-dessus, hydroxy, thiol, nitro, halogène, amino, 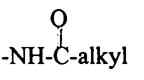 où alkyl est comme défini ci-dessus, 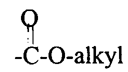 où alkyl est comme défini ci-dessus, 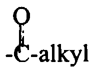 où alkyl est comme défini ci-dessus, ou aryle, 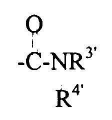 où R^{3'} et R^{4'} sont comme définis ci-dessus, 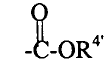 où R^{4'} est comme défini ci-dessus,
AA⁴ et AA⁵ sont chacun : 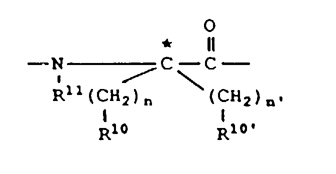 dans laquelle :
R¹¹ est un hydrogène ou un méthyle,
n est égal à zéro,
R¹⁰ est un hydrogène ou un méthyle,
n' est égal à zéro, et
R^{10'} est un alkyle en C₁-C₁₂, ou un cycloalkyle en C₃-C₁₂,
AA⁶ est : 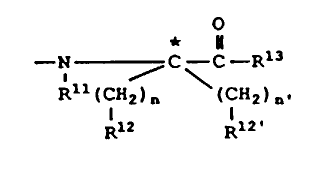 dans laquelle :
R¹¹ est un hydrogène ou un méthyle,
n est égal à zéro,
R¹² est un hydrogène ou un méthyle,
n' est égal à zéro ou est un entier valant 1, 2 ou 3,
R^{12'} est un radical aromatique qui est un groupe phényle, un groupe benzyle, un groupe naphtyle, un groupe biphényle, un groupe pyrényle, un groupe anthracényle ou un groupe fluorényle, non substitués ou substitués par 1 à 4 substituants choisis parmi alkyle comme défini ci-dessus, alcoxy comme défini ci-dessus, thioalcoxy comme défini ci-dessus, hydroxy, thiol, nitro, halogène, amino, 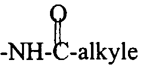 où alkyle est comme défini ci-dessus, 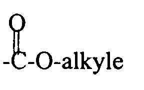 où alkyle est comme défini ci-dessus, 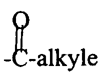 où alkyle est comme défini ci-dessus ou aryle, ou bien
un radical hétéroatomatique qui est 2- ou 3-thiényle, 2- ou 3-furanyle, 2- ou 3-pyrrolyle, 2-, 4- ou 5-imidazolyle, 3-, 4- ou 5-pyrazolyle, 2-, 4- ou 5-thiazolyle, 3-, 4- ou 5-isothiazolyle, 2-, 4- ou 5-oxazolyle, 3-, 4- ou 5-isoxazolyle, 3- ou 5-1,2,4-triazolyle, 4- ou 5-1,2,3-triazolyle, tétrazolyle, 2-, 3- ou 4-pyridinyle, 3-, 4- ou 5-pyridazinyle, 2-pyrazinyle, 2-, 4- ou 5-pyrimidinyle, 2-, 3-, 4-, 5-, 6-, 7- ou 8-quinolinyle, 1-, 3-, 4-, 5-, 6-, 7- ou 8-isoquinolinyle, 2-, 3-, 4-, 5-, 6-ou 7-indolyle, 2-, 3-, 4-, 5-, 6- ou 7-benzo[b]thiényle, ou 2-, 4-, 5-, 6- ou 7-benzoxazolyle, 2-, 4-, 5-, 6- ou 7-benzimidazolyle, 2-, 4-, 5-, 6- ou 7-benzothiazolyle, non substitués ou substitués par 1 à 2 substituants choisis parmi alkyle comme défini ci-dessus, aryle comme défini ci-dessus, alcoxy comme défini ci-dessus, thioalcoxy comme défini ci-dessus, hydroxy, thiol, nitro, halogène, formyle, amino, 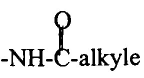 où alkyle est comme défini ci-dessus, 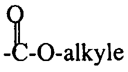 où alkyle est comme défini ci-dessus, 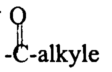 où alkyle est comme défini ci-dessus ou phényle,
R¹³ est -(CH₂)ₙ-CO₂H où n est égal à zéro ou bien est un entier valant 1, 2, 3, 4, 5 ou 6,
-(CH₂)ₙ-OH où n est égal à 0 ou est un entier valant 1, 2, 3, 4, 5 ou 6, ou bien 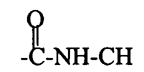 où R¹⁴ est un hydrogène ou -CH₂CO₂H,
la stéréochimie de l'atome C* dans AA¹ est D,
la stéréochimie de l'atome C* dans AA², AA³, AA⁴ ou AA⁵ est D ou L, et
la stéréochimie de l'atome C* dans AA⁶ est L.

2. Un composé selon la revendication 1, dans lequel AA¹ est :
D-Dip,
D-Bip,
D-His,
D-His(Dnp),
D-2-Nal,
D-1-Nal,
D-Phe,
D-Pgl,
D-Tyr,
D-Tyr(OMe),
D-Tyr(OEt),
D-Tyr(OtBu),
D-Trp,
D-Trp(For),
D-Tic,
D-Tza,
D-Pyr,
Ac-D-Dip,
Ac-D-Bip,
Ac-D-His,
Ac-D-His(Dnp),
Ac-D-2-Nal,
Ac-D-1-Nal,
Ac-D-Phe,
Ac-D-Pgl,
Ac-D-Tyr,
Ac-D-Tyr(OMe),
Ac-D-Tyr(OEt),
Ac-D-Tyr(OtBu),
Ac-D-Trp,
Ac-D-Trp(For),
Ac-D-Tic,
Ac-D-Tza,
Ac-D-Pyr,
Ada-D-Dip,
Ada-D-Bip,
Ada-D-His,
Ada-D-His(Dnp),
Ada-D-2-Nal,
Ada-D-1-Nal,
Ada-D-Phe,
Ada-D-Pgl,
Ada-D-Tyr,
Ada-D-Tyr(OMe),
Ada-D-Tyr(OEt),
Ada-D-Tyr(OtBu),
Ada-D-Trp,
Ada-D-Trp(For),
Ada-D-Tic,
Ada-D-Tza,
Ada-D-Pyr,
Adoc-D-Dip,
Adoc-D-Bip,
Adoc-D-His,
Adoc-D-His(Dnp),
Adoc-D-2-Nal,
Adoc-D-1-Nal,
Adoc-D-Phe,
Adoc-D-Pgl,
Adoc-D-Tyr,
Adoc-D-Tyr(OMe),
Adoc-D-Tyr(OEt),
Adoc-D-Tyr(OtBu),
Adoc-D-Trp,
Adoc-D-Trp(For),
Adoc-D-Tic,
Adoc-D-Tza,
Adoc-D-Pyr,
Boc-D-Dip,
Boc-D-Bip,
Boc-D-His,
Boc-D-His(Dnp),
Boc-D-2-Nal,
Boc-D-1-Nal,
Boc-D-Phe,
Boc-D-Pgl,
Boc-D-Tyr,
Boc-D-Tyr(OMe),
Boc-D-Tyr(OEt),
Boc-D-Tyr(OtBu),
Boc-D-Trp,
Boc-D-Trp(For),
Boc-D-Tic,
Boc-D-Tza,
Boc-D-Pyr,
Z-D-Dip,
Z-D-Bip,
Z-D-His,
Z-D-His(Dnp),
Z-D-2-Nal,
Z-D-1-Nal,
Z-D-Phe,
Z-D-Pgl,
Z-D-Tyr,
Z-D-Tyr(OMe),
Z-D-Tyr(OEt),
Z-D-Tyr(OtBu),
Z-D-Trp,
Z-D-Trp(For),
Z-D-Tic,
Z-D-Tza,
Z-D-Pyr,
Fmoc-D-Dip,
Fmoc-D-Bip,
Fmoc-D-His,
Fmoc-D-His(Dnp),
Fmoc-D-2-Nal,
Fmoc-D-1-Nal,
Fmoc-D-Phe,
Fmoc-D-Pgl,
Fmoc-D-Tyr,
Fmoc-D-Tyr(OMe),
Fmoc-D-Tyr(OEt),
Fmoc-D-Tyr(OtBu),
Fmoc-D-Trp,
Fmoc-D-Trp(For),
Fmoc-D-Tic,
Fmoc-D-Tza,
Fmoc-D-Pyr, ou bien
AA² est Ala,
Alg,
Arg,
Asn,
Asp,
Dab,
Glu,
Gln,
Gly,
homoArg,
homoGlu,
homoLys,
Ile,
Leu,
D-Leu,
Lys,
Met,
Met(O),
Met(O₂),
Nva,
Nle,
Orn,
Phe,
Tyr,
Val, ou bien
AA² est absent ;
AA³ est Asn,
Asp,
D-Asp,
N-MeAsp,
Glu,
Gln,
homoPhe,
Phe,
Tyr, ou bien
AA³ est absent ;
AA⁴ est Ala,
Chx,
Gly,
Glu,
Ile,
D-Ile,
Leu,
Nleu,
Nval,
Val, ou
AA⁴ est absent ;
AA⁵ est Ala
Chx,
Gly,
Ile,
D-Ile,
Leu,
Nleu,
Nval,
Val, ou bien
AA⁵ est absent ; et
AA⁶ est 2-Nal,
1-Nal,
Pyr,
Trp,
Tyr (OMe),
Tyr (OEt),
Tyr-(Ot-Bu),
Tyr,
Trp-Gly,
Trp-Asp,
Trp (For),
Dip,
Phe,
Bza, ou bien

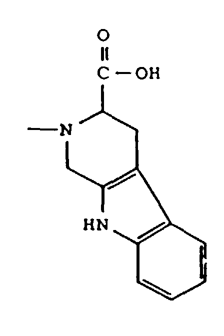

3. Un composé selon la revendication 2, choisi parmi le groupe consistant en :
D-Phe-Leu-Asp-Ile-Ile-Trp;
D-His(Dnp)-Leu-Asp-Ile-Ile-Trp;
D-Trp-Leu-Asp-Ile-Ile-Trp;
D-Tyr-Leu-Asp-Ile-Ile-Trp;
D-Tyr(OMe)-Leu-Asp-Ile-Ile-Trp;
D-Tyr(OEt)-Leu-Asp-Ile-Ile-Trp;
D-2-Nal-Leu-Asp-Ile-Ile-Trp;
D-1-Nal-Leu-Asp-Ile-Ile-Trp;
D-Pgl-Leu-Asp-Ile-Ile-Trp;
D-Pyr-Leu-Asp-Ile-Ile-Trp;
D-Tic-Leu-Asp-Ile-Ile-Trp;
D-Dip-Leu-Asp-Ile-Ile-Trp;
D-Bip-Leu-Asp-Ile-Ile-Trp;
Ac-D-Phe-Leu-Asp-Ile-Ile-Trp;
Ac-D-His(Dnp)-Leu-Asp-Ile-Ile-Trp;
Ac-D-Trp-Leu-Asp-Ile-Ile-Trp;
Ac-D-Tyr-Leu-Asp-Ile-Ile-Trp;
Ac-D-Tyr(OMe)-Leu-Asp-Ile-Ile-Trp;
Ac-D-Tyr(OEt)-Leu-Asp-Ile-Ile-Trp;
Ac-D-2-Nal-Leu-Asp-Ile-Ile-Trp;
Ac-D-1-Nal-Leu-Asp-Ile-Ile-Trp;
Ac-D-Pgl-Leu-Asp-Ile-Ile-Trp;
Ac-D-Pyr-Leu-Asp-Ile-Ile-Trp;
Ac-D-Tic-Leu-Asp-Ile-Ile-Trp;
Ac-D-Dip-Leu-Asp-Ile-Ile-Trp;
Ac-D-Bip-Leu-Asp-Ile-Ile-Trp;
Fmoc-D-Phe-Leu-Asp-Ile-Ile-Trp;
Fmoc-D-His-Leu-Asp-Ile-Ile-Trp;
Fmoc-D-Trp-Leu-Asp-Ile-Ile-Trp;
Fmoc-D-Tyr-Leu-Asp-Ile-Ile-Trp;
Fmoc-D-Tyr(OMe)-Leu-Asp-Ile-Ile-Trp;
Fmoc-D-Tyr(OEt)-Leu-Asp-Ile-Ile-Trp;
Fmoc-D-2-Nal-Leu-Asp-Ile-Ile-Trp;
Fmoc-D-1-Nal-Leu-Asp-Ile-Ile-Trp;
Fmoc-D-Dip-Leu-Asp-Ile-Ile-Trp;
Fmoc-D-Bip-Leu-Asp-Ile-Ile-Trp;
Ada-D-Phe-Leu-Asp-Ile-Ile-Trp;
Ada-D-His-Leu-Asp-Ile-Ile-Trp;
Ada-D-Trp-Leu-Asp-Ile-Ile-Trp;
Ada-D-Tyr-Leu-Asp-Ile-Ile-Trp;
Ada-D-Tyr(OMe)-Leu-Asp-Ile-Ile-Trp;
Ada-D-Tyr(OEt)-Leu-Asp-Ile-Ile-Trp;
Ada-D-2-Nal-Leu-Asp-Ile-Ile-Trp;
Ada-D-1-Nal-Leu-Asp-Ile-Ile-Trp;
Ada-D-Dip-Leu-Asp-Ile-Ile-Trp;
Ada-D-Bip-Leu-Asp-Ile-Ile-Trp;
Ac-D-Phe-D-Leu-Asp-Ile-Ile-Trp;
Ac-D-His-D-Leu-Asp-Ile-Ile-Trp;
Ac-D-Trp-D-Leu-Asp-Ile-Ile-Trp;
Ac-D-Tyr-D-Leu-Asp-Ile-Ile-Trp;
Ac-D-Tyr(OMe)-D-Leu-Asp-Ile-Ile-Trp;
Ac-D-Tyr(OEt)-D-Leu-Asp-Ile-Ile-Trp;
Ac-D-2-Nal-D-Leu-Asp-Ile-Ile-Trp;
Ac-D-1-Nal-D-Leu-Asp-Ile-Ile-Trp;
Ac-D-Dip-D-Leu-Asp-Ile-Ile-Trp;
Ac-D-Bip-D-Leu-Asp-Ile-Ile-Trp;
Ac-D-Phe-Ile-Asp-Ile-Ile-Trp;
Ac-D-His-Ile-Asp-Ile-Ile-Trp;
Ac-D-Trp-Ile-Asp-Ile-Ile-Trp;
Ac-D-Tyr-Ile-Asp-Ile-Ile-Trp;
Ac-D-Tyr(OMe)-Ile-Asp-Ile-Ile-Trp;
Ac-D-Tyr(OEt)-Ile-Asp-Ile-Ile-Trp;
Ac-D-2-Nal-Ile-Asp-Ile-Ile-Trp;
Ac-D-1-Nal-Ile-Asp-Ile-Ile-Trp;
Ac-D-Dip-Ile-Asp-Ile-Ile-Trp;
Ac-D-Bip-Ile-Asp-Ile-Ile-Trp;
Ac-D-Phe-Val-Asp-Ile-Ile-Trp;
Ac-D-His-Val-Asp-Ile-Ile-Trp;
Ac-D-Trp-Val-Asp-Ile-Ile-Trp;
Ac-D-Tyr-Val-Asp-Ile-Ile-Trp;
Ac-D-Tyr(OMe)-Val-Asp-Ile-Ile-Trp;
Ac-D-Tyr(OEt)-Val-Asp-Ile-Ile-Trp;
Ac-D-2-Nal-Val-Asp-Ile-Ile-Trp;
Ac-D-1-Nal-Val-Asp-Ile-Ile-Trp;
Ac-D-Dip-Val-Asp-Ile-Ile-Trp;
Ac-D-Bip-Val-Asp-Ile-Ile-Trp;
Ac-DPhe-Dab-Asp-Ile-Ile-Trp;
Ac-DHis-Dab-Asp-Ile-Ile-Trp;
Ac-DTrp-Dab-Asp-Ile-Ile-Trp;
Ac-DTyr(OMe)-Dab-Asp-Ile-Ile-Trp;
Ac-DTyr(OEt)-Dab-Asp-Ile-Ile-Trp;
Ac-D2Nal-Dab-Asp-Ile-Ile-Trp;
Ac-D3Nal-Dab-Asp-Ile-Ile-Trp;
Ac-DDip-Dab-Asp-Ile-Ile-Trp;
Ac-DBip-Dab-Asp-Ile-Ile-Trp;
Ac-DPhe-Arg-Asp-Ile-Ile-Trp;
Ac-DHis-Arg-Asp-Ile-Ile-Trp;
Ac-DTrp-Arg-Asp-Ile-Ile-Trp;
Ac-DTyr(OMe)-Arg-Asp-Ile-Ile-Trp;
Ac-DTyr(OEt)-Arg-Asp-Ile-Ile-Trp;
Ac-D2Nal-Arg-Asp-Ile-Ile-Trp;
Ac-D3Nal-Arg-Asp-Ile-Ile-Trp;
Ac-DDip-Arg-Asp-Ile-Ile-Trp;
Ac-DBip-Arg-Asp-Ile-Ile-Trp;
Ac-DPhe-hLys-Asp-Ile-Ile-Trp;
Ac-DHis-hLys-Asp-Ile-Ile-Trp;
Ac-DTrp-hLys-Asp-Ile-Ile-Trp;
Ac-DTyr(OMe)-hLys-Asp-Ile-Ile-Trp;
Ac-DTyr(OEt)-hLys-Asp-Ile-Ile-Trp;
Ac-D2Nal-hLys-Asp-Ile-Ile-Trp;
Ac-D3Nal-hLys-Asp-Ile-Ile-Trp;
Ac-DDip-hLys-Asp-Ile-Ile-Trp;
Ac-DBip-hLys-Asp-Ile-Ile-Trp;
Ac-DPhe-hGlu-Asp-Ile-Ile-Trp;
Ac-DHis-Glu-Asp-Ile-Ile-Trp;
Ac-DTrp-Glu-Asp-Ile-Ile-Trp;
Ac-DTyr(OMe)-Glu-Asp-Ile-Ile-Trp;
Ac-DTyr(OEt)-Glu-Asp-Ile-Ile-Trp;
Ac-D2Nal-Glu-Asp-Ile-Ile-Trp;
Ac-D3Nal-Glu-Asp-Ile-Ile-Trp;
Ac-DDip-Glu-Asp-Ile-Ile-Trp;
Ac-DBip-Glu-Asp-Ile-Ile-Trp;
Ac-DPhe-hGlu-Asp-Ile-Ile-Trp;
Ac-DHis-hGlu-Asp-Ile-Ile-Trp;
Ac-DTrp-hGlu-Asp-Ile-Ile-Trp;
Ac-DTyr(OMe)-hGlu-Asp-Ile-Ile-Trp;
Ac-DTyr(OEt)-hGlu-Asp-Ile-Ile-Trp;
Ac-D2Nal-hGlu-Asp-Ile-Ile-Trp;
Ac-D3Nal-hGlu-Asp-Ile-Ile-Trp;
Ac-DDip-hGlu-Asp-Ile-Ile-Trp;
Ac-DBip-hGlu-Asp-Ile-Ile-Trp;
Ac-DPhe-Asp-Asp-Ile-Ile-Trp;
Ac-DHis-Asp-Asp-Ile-Ile-Trp;
Ac-DTrp-Asp-Asp-Ile-Ile-Trp;
Ac-DTyr(OMe)-Asp-Asp-Ile-Ile-Trp;
Ac-DTyr(OEt)-Asp-Asp-Ile-Ile-Trp;
Ac-D2Nal-Asp-Asp-Ile-Ile-Trp;
Ac-D3Nal-Asp-Asp-Ile-Ile-Trp;
Ac-DDip-Asp-Asp-Ile-Ile-Trp;
Ac-DBip-Asp-Asp-Ile-Ile-Trp;
Ac-D-Phe-Lys-Asp-Ile-Ile-Trp;
Ac-D-His-Lys-Asp-Ile-Ile-Trp;
Ac-D-Trp-Lys-Asp-Ile-Ile-Trp;
Ac-D-Tyr-Lys-Asp-Ile-Ile-Trp;
Ac-D-Tyr(OMe)-Lys-Asp-Ile-Ile-Trp;
Ac-D-Tyr(OEt)-Lys-Asp-Ile-Ile-Trp;
Ac-D-2-Nal-Lys-Asp-Ile-Ile-Trp;
Ac-D-1-Nal-Lys-Asp-Ile-Ile-Trp;
Ac-D-Phe-Orn-Asp-Ile-Ile-Trp;
Ac-D-His-Orn-Asp-Ile-Ile-Trp;
Ac-D-Trp-Orn-Asp-Ile-Ile-Trp;
Ac-D-Tyr-Orn-Asp-Ile-Ile-Trp;
Ac-D-Tyr(OMe)-Orn-Asp-Ile-Ile-Trp;
Ac-D-Tyr(OEt)-Orn-Asp-Ile-Ile-Trp;
Ac-D-2-Nal-Orn-Asp-Ile-Ile-Trp;
Ac-D-1-Nal-Orn-Asp-Ile-Ile-Trp;
Ac-D-Phe-Gln-Asp-Ile-Ile-Trp;
Ac-D-His-Gln-Asp-Ile-Ile-Trp;
Ac-D-Trp-Gln-Asp-Ile-Ile-Trp;
Ac-D-Tyr-Gln-Asp-Ile-Ile-Trp;
Ac-D-Tyr(OMe)-Gln-Asp-Ile-Ile-Trp;
Ac-D-Tyr(OEt)-Gln-Asp-Ile-Ile-Trp;
Ac-D-2-Nal-Gln-Asp-Ile-Ile-Trp;
Ac-D-1-Nal-Gln-Asp-Ile-Ile-Trp;
Ac-D-Phe-Leu-Glu-Ile-Ile-Trp;
Ac-D-His-Leu-Glu-Ile-Ile-Trp;
Ac-D-Trp-Leu-Glu-Ile-Ile-Trp;
Ac-D-Tyr-Leu-Glu-Ile-Ile-Trp;
Ac-D-Tyr(OMe)-Leu-Glu-Ile-Ile-Trp;
Ac-D-Tyr(OEt)-Leu-Glu-Ile-Ile-Trp;
Ac-D-2-Nal-Leu-Glu-Ile-Ile-Trp;
Ac-D-1-Nal-Leu-Glu-Ile-Ile-Trp;
Ac-D-Phe-Leu-Asn-Ile-Ile-Trp;
Ac-D-His-Leu-Asn-Ile-Ile-Trp;
Ac-D-Trp-Leu-Asn-Ile-Ile-Trp;
Ac-D-Tyr-Leu-Asn-Ile-Ile-Trp;
Ac-D-Tyr(OMe)-Leu-Asn-Ile-Ile-Trp;
Ac-D-Tyr(OEt)-Leu-Asn-Ile-Ile-Trp;
Ac-D-2-Nal-Leu-Asn-Ile-Ile-Trp;
Ac-D-1-Nal-Leu-Asn-Ile-Ile-Trp;
Ac-D-Dip-Leu-Asn-Ile-Ile-Trp;
Ac-D-Bip-Leu-Asn-Ile-Ile-Trp;
Ac-DPhe-Leu-Phe-Ile-Ile-Trp;
Ac-DHis-Leu-Phe-Ile-Ile-Trp;
Ac-DTrp-Leu-Phe-Ile-Ile-Trp;
Ac-DTyr(OMe)-Leu-Phe-Ile-Ile-Trp;
Ac-DTyr(OEt)-Leu-Phe-Ile-Ile-Trp;
Ac-D2Nal-Leu-Phe-Ile-Ile-Trp;
Ac-D3Nal-Leu-Phe-Ile-Ile-Trp;
Ac-DDip-Leu-Phe-Ile-Ile-Trp;
Ac-DBip-Leu-Phe-Ile-Ile-Trp;
Ac-D-Phe-Glu-Asp-Ile-Ile-Trp;
Ac-D-Phe-Leu-Asp-Val-Ile-Trp;
Ac-D-His-Leu-Asp-Val-Ile-Trp;
Ac-D-Trp-Leu-Asp-Val-Ile-Trp;
Ac-D-Tyr-Leu-Asp-Val-Ile-Trp;
Ac-D-Tyr(OMe)-Leu-Asp-Val-Ile-Trp;
Ac-D-Tyr(OEt)-Leu-Asp-Val-Ile-Trp;
Ac-D-2-Nal-Leu-Asp-Val-Ile-Trp;
Ac-D-1-Nal-Leu-Asp-Val-Ile-Trp;
Ac-D-Dip-Leu-Asp-Val-Ile-Trp;
Ac-D-Bip-Leu-Asp-Val-Ile-Trp;
Ac-D-Phe-Leu-Asp-Chx-Ile-Trp;
Ac-D-His-Leu-Asp-Chx-Ile-Trp;
Ac-D-Trp-Leu-Asp-Chx-Ile-Trp;
Ac-D-Tyr-Leu-Asp-Chx-Ile-Trp;
Ac-D-Tyr(OMe)-Leu-Asp-Chx-Ile-Trp;
Ac-D-Tyr(OEt)-Leu-Asp-Chx-Ile-Trp;
Ac-D-2-Nal-Leu-Asp-Chx-Ile-Trp;
Ac-D-1-Nal-Leu-Asp-Chx-Ile-Trp;
Ac-D-Dip-Leu-Asp-Chx-Ile-Trp;
Ac-D-Bip-Leu-Asp-Chx-Ile-Trp;
Ac-D-Phe-Leu-Asp-D-Ile-Ile-Trp;
Ac-D-His-Leu-Asp-D-Ile-Ile-Trp;
Ac-D-Trp-Leu-Asp-D-Ile-Ile-Trp;
Ac-D-Tyr-Leu-Asp-D-Ile-Ile-Trp;
Ac-D-Tyr(OMe)-Leu-Asp-D-Ile-Ile-Trp;
Ac-D-Tyr(OEt)-Leu-Asp-D-Ile-Ile-Trp;
Ac-D-2-Nal-Leu-Asp-D-Ile-Ile-Trp;
Ac-D-1-Nal-Leu-Asp-D-Ile-Ile-Trp;
Ac-D-Dip-Leu-Asp-D-Ile-Ile-Trp;
Ac-D-Bip-Leu-Asp-D-Ile-Ile-Trp;
Ac-D-Phe-Leu-Asp-Ile-D-Ile-Trp;
Ac-D-His-Leu-Asp-Ile-D-Ile-Trp;
Ac-D-Trp-Leu-Asp-Ile-D-Ile-Trp;
Ac-D-Tyr-Leu-Asp-Ile-D-Ile-Trp;
Ac-D-Tyr(OMe)-Leu-Asp-Ile-D-Ile-Trp;
Ac-D-Tyr(OEt)-Leu-Asp-Ile-D-Ile-Trp;
Ac-D-2-Nal-Leu-Asp-Ile-D-Ile-Trp;
Ac-D-1-Nal-Leu-Asp-Ile-D-Ile-Trp;
Ac-D-Dip-Leu-Asp-Ile-D-Ile-Trp;
Ac-D-Bip-Leu-Asp-Ile-D-Ile-Trp;
Ac-D-Phe-Leu-Asp-Ile-Val-Trp;
Ac-D-His-Leu-Asp-Ile-Val-Trp;
Ac-D-Trp-Leu-Asp-Ile-Val-Trp;
Ac-D-Tyr-Leu-Asp-Ile-Val-Trp;
Ac-D-Tyr(OMe)-Leu-Asp-Ile-Val-Trp;
Ac-D-Tyr(OEt)-Leu-Asp-Ile-Val-Trp;
Ac-D-2-Nal-Leu-Asp-Ile-Val-Trp;
Ac-D-1-Nal-Leu-Asp-Ile-Val-Trp;
Ac-D-Dip-Leu-Asp-Ile-Val-Trp;
Ac-D-Bip-Leu-Asp-Ile-Val-Trp;
Ac-D-Phe-Leu-Asp-Ile-Chx-Trp;
Ac-D-His-Leu-Asp-Ile-Chx-Trp;
Ac-D-Trp-Leu-Asp-Ile-Chx-Trp;
Ac-D-Tyr-Leu-Asp-Ile-Chx-Trp;
Ac-D-Tyr(OMe)-Leu-Asp-Ile-Chx-Trp;
Ac-D-Tyr(OEt)-Leu-Asp-Ile-Chx-Trp;
Ac-D-2-Nal-Leu-Asp-Ile-Chx-Trp;
Ac-D-1-Nal-Leu-Asp-Ile-Chx-Trp;
Ac-D-Dip-Leu-Asp-Ile-Chx-Trp;
Ac-D-Bip-Leu-Asp-Ile-Chx-Trp;
Ac-D-Phe-Leu-Asp-Ile-Ile-2-Nal;
Ac-D-His-Leu-Asp-Ile-Ile-2-Nal;
Ac-D-Trp-Leu-Asp-Ile-Ile-2-Nal;
Ac-D-Tyr-Leu-Asp-Ile-Ile-2-Nal;
Ac-D-Tyr(OMe)-Leu-Asp-Ile-Ile-2-Nal;
Ac-D-Tyr(OEt)-Leu-Asp-Ile-Ile-2-Nal;
Ac-D-2-Nal-Leu-Asp-Ile-Ile-2-Nal;
Ac-D-1-Nal-Leu-Asp-Ile-Ile-2-Nal;
Ac-D-Dip-Leu-Asp-Ile-Ile-2-Nal;
Ac-D-Bip-Leu-Asp-Ile-Ile-2-Nal;
Ac-D-Phe-Leu-Asp-Ile-Ile-1-Nal;
Ac-D-His-Leu-Asp-Ile-Ile-1-Nal;
Ac-D-Trp-Leu-Asp-Ile-Ile-1-Nal;
Ac-D-Tyr-Leu-Asp-Ile-Ile-1-Nal;
Ac-D-Tyr(OMe)-Leu-Asp-Ile-Ile-1-Nal;
Ac-D-Tyr(OEt)-Leu-Asp-Ile-Ile-1-Nal;
Ac-D-2-Nal-Leu-Asp-Ile-Ile-1-Nal;
Ac-D-1-Nal-Leu-Asp-Ile-Ile-1-Nal;
Ac-D-Dip-Leu-Asp-IIe-Ile-1-Nal;
Ac-D-Bip-Leu-Asp-Ile-Ile-1-Nal;
Ac-D-His-Leu-D-Asp-Ile-D-Ile-Trp;
Ac-D-Phe-Leu-D-Asp-Ile-D-Ile-Trp;
Ac-D-Bip-Leu-D-Asp-Ile-D-Ile-Trp;
Ac-D-Dip-Leu-D-Asp-Ile-D-Ile-Trp;
Ac-D-2-Nal-Leu-D-Asp-Ile-D-Ile-Trp;
Ac-D-1-Nal-Leu-D-Asp-Ile-D-Ile-Trp;
Ac-D-Trp-Leu-D-Asp-Ile-D-Ile-Trp;
Ac-D-Dip-Asn-Ile-Ile-Trp;
Ac-D-Dip-Phe-Ile-Ile-Trp;
Ac-D-Dip-Ile-Ile-Trp;
Ac-D-Dip-Asp-Ile-Ile-Trp;
Ac-D(NMe)-Dip-Leu-Asp-Ile-Ile-Trp;
Ac-D-Dip-Leu-Asp-Ile-Ile-(NMe)Trp;
Ac-D-Dip-Leu-Asp-Ile-(NMe)Ile-Trp;
Ac-D-Dip-Leu-Asp-(NMe)Ile-Ile-Trp;
Ac-D-Dip-Leu-(NMe)Asp-Ile-Ile-Trp;
Ac-D-Dip-(NMe)Leu-Asp-Ile-Ile-Trp;
Ac-D-Phe-Asp-Ile-Ile-Trp;
Ac-D-His-Asp-Ile-Ile-Trp;
Ac-D-Trp-Asp-Ile-Ile-Trp;
Ac-D-Tyr-Asp-Ile-Ile-Trp;
Ac-D-Tyr(OMe)-Asp-Ile-Ile-Trp;
Ac-D-Tyr(OEt)-Asp-Ile-Ile-Trp;
Ac-D-2-Nal-Asp-Ile-Ile-Trp;
Ac-D-1-Nal-Asp-Ile-Ile-Trp;
Ada-D-Phe-Asp-Ile-Ile-Trp;
Ada-D-His-Asp-Ile-Ile-Trp;
Ada-D-Trp-Asp-Ile-Ile-Trp;
Ada-D-Tyr-Asp-Ile-Ile-Trp;
Ada-D-Tyr(OMe)-Asp-Ile-Ile-Trp;
Ada-D-Tyr(OEt)-Asp-Ile-Ile-Trp;
Ada-D-2-Nal-Asp-Ile-Ile-Trp;
Ada-D-1-Nal-Asp-Ile-Ile-Trp;
Ada-D-Dip-Asp-Ile-Ile-Trp;
Ada-D-Bip-Asp-Ile-Ile-Trp;
Ac-D-Phe-Asp-Ile-Ile-2-Nal;
Ac-D-Phe-Asp-Ile-Ile-1-Nal;
Ac-D-His-Asp-Ile-Ile-2-Nal;
Ac-D-His-Asp-Ile-Ile-1-Nal;
Ac-D-Tyr-Asp-Ile-Ile-2-Nal;
Ac-D-Tyr-Asp-Ile-Ile-1-Nal;
Ac-D-Trp-Asp-Ile-Ile-2-Nal;
Ac-D-Trp-Asp-Ile-Ile-1-Nal;
Ac-D-Dip-Asp-Ile-Ile-2-Nal;
Ac-D-Dip-Asp-Ile-Ile-1-Nal;
Ac-D-Bip-Asp-Ile-Ile-2-Nal;
Ac-D-Bip-Asp-Ile-Ile-1-Nal;
Ac-D-Phe-Leu-Asp-Ile-Trp;
Ac-D-His-Leu-Asp-Ile-Trp;
Ac-D-Tyr-Leu-Asp-Ile-Trp;
Ac-D-Dip-Leu-Asp-Ile-Trp;
Ac-D-Trp-Leu-Asp-Ile-Trp;
Ac-DPhe-Leu-Asp-Ile-Ile-Trp-Gly;
Ac-DHis-Leu-Asp-Ile-Ile-Trp-Gly;
Ac-DTrp-Leu-Asp-Ile-Ile-Trp-Gly;
Ac-DTyr(OMe)-Leu-Asp-Ile-Ile-Trp-Gly;
Ac-DTyr(OEt)-Leu-Asp-Ile-Ile-Trp-Gly;
Ac-D2Nal-Leu-Asp-Ile-Ile-Trp-Gly;
Ac-D3Nal-Leu-Asp-Ile-Ile-Trp-Gly;
Ac-DDip-Leu-Asp-Ile-Ile-Trp-Gly;
Ac-DBip-Leu-Asp-Ile-Ile-Trp-Gly;
Ac-DPhe-Leu-Asp-Ile-Ile-Trp-Asp;
Ac-DHis-Leu-Asp-Ile-Ile-Trp-Asp;
Ac-DTrp-Leu-Asp-Ile-Ile-Trp-Asp;
Ac-DTyr(OMe)-Leu-Asp-Ile-Ile-Trp-Asp;
Ac-DTyr(OEt)-Leu-Asp-Ile-Ile-Trp-Asp;
Ac-D2Nal-Leu-Asp-Ile-Ile-Trp-Asp;
Ac-D3Nal-Leu-Asp-Ile-Ile-Trp-Asp;
Ac-DDip-Leu-Asp-Ile-Ile-Trp-Asp; et
Ac-DBip-Leu-Asp-Ile-Ile-Trp-Asp-Asp.

4. Un composé selon la revendication 3, choisi parmi le groupe consistant en :
Ac-D-Phe-Leu-Asp-Ile-Ile-Trp;
D-2-Nal-Leu-Asp-Ile-Ile-Trp;
Ac-D-2-Nal-Leu-Asp-Ile-Ile-Trp;
D-1-Nal-Leu-Asp-Ile-Ile-Trp;
Ac-D-Phe-Leu-Asp-Ile-Trp;
Ac-D-His-Leu-D-Asp-Ile-D-Ile-Trp;
Ac-D-Phe-Orn-Asp-Ile-Ile-Trp;
Ac-D-Phe-Glu-Asp-Ile-Ile-Trp;
Ac-D-Tyr-Leu-Asp-Ile-Ile-Trp;
Ac-D-Phe-Asp-Ile-Ile-Trp;
Fmoc-D-Phe-Leu-Asp-Ile-Ile-Trp;
Ac-D-Dip-Leu-Asp-Ile-Ile-Trp;
Ac-D-Dip-Ile-Ile-Trp;
Ac-D-Dip-Asp-Ile-Ile-Trp;
Ac-D-Dip-Leu-Phe-Ile-Ile-Trp;
Ac-D-Dip-Leu-Asp-Ile-Lys-Trp;
Ac-D-Dip-Leu-Asp-Ile-Glu-Trp;
Ac-D-Dip-Leu-Asp-Glu-Ile-Trp;
Ac-D-Dip-Glu-Asp-Ile-Ile-Trp;
Ac-D-Dip-Orn-Asp-Ile-Ile-Trp;
Ac-D-Dip-Leu-Asp(N-Me)-Ile-Ile-Trp; et
Ac-D-Dip-D-Leu-Asp-Ile-Ile-Trp.

5. Une composition pharmaceutique comprenant un composé selon les revendications 1 à 4, en mélange avec un excipient, diluant ou support pharmaceutiquement acceptable.

6. Utilisation d'un composé selon l'une quelconque des revendications 1 à 4, pour la préparation d'une composition pharmaceutique utile pour le traitement de l'hypertension, des troubles métaboliques et endocriniens, de l'insuffisance cardiaque, de l'infarctus du myocarde, du choc endotoxique, de l'hémorragie subarachnoïde, de l'arythmie, de l'asthme, de l'insuffisance rénale algue, de la pré-éclampsie, du diabète et des troubles neurologiques, et pour inhiber un taux élevé d'endothéline.

7. Un procédé de préparation d'un composé selon l'une quelconques des revendications 1 à 4, de formule I :
AA¹-AA²-AA³-AA⁴-AA⁵-AA⁶
dans laquelle AA¹, AA², AA³, AA⁴, AA⁵ et AA⁶ ont les significations ci-dessus, ou un de leurs sels pharmaceutiquement acceptable comprenant le couplage séquentiel par étapes des acides aminés choisis parmi AA¹, AA², AA³, AA⁴, AA⁵ ou AA⁶ à l'acide aminé précédent en utilisant la méthodologie classique de la synthèse peptidique et, après déprotection classique pour obtenir un composé de formule I, et le cas échéant, conversion du composé de formule I en un sel pharmaceutiquement acceptable d'un composé de formule I par des méthodes classiques et, si on le désire aussi, transformation du sel pharmaceutiquement acceptable du composé de formule I en un composé de formule I par des méthodes classiques.
